(19) **Europäisches Patentamt / European Patent Office / Office européen des brevets**

(11) **EP 4 194 565 A1**

(12) # EUROPEAN PATENT APPLICATION
published in accordance with Art. 153(4) EPC

(43) Date of publication:
**14.06.2023 Bulletin 2023/24**

(21) Application number: **21856081.1**

(22) Date of filing: **20.07.2021**

(51) International Patent Classification (IPC):
**C12Q 1/6837** (2018.01)    **A61B 10/02** (2006.01)

(52) Cooperative Patent Classification (CPC):
**A61B 10/02; C12Q 1/6837**

(86) International application number:
**PCT/KR2021/009297**

(87) International publication number:
**WO 2022/035075 (17.02.2022 Gazette 2022/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:
10.08.2020  KR 20200100151
10.08.2020  KR 20200100165
10.08.2020  KR 20200100168
11.08.2020  KR 20200100561
11.08.2020  KR 20200100579
12.08.2020  KR 20200101117
12.08.2020  KR 20200101124

(71) Applicant: **Cutis Biomedical Research Center**
**Seoul 07327 (KR)**

(72) Inventors:
• **LEE, Kwang Hoon**
  **Seoul 07333 (KR)**
• **JEONG, Do Hyeon**
  **Seoul 04091 (KR)**
• **KIM, Seo Hyeong**
  **Seoul 03764 (KR)**
• **KIM, Ji Hye**
  **Seoul 07973 (KR)**
• **LEE, Sung Jae**
  **Seoul 04317 (KR)**

(74) Representative: **M. Zardi & Co S.A.**
**Via G. B. Pioda, 6**
**6900 Lugano (CH)**

(54) **MINIMALLY INVASIVE KIT FOR DIAGNOSING SKIN CONDITION, COMPRISING MICRONEEDLE PATCH**

(57) A minimally invasive kit for diagnosing a skin condition, according to one embodiment of the present invention, comprises: a device capable of quantifying the expression level of an RNA gene from a specimen extracted from the skin of a subject; and a microneedle patch including a plurality of microneedles having a solid structure and made of a biodegradable polymer hyaluronic acid. When the microneedle patch is applied to the skin of the subject and is detached after applied for a predetermined time, a specimen from the skin of the subject, which has been attached to the microneedle surfaces of the microneedle patch or an extract therefrom, is quantitatively analyzed by the device, and the device quantifies an expression level of each skin condition-related RNA biomarker gene to be diagnosed from the specimen from the skin of the subject, which has been attached to the microneedle surfaces of the microneedle patch or the extract therefrom, such that the diagnosis of each skin condition is carried out on the basis of a value of the quantification.

[Fig 2]

## Description

## Technical Field

The present invention relates to a minimally invasive kit for diagnosing a skin condition, comprising a microneedle patch.

## Background Art

The present invention of which priority right is claimed from seven Korean Patent application documents as will be listed below. All technical ideas explained in the description, claims, and drawings of the seven Korean Patent application documents will be described in the description of the present invention.

Korean patent application laid-open No. 10-2020-0100151 (entitled: Minimally invasive kit for diagnosing skin wrinkle or skin elasticity, comprising microneedle patch)

Korean patent application laid-open No. 10-2020-0100165 (entitled: Minimally invasive kit for evaluating skin hydration, comprising microneedle patch, and biomarker for evaluating skin hydration)

Korean patent application laid-open No. 10-2020-0100168 (entitled: Minimally invasive kit for evaluating skin pigmentation, comprising microneedle patch, and biomarker for evaluating skin pigmentation)

Korean patent application laid-open No. 10-2020-0100579 (entitled: Minimally invasive kit for evaluating skin sebum, comprising microneedle patch, and biomarker for evaluating skin sebum)

Korean patent application laid-open No. 10-2020-0101117 (entitled: Minimally invasive kit for evaluating rosacea skin, comprising microneedle patch, and biomarker for evaluating rosacea skin)

Korean patent application laid-open No. 10-2020-0101124 (entitled: Minimally invasive kit for evaluating stinging skin, comprising microneedle patch, and biomarker for evaluating stinging skin)

Korean patent application laid-open No. 10-2020-0100561 (entitled: Minimally invasive kit for predicting acne cosmetica, comprising microneedle patch, and biomarker for evaluating acne cosmetica)

The present invention is to diagnose, measure, or evaluate various skin conditions accurately. The skin conditions treated in the present invention are the following seven conditions.

- Skin aging (related to skin wrinkle or skin elasticity)

- Skin hydration

- Skin pigmentation

- Skin sebum

- Rosacea skin

- Stinging skin

- Acne cosmetica

Hereinafter, the technologies currently used to diagnose, measure or evaluate the above-mentioned seven skin conditions and the problems the technologies have had, which are recognized by the inventor(s), will be described in detail below.

## <Skin aging>

All living bodies become aged since they are born, and further, their skin becomes aged. Skin aging factors are classified into an internal factor developed with age and an external factor developed with the long-term exposure of skin to environmental factors such as sunlight, cold, wind, smog, and the like.

As skin is aged, amounts of collagen, elastin, and hyaluronic acid as main ingredients of extracellular matrix in the dermis layer and the number of fibroblast cells are decreased, and as a result, wrinkles are made and elasticity becomes reduced to cause the skin to sag. The reduction of the extracellular matrix in the dermis layer is caused by collagenase (MMP-1) and gelatinase (MMP-2, MMP9) as matrix metalloproteinase (MMP) enzymes, and in real aged skin, the activation and expression of the enzymes are drastically increased.

Skin barrier represents the stratum corneum as the last form of differentiation of the epidermis layer, which is called "brick and mortar". The "bricks" include corneocytes from which nucleus disappears, proteins in various cells, and degradation products having natural moisturizing factors, and cornified envelopes connected to the degradation products. The "mortar" is intercorneocyte lipids consisting of ceramides, cholesterol, and fatty acids and has a multi-layered plate. If aging is developed, dry skin is observed to a rate of about 75%, and with the age, problems may occur in important factors (lipid content in the stratum corneum and epidermis pH) for keeping the skin barrier and exerting the basic functions. On the aged skin barrier, the number of lamellas becomes small, lamella body secretion becomes decreased, and disorder of lipid metabolism such as lipid reduction in the stratum corneum happens. Further, production speed restriction enzymes for ceramides, cholesterol, and fatty acids become deteriorated in function, especially the synthesis of cholesterol may be deteriorated badly, thereby decreasing the amount of total lipids. Further, the expression of the cornified envelopes and the natural moisturizing factors becomes decreased.

To measure skin aging conditions, develop new mechanisms for skin aging, determine treatment efficacy, perform prediction test for prognosis, and develop new treating agents, accordingly, appropriate efficacy evaluation is needed. As the cosmetics made through animal tests, the cosmetics made of materials obtained through animal tests, or the imported cosmetics made through animal tests or made of materials obtained through animal tests are prohibited in distribution and sales, however, the efficacy evaluation for the studies of skin aging mechanisms or the cosmetics has been made using devices applicable to the human body, tape stripping, skin tissue biopsy, or ex vivo test using 3D skin.

As methods for measuring skin aging using the devices applicable to the human body, the following various technologies have been used at the present time of the filing date of the invention.

- Method for imaging only the morphological portion of skin using a PRIMOS device

- Method for measuring an average value between repeated contraction and relaxation of skin using negative pressure of a Cutometer device

- Method for measuring changes in transepidermal water loss (TEWL) related to the disorder of skin barrier function using a Tewameter device

- Method for measuring changes in water content through conductivity of water using a Corneometer device

- Method for measuring the acidity of the skin surface using a Skin-pHmeter device.

However, it is hard that the above-mentioned methods are utilized in the studies of specific mechanisms of skin aging, and they fail to provide the rationale capable of scientifically explaining changes in specific factors. Further, they fail to provide specific data for the changes in the extracellular matrix including collagen in the dermis as the most important target of the skin aging.

Recently, a non-invasive tape stripping method using a skin specimen applied to a tape detachably attached to a lesion area has been suggested. The skin aging is generally caused by changes in the dermis such as degeneration of collagen or elastic fibers, but the materials attached to the tape are restricted to the stratum corneum, so that through the tape stripping method, the changes in the dermis cannot be reflected.

To solve such problems, a skin biopsy is used, but in this case, a surgeon's skill is needed. Since the skin biopsy is a very invasive method, further, a patient's pain and reluctance occur, and the scar on his or her skin remains after test. By such reasons, it is hard to perform the skin biopsy before and after test medicines are used one by one in efficacy evaluation test in real human body, and the patient's compliance is very low.

The ex vivo test using 3D artificial skin shows the most developed structure that copies the skin including all of cells in the epidermis and dermis of the human body, but the test is high in price and does not include structures existing in skin, such as blood vessels, hair follicles, and subcutaneous fat, so that the ex vivo test does not reflect the changes in skin actually, thereby failing to allow the skin biopsy to be replaced thereby.

Accordingly, there is no method for replacing the animal test or skin biopsy in the studies of skin aging mechanisms or in the studies of efficacy evaluation of anti-aging agents, and therefore, there is a definite need for developing a non-invasive method for actually measuring changes in the extracellular matrix including collagen in the dermis.

**<Skin hydration>**

Skin serves as a protection barrier from external physical and chemical stimulation and to keep water therein. The skin barrier exists in the stratum corneum of the epidermis of the skin consisting of a plurality of layers and primarily protects the skin from external physical stimulation. The skin barrier has a structure of a multi-layered plate made with firm combination between corneocytes and intercorneocyte lipids. The skin barrier is called "brick and mortar". The "bricks" include corneocytes from which nucleus disappears, proteins in various cells, and degradation products having natural moisturizing factors, and cornified envelopes connected to the degradation products. The "mortar" is intercorneocyte lipids consisting of ceramides, cholesterol, and fatty acids. Accordingly, the skin barrier has the functions of preventing water loss and keeping water content. The stratum corneum is formed by the proliferation and differentiation of keratinocytes, periodically falls off from the skin by means of actions of proteases, and keeps homeostasis.

Dry skin means a skin condition where corneocytes are increased and skin surface becomes rough, tight or itchy. Owing to the internal factors occurring by skin diseases such as atopic dermatitis, psoriasis, and the like and the external factors occurring by dry environments, weather conditions such as wind, soap, chemical substances such as organic solvents, excessive bathing or face washing, ultraviolet light, physical stimulation, and the like, the dry skin is caused by the decrease of the natural moisturizing factors, intercorneocyte lipids, and sebum, the abnormal falling of the stratum corneum, and other reasons. Accordingly, appropriate use of moisturizing agents or lifestyle management are needed to avoid the dry skin.

Accordingly, there is a need for appropriate efficacy evaluation for the studies of mechanisms capable of recognizing skin hydration condition and skin hydration disorder and for the development of moisturizing agents or skin care capable of correcting or treating the skin hydration disorder. As the cosmetics made through animal tests, the cosmetics made of materials obtained through animal tests, or the imported cosmetics made through animal tests or made of materials obtained through animal tests are prohibited in distribution and sales, the efficacy evaluation for the studies of skin hydration mechanisms or the cosmetics has been made using devices applicable to the human body, tape stripping, skin tissue biopsy, or ex vivo test using 3D skin.

There are Corneometer, Tewameter, and Skin-pHmeter as the devices applicable to the human body for measuring skin hydration. The Corneometer serves to measure the skin hydration using the fact that capacitance as quantity of electricity storing electric charges is proportional to water content, and in this case, the water content is measured up to a depth of 30 to 40 $\mu$m under the stratum corneum. The larger the water content is, the higher the numerical value is. The Tewameter has a close relation with the damage of the skin barrier and serves to measure transepidermal water loss (TEWL). If the skin barrier is damaged, an amount of water evaporated and thus lost to the epidermis becomes increased, and in this case, the water evaporated from the epidermis of skin is sensed by the device and provided with numerical values. The higher the numerical value is, the larger the amount of water loss is. The Skin-pHmeter for measuring skin acidity serves to check whether hydrogen ion concentration measured on the skin surface is over weak acidity (pH 4.5 to 5.5) as the acidity of normal skin.

The devices applicable to the human body are very sensitive and thus much influenced by measurement environments and measuring persons. Accordingly, when the measurement is conducted by a skilled person, while the devices are being exposed in constant temperature and humidity conditions for a given time, reliable measurement results can be obtained. Further, the devices just measure skin water content, transepidermal water loss, and skin pH to thus recognize a degree of dryness of skin, but if the skin hydration is abnormal because of various reasons, it is hard to develop the reason mechanisms, treatment methods or care methods through the devices.

Even in the case of the non-invasive tape stripping method for analyzing a skin specimen applied to a tape detachably attached to a lesion area, it just measures the stratum corneum as the uppermost layer of the epidermis of skin, thereby providing no specific data on the changes related to the hydration that exist in the region from the stratum granulosum below the stratum corneum to the basal cell layer and the dermis layer.

To solve such problems, a skin biopsy is used, but in this case, a surgeon's skill is needed. Since the skin biopsy is a very invasive method, further, a patient's pain and reluctance occur, and the scar on his or her skin remains after test. By such reasons, it is hard to perform the skin biopsy before and after test medicines are used one by one in efficacy evaluation test in real human body, and the patient's compliance is very low.

The ex vivo test using 3D artificial skin shows the most developed structure that copies the skin including all of cells in the epidermis and dermis layers of the human body, but the test is high in price and does not include structures existing in skin, such as blood vessels, hair follicles, and subcutaneous fat, so that the ex vivo test does not reflect the changes in skin actually, thereby failing to allow the skin biopsy to be replaced thereby.

Accordingly, there is no method for replacing the animal test or skin biopsy in the studies of skin hydration mechanisms or in the studies of efficacy evaluation of skin moisturizing agents, and therefore, there is a definite need for developing a non-invasive method for measuring changes in the total layers of the epidermis and dermis of skin.

**<Skin pigmentation>**

Melanin is a phenol-based polymer distributed in a nature and a complex polymer of a black pigment and protein. Melanin has a function of blocking a given amount of ultraviolet light to thus keep skin temperature appropriately and protect the skin from the ultraviolet light. Further, melanin is responsible for determining skin colors of humans. Skin melanin is produced in melanocytes, and as melanin expression genes are different according to races, accordingly, amounts of melanocytes are adjusted according to the melanin expression genes, so that the skin colors are determined.

If the skin is exposed to ultraviolet light, keratinocytes secrete melanocyte-stimulating hormone, and the melanocyte-stimulating hormone is combined with melanocorin-1 receptor (MC1-R) of the melanocytes and acts with the nucleuses of the melanocytes, so that it expresses enzymes such as tyrosinase, TYRP1, TYRP2, etc. and starts to produce melanin.

A process of producing melanin is as follows. First, melanosomes as small organisms in melanocytes are produced, and next, amino acids as tyrosine and DOPA are oxidized from the melanosomes and then oxidized to DOPA-quinone by means of tyrosinase as oxidase. After that, automatic oxidation reaction occurs to produce 5, 6-dihydroxyindol and indol-6, 6-quinone, and dark and brown melanin is finally produced. Next, a large number of melanosomes move to the keratinocytes therearound through the dendrites of the melanocytes and are thus dissolved, and accordingly, the melanocytes are accumulated in the keratinocytes to express the skin colors.

The types of skin according to skin colors are classified into four types of Caucasian skin so as to determine an initial amount of psoriasis treated, which are made for the first time in 1975 by American dermatologist Fitzpatrick. After that, two types of skin are added so that Caucasians have skin types I, II, III, and IV, Asians have skin type V, and black Africans have skin type VI. However, even the Asians have various skin types, which are revealed by many researchers, and further, various errors have happened, so that at present, skin type classification from the skin type I that always has sunburn to the skin type VI that has tanning, without sunburn, is given with respect to skin reactions to the ultraviolet light of the sun.

To perform the evaluation or measurement of skin pigmentation, the development of new reasons and mechanisms for changes of skin pigmentation in various skin pigment diseases, the determination of skin pigment change treatment efficacy, prediction test for prognosis, and the development of new skin pigment treating agents, accordingly, appropriate efficacy evaluation is needed. As the cosmetics made through animal tests, the cosmetics made of materials obtained through animal tests, or the imported cosmetics made through animal tests or made of materials obtained through animal tests are prohibited in distribution and sales, the efficacy evaluation for the studies of skin pigmentation mechanisms or the cosmetics has been made using devices applicable to the human body, tape stripping, skin tissue biopsy, or ex vivo test using 3D skin.

As methods for measuring skin pigments using devices applicable to the human body, there are a method using Mexameter that measures a skin absorption rate of melanin as a main factor determining skin colors and hemoglobin under absorbance principle and a method using Spectrophotometer that measures spectral reflectance of skin colors to calculate brightness and saturation of skin with tristimulus values. However, the devices measure only the skin colors or rubeosis, and accordingly, they are hard to be used for the studies of specific mechanisms of the skin pigmentation. Further, the devices fail to scientifically explain the changes of specific factors and do not provide specific data on the changes of the melanogenesis as the most important target of the skin pigmentation.

Accordingly, a non-invasive tape stripping method for analyzing a skin specimen applied to a tape detachably attached to a lesion area is suggested. The main mechanism of the skin pigmentation is caused by the changes in the melanocytes existing in the basal layer of skin, but the materials attached to the tape are limited to the stratum corneum, so that it is hard to reflect the changes in the epidermis layer (stratum granulosum, epithelial cell layer, and basal cell layer) or the dermis layer below the stratum corneum.

To solve such problems, a skin biopsy is used, but in this case, a surgeon's skill is needed. Since the skin biopsy is a very invasive method, further, a patient's pain and reluctance occur, and the scar on his or her skin remains after test. By such reasons, it is hard to perform the skin biopsy before and after test medicines are used one by one in efficacy evaluation test in real human body, and the patient's compliance is very low.

The ex vivo test using 3D artificial skin shows the most developed structure that copies the skin including all of cells in the epidermis and dermis layers of the human body, but the test is high in price and does not include structures existing in skin, such as blood vessels, hair follicles, and subcutaneous fat or various immunocytes existing in the epidermis and dermis of skin, so that the ex vivo test does not reflect the changes in skin actually, thereby failing to allow the skin biopsy to be replaced thereby.

Accordingly, there is no method for replacing the animal test or skin biopsy in the studies of skin pigmentation mechanisms or in the studies of efficacy evaluation of skin pigmentation treating agents, and therefore, there is a definite need for developing a non-invasive method for measuring changes in the total layers of the epidermis of skin.

## <Skin sebum>

Skin sebum is a substance produced by skin's sebaceous glands and consists of triglceride, wax ester, and squalene. The sebaceous glands are hair's accessory glands and exist in the dermis layer, and the secreted sebum is coated on the surface of skin to prevent water from emitting to the outside. Further, the sebum neutralizes alkali, has a sterilization function, prevents skin from being dry, and delays skin aging.

Therefore, when an appropriate amount of sebum is kept, healthy skin is ensured, but if sebum is excessively secreted owing to male hormone, hyperkeratinization of hair follicles, inflammation by propionibacterium acnes, environmental factors such as stress, and genetic factors, the skin pores are blocked to cause skin troubles. The diseases related to the skin troubles are acnes, seborrhoeic dermatitis, and the like.

Sebum secretion is different depending upon age and gender. In infancy, an amount of sebum secreted is small, in youth, sebum is increased in amount, and with age, sebum is decreased in amount. Further, males have a larger amount of sebum caused by hormone than females. Generally, the skin having a larger amount of sebum than dry skin is called oily skin. The area in the middle of a person's face that includes the forehead, the portion between the eyebrows, and the nose, in which a lot of sebaceous glands exist, is called T-zone. The area covering the cheeks and chin is called U-zone. Generally, in the U-zone the number of sebaceous glands distributed is small. A person who has the dry U-zone and the oily T-zone is sorted to have complex skin. In the case of the oily skin, it is important to select appropriate cleaning and moisturizing agents.

In the studies of mechanisms for secretion disorder or hypersecretion of skin sebum under the recognition of skin sebum conditions, appropriate efficacy evaluation is needed. Further, in the development of cleaning and treating agents for collecting and treating skin sebum disorder and in the development of a skin care method, appropriate efficacy evaluation is needed.

As the cosmetics made through animal tests, the cosmetics made of materials obtained through animal tests, or the imported cosmetics made through animal tests or made of materials obtained through animal tests are prohibited in distribution and sales, the efficacy evaluation for the skin sebum disorder or hypersecretion mechanisms or the functional cosmetics related thereto has been made using devices applicable to the human body, tape stripping, skin tissue biopsy, or ex vivo test using 3D skin.

As a device applicable to the human body for measuring skin sebum, there is Sebumeter made by Courage + Khazaka Electronic. A sebum collection tape with a thickness of 0.1 mm is attached to skin, and then, the absorbed sebum to the tape is measured. If the tape becomes transparent by the sebum, light transmittance becomes high, so that an amount of sebum is measured.

The device applicable to the human body is very sensitive and thus much influenced by measurement environments and measuring persons. Accordingly, when the measurement is conducted by a skilled person, while the device is being exposed in constant temperature and humidity conditions for a given time, reliable measurement results can be obtained. Further, the device measures just the sebum secreted from the skin surface, and accordingly, it is not proper for the use in the studies of specific mechanisms of skin sebum secretion. Further, the device fails to scientifically explain the changes of specific factors and do not provide specific data on the changes in the sebaceous glands existing in the dermis.

Accordingly, a non-invasive tape stripping method for analyzing a skin specimen applied to a tape detachably attached to a lesion area is suggested, but in this method, a specimen collection area is restricted to the stratum corneum, thereby failing to reflect the changes in the dermis layer related to the skin sebum.

To solve such problems, a skin biopsy is used, but in this case, a surgeon's skill is needed. Since the skin biopsy is a very invasive method, further, a patient's pain and reluctance occur, and the scar on his or her skin remains after test. By such reasons, it is hard to perform the skin biopsy before and after test medicines are used one by one in efficacy evaluation test in real human body, and the patient's compliance is very low.

The ex vivo test using 3D artificial skin shows the most developed structure that copies the skin including all of cells in the epidermis and dermis layers of the human body, but the test is high in price and does not include structures existing in skin, such as blood vessels, hair follicles, and subcutaneous fat or various immunocytes existing in the epidermis and dermis of skin, so that the ex vivo test does not reflect the changes in skin actually, thereby failing to allow the skin biopsy to be replaced thereby.

Accordingly, there is no method for replacing the animal test or skin biopsy in the studies of mechanisms of skin sebum secretion conditions or in the studies of efficacy evaluation of skin sebum, and therefore, there is a definite need for developing a non-invasive method for measuring changes in the dermis layer of skin.

## <Rosacea skin>

The sensitive skin as one of four Baumann skin types is classified into four skin types, that is, acne type, stinging type, allergic type, and rosacea type. The sensitive skin may have one of the four skin types, and otherwise, it may have two or more skin types, together. In the Baumann skin type questionnaire for 27,485 American patients, it is observed that 73% of them have the sensitive skin, and 49% of the patients with the sensitive skin have rosacea skin type.

Rosacea causes facial redness, occurs in the central face, and is a skin condition that appears owing to telangiectasia. Further, rosacea may occur by various reasons such as menopause hormone imbalance, sensitivity caused by skin barrier weakening, steroid therapy side effects, and the like. Rosacea occurs temporarily by external stimulation, but if it becomes chronic, it turns into chronic inflammatory disease.

Rosacea skin causes redness and flushing when a cosmetic material having specific ingredients is applied to the face, and in some cases, it may accompany papules or telangiectasia. In the case of the rosacea skin, the drastic changes in face temperature have to be minimized, and the use of a cleansing or moisturizing agent with antiinflammatory efficacy or a sunscreen is recommended. For example, they may be aloe, argan oil, bisabolol, caffeine, chamomile, cucumber, green tea, witch hazel, niacinamide, salicylic acid, and the like.

To develop new mechanisms for the rosacea skin, to predict all kinds of cosmetic ingredients causing the rosacea skin, and to develop new rosacea skin treating agents, accordingly, appropriate efficacy evaluation is needed. As the cosmetics made through animal tests, the cosmetics made of materials obtained through animal tests, or the imported cosmetics made through animal tests or made of materials obtained through animal tests are prohibited in distribution and sales, the efficacy evaluation for the studies of rosacea skin mechanisms or the cosmetics has been made using devices applicable to the human body, tape stripping, skin tissue biopsy, or ex vivo test using 3D skin.

As devices applicable to the human body for measuring rosacea skin, there are Mexameter (made by Courage + Khazaka Electronic) and Spectrophotometer. The Mexameter measures a melanin value and a hemoglobin value (causing erythema) simultaneously, and in specific, the Mexameter is a spectroscopic device that measures the reflectivity of light emitted from a probe through three different wavelength bands (568 nm, 660 nm, and 880 nm) corresponding to melanin and erythema, and in this case, an amount of erythema is provided with erythema index (EI). The Spectrophotometer is a device for measuring the spectral transmittance of an object color, and colors are calculated with L*, a*, and b* values in a CIE colorimetric system. The L* value represents lightness, the a* value red color, and B* yellow color. As the values become close to zero, the colors are achromatic colors, and if the values become distant from zero, the colors are high in chromaticity.

The devices applicable to the human body are very sensitive and thus much influenced by measurement environments and measuring persons. Accordingly, when the measurement is conducted by a skilled person, while the devices are being exposed in constant temperature and humidity conditions for a given time, reliable measurement results can be obtained. Further, the devices measure just the color of the skin surface, and accordingly, it is hard to be used for the studies of specific mechanisms of the rosacea skin and to scientifically explain the changes of specific factors. Further, the devices do not provide specific data on the capillaries existing in the dermis, which are important in the rosacea skin.

The ex vivo test using 3D artificial skin shows the most developed structure that copies the skin including all of cells in the epidermis and dermis layers of the human body, but the test is high in price and does not include structures existing in skin, such as blood vessels, hair follicles, and subcutaneous fat or various immunocytes existing in the epidermis and dermis of skin, so that the ex vivo test does not reflect the changes in skin actually, thereby failing to allow the skin biopsy to be replaced thereby.

Accordingly, there is a need for a new test for predicting rosacea skin so as to use treating agents or cosmetics good for the rosacea skin.

**<Stinging skin>**

Stinging skin is the skin that reacts with stimulating materials, stress, and environmental factors to provide irritation sensation and stimulation reaction. The stinging skin is defined in subjective and objective views. The subjective stinging skin causes subjective symptoms such as burning sensation, itchiness, and the like, without having objective inflammatory symptoms. The objective stinging skin causes objective symptoms accompanying skin lesions such as erythema, wheal, blister, and the like, which are observed by the dermatologist.

It is known that the sensitive skin is caused by mechanisms such as signal transmission increase of sensory nerves, immune reaction increase, skin barrier weakening, and the like, but the sensitive skin may be caused very complicatedly. The internal factors in the factors causing the sensitive skin include heredity, overwork, stress, lack of sleep, and the like, and the external factors include cosmetics, cleaning agents, UV light, chemicals, environments, and the like. In the Baumann skin type questionnaire for 27,485 American patients, it is observed that 73% of them have the sensitive skin, and 15% of the patients with the sensitive skin have stinging skin.

To measure the degree of stinging skin or develop new mechanisms of the stinging skin, to perform the prediction test for prognosis, and to develop topical agents or cosmetics for preventing the stinging skin in advance, accordingly, appropriate efficacy evaluation is needed. As the cosmetics made through animal tests, the cosmetics made of materials obtained through animal tests, or the imported cosmetics made through animal tests or made of materials obtained through animal tests are prohibited in distribution and sales, there are, as the currently used methods for evaluating the mechanism studies of the stinging skin or the individual sensitivity, a subjective measurement method for determining the degree of irritation according to the opinions of subjects, an objective measurement method for observing the degree of irritation by the visual inspection of the dermatologist, and a skin tissue biopsy.

The subjective measurement method for determining the degree of irritation according to the opinions of subjects includes a lactic acid sting test and a burning sensation or pain provocation test using a mixture solution of chloroform and methanol, and the objective measurement method includes a method for applying SLS, DMSO, ammonium hydroxide, sodium hydroxide, and the like to skin to thus observe changes in skin, such as, erythema, blister, and the like. However, there is no standardized test method for diagnosing the stinging skin yet, and in the case of the lactic acid sting test cited in many studies, after the application of lactic acid, the test is dependant upon the subjective senses of the subject, so that the reproducibility and accuracy of the test are deteriorated to fail to provide objective quantification, and therefore, it is hard to consider the lactic acid sting test as a perfect test. There is a need for an objective sting diagnosis method that can be reproduced, excepting subjective evaluation.

To solve such problems, a skin biopsy is used, but in this case, a surgeon's skill is needed. Since the skin biopsy is a very invasive method, further, a patient's pain and reluctance occur, and the scar on his or her skin remains after test. By such reasons, it is hard to perform the skin biopsy before and after test medicines are used one by one in efficacy evaluation test in real human body, and the patient's compliance is very low.

Accordingly, there are no other methods for objectively evaluating the degree of stinging skin, excepting the lactic acid sting test, so as to develop studies of mechanisms of the subjective stinging skin and the topical agents or cosmetics for preventing the subjective stinging skin in advance. Therefore, there is a need for discovering biomarkers specifically expressed in the stinging skin, a non-invasive method for evaluating the degree of stinging skin using the biomarkers, and a kit for performing the evaluation.

**<Acne cosmetica>**

Sensitive skin is classified into four skin types, that is, acne skin having lesions such as papules and pustules, rosacea skin having repeated flushing and burning symptoms, stinging skin having stinging symptom, and allergic skin having erythema and itching symptoms when coming into contact with allergen.

Acne is a common inflammatory disease of the pilosebaceous unit of the skin and affects about 80% of the adolescent or young people. Acne is generated by various factors such as hyperkeratinization of hair follicles, inflammatory reaction, sebum secretion increase, colonization of *proprionibacterium acnes,* and the like. In addition, acne causes various clinical manifestation by complex factors such as environmental factors. Acne includes non-inflammatory lesion having open comedo called black head and closed comedo called white head and inflammatory lesion having papules, pustules, and nodules. Mild acne has comedos as main lesions, and if the acne becomes a little bad, papules and pustules appear as lesions. Further, severe acne has nodules and pseduocyst as main lesions.

Cosmetics have a significant impact on the acne generated after adolescence. In the case of some of patients with acne skin, acne form eruption frequently occurs after they have used cosmetics. In the Baumann skin type questionnaire for 27,485 American patients, it is observed that 73% of them have the sensitive skin, and 58% of the patients with the sensitive skin have acne cosmetica. Acne-causing capability after the use of cosmetics is obtained by comedo-causing capability caused by horny plug and the formation of papules and pustules.

Various results are provided according to testing technologies. If a concentration of isopropyl myristate or vaseline is less than 10%, a rabbit ear causes comedos early, but the human body causes papules and pustules. An emulsifier such as sodium lauryl sulfate causes papules and pustules in proportion to its capacity. Comedo-producing ingredients such as isopropyl myristate, isopropyl palmitate, and coconut oil cause acne cosmetica, and therefore, a person having the acne cosmetica has to use the cosmetics in which the above ingredients are not contained. However, accurate generation mechanisms of the acne cosmetica are not known well, and there is no test method for predicting whether which type of skin causes the acne cosmetica.

To discover a new mechanism in the generation of the acne cosmetica, to predict whether which kinds of cosmetic ingredients cause the acne cosmetica, and to develop new treating agents, accordingly, new appropriate evaluation is needed. As the cosmetics made through animal tests, the cosmetics made of materials obtained through animal tests, or the imported cosmetics made through animal tests or made of materials obtained through animal tests are prohibited in distribution and sales, there are, as the currently used methods for evaluating the acne generation mechanism studies or individual acne cosmetica, a method for observing a degree of acne cosmetica by the visual inspection of the dermatologist and a test method having primary variables for acne in the guideline of ministry of food and drug safety. Recently, animal tests stop in cosmetics, and usage test is carried out. Otherwise, human body tests are carried out with a method for checking fine comedo generation through strong adhesive or cationic polymer surface biopsy after cosmetics have been applied to a closed area for four weeks.

As the test method having primary variables for acne in the guideline of ministry of food and drug safety, there are a method for measuring the types and number of lesions on a subject face before and after action of a test material to thus record the corresponding grades in first to fifth grades and a method for measuring the number of lesions to thus calculate Michaelson's acne severity index (ASI). However, the determination is made according to a tester's subjective standard, and accordingly, there is a limitation in objective quantification. Further, only the current condition of acne seen with the naked eye is recorded, and accordingly, it is hard to scientifically explain the studies of the specific mechanisms of the acne cosmetica generation or the changes of specific factors, so that the above test methods are not a perfect test method. The ex vivo test using 3D artificial skin shows the most developed structure that copies the skin including all of cells in the epidermis and dermis layers of the human body, but the test is high in price and does not include structures existing in skin, such as blood vessels, hair follicles, and subcutaneous fat or various immunocytes existing in the epidermis and dermis of skin, so that the ex vivo test does not reflect the changes in skin actually. Accordingly, there is a need for a test for predicting acne cosmetica generation so as to select appropriate treating agents or cosmetics for the skin having frequent acne form eruption owing to the use of cosmetics after adolescence.

The problems the technologies at present used after the filing date of the invention have had so as to measure or evaluate the above-mentioned seven skin conditions have been explained. The inventor(s) make use of microneedles so as to diagnose, measure or evaluate skin conditions. Each microneedle having a length less than or equal to 1 mm penetrates the stratum corneum with minimal invasion, makes a fine hole in the skin, and delivers a drug to the hole of the skin. Recently, the microneedles are used to collect body specimens and thus predict and diagnose diseases, and accordingly, they become widened in the use range thereof. In specific, a body specimen such as a body fluid, blood, and the like is collected through the fine holes formed by the microneedles and then used in detecting biomarkers and predicting diseases. For example, hollow microneedles have capillaries in the interiors thereof, and after they are attached to the skin, blood is extracted through the capillaries. While a patient's pain is being minimized, advantageously, the blood is extracted in a relatively safe manner to detect the glucose and cholesterol in the blood biomarkers, but if the microneedles are broken in the skin, side effects or bleeding may be generated.

In the case of swellable microneedles, further, after they are attached to the skin, they absorb an interstitial liquid from the skin and thus swell to seal punched portions, so that the microneedles can be attached to the skin, without any adhesive. Next, the absorbed body fluid is separated and used to detect biomarkers and monitor and predict diseases. However, the swellable microneedle patch has to be attached to the skin for long hours so as to absorb the body fluid needed for the analysis. In this case, it is hard to stably attach the patch owing to various environmental factors.

In the case of dissolving microneedles used in the present invention, a polymer dissolved in the body and effective ingredients are mixed and solidifiedly made to the form of microneedles. If the dissolving microneedles are attached to skin, they are molten efficiently by a body fluid and easily deliver a drug to the interior of the skin. Further, the dissolving microneedles are not made of metals or plastics, so that since they have no danger of being broken in the skin, they can be used safely. The present applicant desires to use the dissolving microneedles for new purposes according to the present invention. the new purposes are to collect a skin specimen (RNA, DNA, protein) using the dissolving microneedles for the first time, analyze RNA microarray using the specimen, discover acne cosmetica biomarkers based on the analyzed results, and to conduct acne cosmetica prediction using the biomarkers and efficacy evaluation of treating agents or cosmetics.

Recently, studies of skin type analysis have been actively made under direct-to-consumer (DTC) service, and the service provides the information obtained by analyzing single nucleotide polymorphism (SNP) of gene DNA related to skin. Generally, oral epithelial cells are collected and the nucleic sequence for DNA is analyzed with the collected oral epithelial cells. It is compared whether the nucleic sequence for a specific gene is different from that of a normal person, and thus, a degree of danger is suggested. Even if skin genes are inspected through a non-invasive method, limitations still exist. Every cell in the body has the same DNA, and because DNA does not change, it does not reflect the real skin condition changing according to various environmental factors. If an individual who is born with specific SNPs is recovered through acquired care or treatment, the corresponding results cannot be reflected on SNP analysis. Because of such properties, it is hard that the SNP analysis is adopted in the discovery, mechanism study, and efficacy evaluation of a new hit compound. Besides, the current SNP analysis using oral epithelial cells does not reflect the real skin condition because an area where a specimen is collected is not skin, but oral epithelial cells. Contrarily, RNA is a material translated into proteins acting in real cells and provides specific expression every cell, and accordingly, RNA reflects the characteristics of the real skin changing by various factors. Therefore, the observation of the expression of RNA and protein levels in real skin cells is necessary for accurate analysis of skin conditions.

Unlike DNA genome analysis, RNA transcriptome analysis enables the genes that are specifically expressed only in skin cells or differently expressed according to environmental conditions to be detected and checks a difference in amounts of expression of genes between a test group and a control group, so that the RNA transcriptome analysis is adopted in the efficacy evaluation, mechanism studies, and candidate discovery that require comparison before and after a test. Through microarray technology, further, about forty thousand genes from one sample are compared in expression, which is very useful in the prediction of diseases or the discovery of treatment biomarkers. As mentioned above, however, there are limitations in collecting RNA and proteins in a non-invasive method. The inventor(s) propose a method for overcoming the above-mentioned limitations.

**Disclosure**

**Technical Problem**

Accordingly, the present invention has been made in view of the above-mentioned problems occurring in the conventional skin condition diagnosis, measurement, or evaluation methods. For example, in the case of the tissue biopsy among the conventional methods, a surgeon's skill is needed, and a patient's pain occurs, so that the tissue biopsy causes the patient's reluctance and the scar on his or her skin remains after a test. In the case of the conventional tape stripping, materials attached to a tape are limited to the stratum corneum, and accordingly, the tape stripping fails to provide perfect results, so that it is just used as an auxiliary method. In the case of the conventional SNP array analysis, further, the DNA of a human specimen is used, but since DNA is determined at birth and does not change, it is hard to reflect the real skin conditions changing by various environmental factors.

It is an object of the present invention to provide a minimally invasive kit for diagnosing a skin condition that is capable of allowing a patient to rarely feel pain and have no scar on the skin and many conveniences in use, unlike the existing tissue biopsy, collecting the skin factors in the stratum corneum to thus enhance the reliability of analysis results when compared with the existing tape stripping, and reflecting his or her real skin condition changing by various environmental factors.

It is another object of the present invention to provide a biomarker for measuring a skin condition that is capable of making use of a skin specimen as a biological sample.

It is yet another object of the present invention to provide the following material and methods through the accomplishment of the above-mentioned objects of the present invention.

- Composition for skin condition prediction

- Skin condition prediction test method and skin type classification method

- Method for screening inducing or inhibiting candidates of skin conditions and evaluating efficacy of treating agents

- Method for evaluating efficacy in the application of the human body in animal replacement tests of general cosmetics, functional cosmetics, medical devices, medicines, and the like for improving skin conditions.

**Technical Solution**

To accomplish the above-mentioned objects, according to the present invention, there is provided a minimally invasive kit for diagnosing a skin condition, including: a device for quantifying the expression levels of RNA genes from a specimen extracted from the skin of a subject; and a microneedle patch comprising a plurality of microneedles each having a solid structure and made of a bioidegradable polymer hyaluronic acid. When the microneedle patch is applied to the skin of the subject and is then detached after applied for a predetermined time, the specimen attached to the microneedle surfaces of the microneedle patch from the skin of the subject or an extract extracted from the specimen is quantitatively analyzed by the quantifying device, and the quantifying device quantifies the expression levels of RNA biomarker genes related to the skin condition to be diagnosed from the specimen attached to the microneedle surfaces of the microneedle patch from the skin of the subject or the extract extracted from the specimen, such that the skin condition is diagnosed based on the quantified values by the quantifying device.

If the skin condition to be diagnosed is skin aging, the skin condition may be diagnosed using two types of biomarker genes having the RNA biomarker genes related to collagen/elasticity and the RNA biomarker genes related to the skin barrier function/water synthesis. The biomarkers related to the collagen/elasticity may include one or two genes selected from COL1A1, COL3A1, FN1, GSTA3, PON1, PINK1, COL4A4, and MMP8, and the biomarkers related to the skin barrier function/water synthesis may include one or two genes selected from IVL, HAS2, HAS3, AQP3, CERS6, CLDN1, SLC9A1, TGM1, SPINK5, KLF4, LCE1A, LCE1B, LCE1F, LCE2A, BGN, and AZGP1.

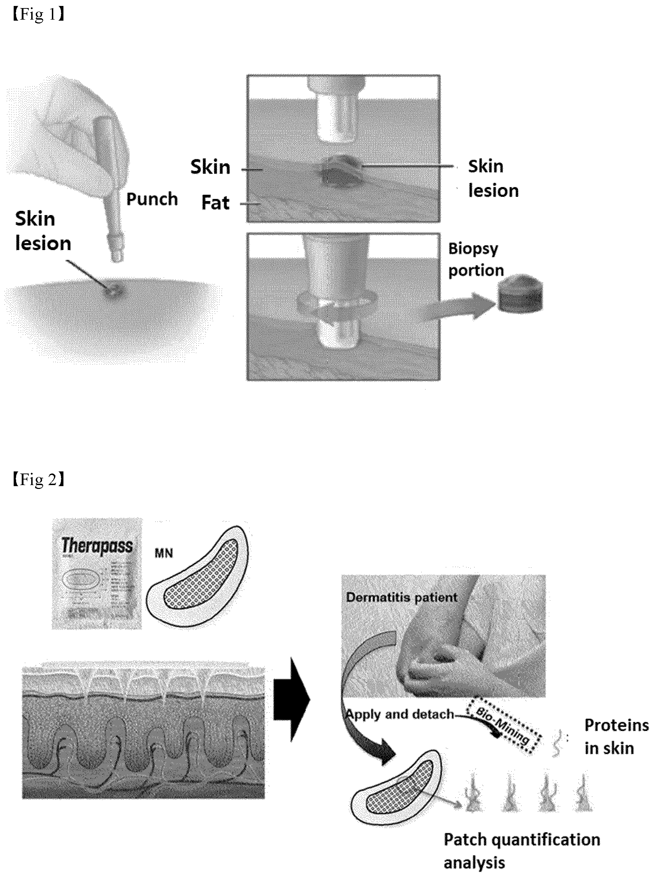

If the skin condition to be diagnosed is skin hydration, the skin condition may be diagnosed using the RNA biomarker genes related to the skin hydration, and the biomarkers related to the skin hydration may include one or two genes selected from CDSN, FLG, FLG2, LOR, KLF4, KRT10, LCE1A, LCE2A, LCE2B, LCE2C, SMPD3, CDH1, ITGB4, IVL, SPINK5, CLDN1, AQP3, BGN, HAS3, TGM1, CLDN7, CERS3, CLDN4, and KRT1.

If the skin condition to be diagnosed is skin pigmentation, the skin condition may be diagnosed using the RNA biomarker genes related to the skin pigmentation, and the biomarkers related to the skin pigmentation may include one or two genes selected from CAT, CLU, DSG1, GPNMB, GPX4, GSTM2, GSTP1, MLANA, PAX3, SOX10, TFAP2A, TYR, TYRP1, MC1R, F2RL1, and CLDN1.

If the skin condition to be diagnosed is skin sebum, the skin condition may be diagnosed using the RNA biomarker genes related to the skin sebum, and the biomarkers related to the skin sebum may include one or two genes selected from MFAP2, IGF1, HSD11B1, GPAM, CPT1C, AR, MPZL3, AQP3, SREBF2, and HSD17B2.

If the skin condition to be diagnosed is rosacea skin, the skin condition may be diagnosed using the RNA biomarker genes related to the rosacea skin, and the biomarkers related to the rosacea skin may include one or two genes selected from COL3A1, TAC1, KLK5, CAMP, MMP9, TRPA1, IL13RA1, HSD3B1, CXCR4, ANGPT2, CXCL2, CXCR5, and PSMB9.

If the skin condition to be diagnosed is stinging skin, the skin condition may be diagnosed using the RNA biomarker genes related to the stinging skin, and the biomarkers related to the stinging skin may include one or two genes selected from IVL, LOR, FLG, FLG2, PGF, CYR61, HLA-B, IGHA1, MMP3, RBP4, and G0S2.

If the skin condition to be diagnosed is acne cosmetica, the skin condition may be diagnosed using the RNA biomarker genes related to the acne cosmetica, and the biomarkers related to the acne cosmetica may include one or two genes selected from MMP3, MMP12, CCR1, AKR1B10, THY1, and IL-6.

Further, the minimally invasive kit according to the present invention may include a device for extracting RNA from the skin specimen of the subject. The RNA acquired by the extracting device is amplified and thus provided to the quantifying device.

In addition, the minimally invasive kit according to the present invention may further include other components.

**Advantageous Effects**

The usefulness of the biomarkers for diagnosing the respective skin conditions is revealed under the minimally invasive skin specimen acquiring method according to the present invention. The biomarkers useful in diagnosing, evaluating, or measuring the respective skin conditions will be explained in detail in embodiments of the present invention as will be discussed later.

With the biomarkers, mRNAs of any one or more genes or levels of protein thereof can be measured. As it is checked that the genes or the levels of protein thereof increase or decrease in the subject whose skin condition is measured, the increasing or decreasing level is measured and usefully used in measurement and evaluation of the skin condition of the subject.

According to the present invention, the problems occurring in the conventional skin type diagnosis method using the SNP array analysis in which DNA of a human specimen is used can be solved. In the conventional SNP array analysis, the DNA of the human specimen is used, but since DNA is determined at birth and does not change, it is hard to reflect the real skin conditions changing by various environmental factors. According to the present invention, further, the problems occurring in the conventional skin aging diagnosis methods using measurements using applicable devices to the human body, tape stripping, tissue biopsy, and ex vivo test using 3D artificial skin can be solved.

The conventional measurements using applicable devices to the human body are used to tomographically show the surface of the skin, so that it is hard that they are utilized in the study of specific mechanisms or they are difficult in scientifically explaining changes in specific factors.

In the case of the conventional tape stripping, materials attached to a tape are limited to the stratum corneum, and accordingly, the tape stripping fails to provide perfect results, so that it is just used as an auxiliary method.

In the case of the conventional tissue biopsy, a surgeon's skill is needed, and a patient's pain occurs, so that the tissue biopsy causes the patient's reluctance and the scar on his or her skin remains after the test.

The conventional ex vivo test using the 3D artificial skin is high in price and does not include structures existing in skin, such as blood vessels, hair follicles, and subcutaneous fat, and accordingly, the ex vivo test does not reflect changes in skin actually, so that the skin biopsy cannot be replaced thereby.

According to the present invention, a new minimally invasive skin condition measuring kit and biomarkers for measuring skin conditions are provided so that a patient rarely feels pain, does not have any scar, and has many conveniences in use, unlike the existing tissue biopsy, and further, the skin factors in the stratum corneum are collected to enhance the reliability of the analysis results when compared with the existing tape stripping.

If the kit or biomarkers of the present invention is (are) utilized, the kit or biomarkers may be used in developing and screening specific skin condition inducing or inhibiting materials and treatment agents, in providing skin type information of individuals accurately, and in developing customized cosmetics under scientific classification in the skin types for individuals, in a more efficient way when compared with the conventional technologies.

According to the present invention, further, a new method for evaluating efficacy in the application to the human body is provided so that animal tests can be replaced in developing general cosmetics, functional cosmetics, medical devices, and medicines for improving skin conditions.

**Brief Description of Drawings**

FIG. 1 shows a conventional skin biopsy method.

FIG. 2 shows a concept view of a skin biopsy method according to the present invention.

FIG. 3 shows high-resolution photographs related to the measurements of the wrinkles around the eyes.

FIGs. 4 and 5 show the microarray procedure introduced in the manual of GeneChip® Human Gene 2.0 ST Array (Affymetrix) platform.

FIG. 6 shows gene alignment results performed using R 4.0.0 program.

FIG. 7 shows a table in which the genes related to collagen or elasticity among skin aging factors are selected and listed.

FIG. 8 shows a table in which the genes related to skin barrier function or water synthesis among the skin aging factors are selected and listed.

FIGs. 9 to 12 show heatmap analysis results so as to visually compare and analyze the expression levels of the skin aging biomarker candidates selected through the RNA microarray analysis.

FIGs. 13 to 20 show the results of bivariate correlation analysis conducted to check the correlation between the skin aging biomarker candidates selected through the RNA microarray analysis and the devices (PRIMOS, Cutometer, Corneometer, Tewameter, and Skin-pHmeter) applicable to the human body.

FIG. 21 shows high-resolution photographs taken using PRIMOS.

FIG. 22 shows the roughness of the wrinkles around the eyes measured using PRIMOS.

FIG. 23 shows the skin elasticity measured using Cutometer.

FIG. 24 shows the water content measured using Corneometer.

FIG. 25 is a table showing collagen/elasticity-related skin aging efficacy evaluation biomarker candidates.

FIG. 26 is a table showing skin barrier function/water synthesis-related skin aging efficacy evaluation biomarker candidates.

FIG. 27 shows a heatmap representing the expression of the collagen/elasticity-related skin aging efficacy evaluation biomarker candidates using the RNA microarray analysis results for the subjects subjected to skin aging efficacy evaluation.

FIG. 28 shows a heatmap representing the expression of the skin barrier function/water synthesis-related skin aging efficacy evaluation biomarker candidates.

FIGs. 29 to 32 show the results of bivariate correlation analysis conducted to check the correlation between the skin aging biomarker candidates selected through the skin aging efficacy evaluation RNA microarray analysis and the devices (PRIMOS, Cutometer, and Corneometer) applicable to the human body.

FIG. 33 shows gene alignment results.

FIG. 34 shows the summary of the gene expression values checked through the microarray test results for the genes related to skin hydration, through document analysis.

FIGs. 35 to 38 show heatmap analysis results so as to visually compare and analyze the expression levels of the skin hydration biomarker candidates selected through the RNA microarray analysis.

FIGs. 39 to 45 show the results of bivariate correlation analysis conducted to check the correlation between the skin hydration biomarker candidates selected through the RNA microarray analysis and the measurement results of the devices (Corneometer, Tewameter, and Skin-pHmeter) applicable to the human body.

FIG. 46 shows skin hydration efficacy evaluation results.

FIG. 47 is a table showing the gene expression values obtained by selecting the genes having the same tendency as the skin hydration (water content/water loss/skin pH) biomarkers of FIGs. 35 to 38 through the skin hydration efficacy evaluation RNA microarray analysis.

FIG. 48 is a graph showing visual comparison and analysis results of the expression levels of the skin hydration efficacy evaluation biomarker candidates selected through skin hydration efficacy evaluation RNA microarray analysis.

FIG. 49 shows gene alignment results.

FIG. 50 is a table showing the summary of the gene expression values checked through the microarray test results for the genes related to skin pigmentation, through document analysis.

FIGs. 51 and 52 show heatmap analysis results so as to visually compare and analyze the expression levels of the skin pigmentation biomarker candidates selected through RNA microarray analysis.

FIGs. 53 to 58 show the results of bivariate correlation analysis conducted to check the correlation between the skin pigmentation biomarker candidates CAT, CLU, DSG1, GPNMB, GPX4, GSTM2, GSTP1, MC2R, MLANA, PAX3, SOX10, TFAP2A, TYR, and TYRP1 selected through the RNA microarray analysis and the measurement results of the devices (Spectrophotometer and Mexameter) applicable to the human body.

FIGs. 59 and 60 show the measurement results of skin colors using Spectrophotometer and the measurement results of melanin around eyes using Mexameter in skin whitening efficacy evaluation.

FIG. 61 is a table showing skin whitening efficacy biomarker candidates.

FIG. 62 shows heatmap analysis results so as to visually compare and analyze the expression levels of the skin whitening efficacy evaluation biomarker candidates selected through skin whitening efficacy evaluation RNA microarray analysis.

FIGs. 63 and 64 show the analysis results of the correlation between the skin whitening biomarker candidates selected through the RNA microarray analysis for the subjects subjected to the skin whitening efficacy evaluation and the applicable devices to the human body.

FIG. 65 shows gene alignment results.

FIG. 66 is a table showing the gene expression values checked through the microarray test results for the genes related to the skin sebum through document analysis.

FIG. 67 shows heatmap analysis results so as to visually compare and analyze the expression levels of the skin sebum biomarker candidates selected through the RNA microarray analysis.

FIGs. 68 and 69 show the results of bivariate correlation analysis conducted to check the correlation between the skin sebum biomarker candidates selected through the RNA microarray analysis and the measurement results of the device (Sebumeter) applicable to the human body.

FIG. 70 shows gene alignment results.

FIG. 71 is a table showing the gene expression values checked through the microarray test results for the genes related to rosacea skin through document analysis.

FIG. 72 shows heatmap analysis results so as to visually compare and analyze the expression levels of the rosacea skin biomarker candidates selected through the RNA microarray analysis.

FIGs. 73 to 75 show the results of bivariate correlation analysis conducted to check the correlation between the rosacea skin biomarker candidates selected through the RNA microarray analysis and the measurement results of the devices (Mexameter and Spectrophotometer) applicable to the human body.

FIG. 76 shows a lactic acid sting test.

FIG. 77 shows gene alignment results.

FIG. 78 is a table showing the gene expression values checked through the microarray test results for the genes related to stinging skin through document analysis.

FIGs. 79 and 80 show heatmap analysis results so as to visually compare and analyze the expression levels of the stinging skin biomarker candidates selected through the RNA microarray analysis.

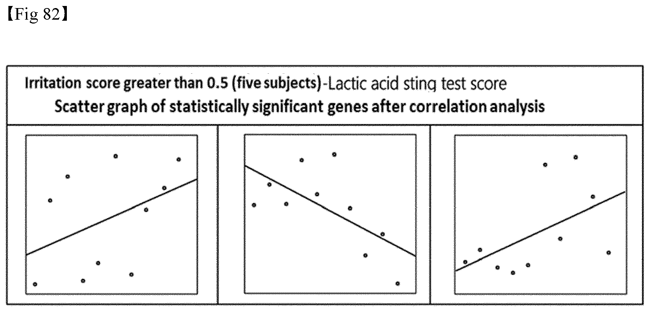

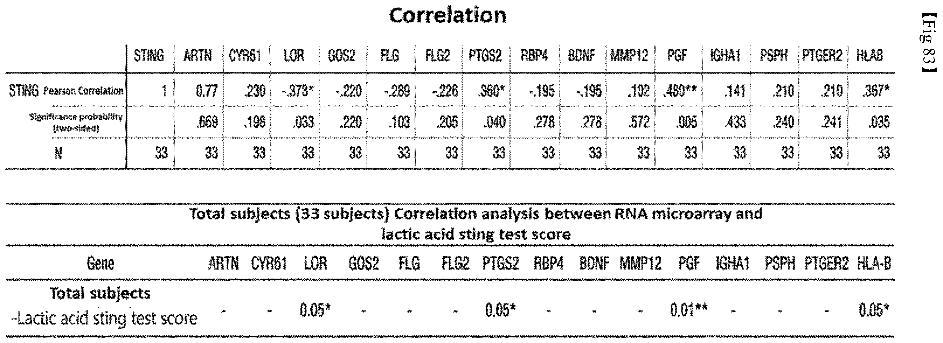

FIGs. 81 to 84 show the results of bivariate correlation analysis conducted to check the correlation between the stinging skin biomarker candidates selected through the RNA microarray analysis and lactic acid sting test scores.

FIG. 85 shows gene alignment results conducted using R 4.0.0 program.

FIG. 86 is a table showing the gene expression values checked through the microarray test results for the genes related to acne through document analysis.

FIG. 87 shows heatmap analysis results so as to visually compare and analyze the expression levels of the acne cosmetica biomarker candidates selected through the RNA microarray analysis after total ten subjects including five test subjects having high acne cosmetica activity and five control subjects having low acne cosmetica activity and the lactic acid sting test score less than or equal to 0.5 had been selected according to the physical findings of a dermatologist.

FIGs. 88 to 94 show the correlation analysis results between the expression levels of genes and the physical findings.

FIGs. 95 to 98 show the correlation analysis results between the expression levels of genes and the oily scores according to visual determination.

FIGs. 99 to 101 show the correlation analysis results between the expression levels of genes and the measurement results of the device (Sebumeter) applicable to the human body.

**Mode for Invention**

Hereinafter, an explanation of embodiments of the present invention will be given in detail with reference to the attached drawings. Before the present invention is disclosed and described, it is to be understood that the disclosed embodiments are merely exemplary of the invention, which can be embodied in various forms. However, this does not limit the invention within specific embodiments and it should be understood that the invention covers all the modifications, equivalents, and replacements within the idea and technical scope of the invention.

Hereinafter, embodiments of the present invention are disclosed in detail with reference to the attached drawings so as to allow them to be easily carried out by a person having ordinary skill in the technical scope of the invention.

FIG. 1 shows a conventional skin biopsy method, and FIG. 2 shows a concept view of a skin biopsy method according to the present invention.

FIG. 2 shows a concept view of a skin biopsy method according to the present invention.

On the top of the left side of FIG. 2 is shown a microneedle patch, which has been already known to the art when the present invention is filed. Further, on the bottom of the left side of FIG. 2 is shown a state in which the microneedle patch is applied to skin, more specifically a state in which microneedles of the microneedle patch are inserted into the dermis layer of skin. On the right side of FIG. 2 is conceptually shown the technical idea of the present invention in which after the microneedle patch has been applied to a subject, a biopsy is carried out by using proteins in the skin applied to the microneedles of the microneedle patch. A term bio-mining as used herein is used to describe a process of mining the proteins in the skin of the subject so as to carry out the biopsy.

The microneedle patch, which is used as a skin specimen collection means according to the present invention and made of a bioidegradable polymer hyaluronic acid, has a solid structure and is made by means of droplet extension (DEN), but according to the present invention, the microneedle patch may be made by means of other methods, for example, molding, without being limited thereto. Only if the microneedle patches, which are made of the same material and structure as one another, irrespectively of their manufacturing methods, they may provide minor differences in their skin specimen collection performance.

The inventor(s) have conducted clinical tests so as to produce biomarkers useful in measuring and evaluating skin conditions. Subjects of the clinical tests are selected as follows.

### Selecting subjects of clinical test

The inventors conducted clinical tests for healthy 33 subjects being 20 to 70 years old to discover the biomarkers related to their skin conditions and develop a method and kit for diagnosing the skin conditions by using the biomarkers. Table 1 shows clinical characteristics of the subjects.

[Table 1]

| The number of samples (subjects) | | 33 | |
|---|---|---|---|
| Male (subjects) | 19 | Female(subjects) | 14 |
| Age (Average $\pm$ SD) | | $37.12 \pm 12.90$ | |

In this case, the following persons were excluded in the subjects: (1) persons who did not agree to this study voluntarily after the approval of IRB so that they did not make an agreement; (2) persons who did not agree to the supply of human-derived materials; (3) Child-bearing or feeding women and fertile women who did not agree to contraceptive services determined in protocol; (4) persons who used external medicine containing steroid for one month or more so as to treat skin diseases; (5) persons who used oral steroid, oral antibiotics, and immunosuppressive drugs for recent one month; (6) patients who had target lesion areas infected (with fungi, germs, and viruses); (7) persons who had ultraviolet light treatment; (8) persons who had serious systemic diseases; (9) subjects (illiterate persons) who could not read and understand the agreement; and (10) persons who were not proper for the tests under the determination of the person in charge.

An explanation of the clinical tests will be given below according to the respective skin conditions diagnosed, evaluated or measured in the present invention.

### <Skin Aging>

### Selection of subject according to skin aging, specialty history taking, evaluation using device applicable to the human body, lactic acid sting test, and skin specimen collection

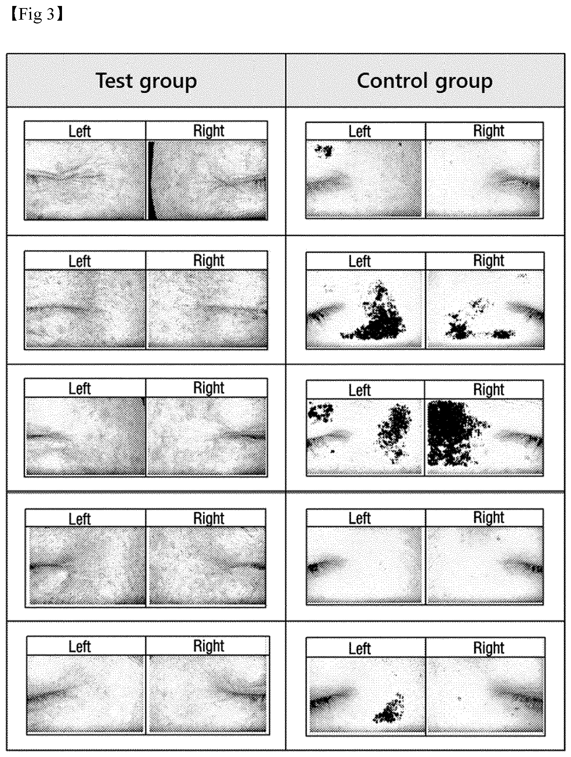

The subjects waited under constant temperature and humidity condition for 30 minutes after they washed their face, and next, the survey and specialty history taking for the subjects were conducted. After that, wrinkles around the eyes on normal facial expressions, skin elasticity, water content, transepidermal water loss, and skin acidity were measured. In specific, the wrinkles around the eyes were measured using a measurement device PRIMOS[CR], the skin elasticity using Cutometer® Dual MPA 580, the water content using Corneometer, the transepidermal water loss using Tewameter, and the skin acidity using Skin-pHmeter. Among them, High-resolution photographs as shown in FIG. 3 may be referred with respect to the measurements of the wrinkles around the eyes. The wrinkles around the eyes were quantified with roughness (Ra) values, the skin elasticity with R2 values, the water content with A.U. values, the transepidermal water loss with $g/h/m^2$ values, and the skin acidity with pH values.

The physical findings of the wrinkles around the eyes of the subjects were obtained by the visual determination of a dermatologist on the basis of visual evaluation standards of test method guide line amendment for labels, advertisements and regulations of cosmetics.

Table 2 shows the skin aging results for five subjects (control group) in their twenties and five subjects (test group) in their fifties or more, which were obtained through the devices applicable to the human body.

[Table 2]

| | Test group (who is in the fifties or more) | Control group(who in the twenties) |
|---|---|---|
| The number of samples (subjects) | 5 | 5 |

(continued)

|  | Test group (who is in the fifties or more) | Control group(who in the twenties) |
|---|---|---|
| Age (average $\pm$ SD) | 60.80 $\pm$ 5.72 | 24.20 $\pm$ 1.15 |
| physical findings (score) | 3.60 $\pm$ 0.84 | 0.80 $\pm$ 0.79 |
| Wrinkle around eyes (Ra) | 32.10 $\pm$ 3.69 | 18.24 $\pm$ 3.23 |
| Skin elasticity (R2) | 0.61 $\pm$ 0.06 | 0.76 $\pm$ 0.02 |
| Water content (A.U.) | 51.06 $\pm$ 18.61 | 61.74 $\pm$ 11.58 |
| Transepidermal water loss (g/h/m$^2$) | 17.07 $\pm$ 7.21 | 15.71 $\pm$ 5.34 |
| Skin acidity (pH) | 4.89 $\pm$ 0.40 | 5.26 $\pm$ 0.47 |

Further, after the microneedle patch was attached to each subject's face for 15 minutes, a skin specimen was collected.

### Collection of RNA from skin specimen

The skin specimens collected from the subjects were provided to RNeasy® Plus Mini Kit (Qiagen), and high purity RNA was extracted using the kit. Next, quality control (QC) of RNA samples was checked using Nanodrop and 2100 Bioanalyzer devices, and then, RNA microarray analysis was conducted.

### RNA microarray analysis

The samples were subjected to flexible and high sensitive pre-treatment using GeneChip® WT Pico Kit (Applied Biosystems) through which a small amount of total RNA (100 pg) separated by the above-mentioned method was amplified to cRNA. The amplified RNA was subjected to microarray analysis according to the manual of GeneChip® Human Gene 2.0 ST Array (Affymetrix) platform. An explanation of the amplification will be given in detail below.

The separated RNA was amplified using the GeneChip® WT Pico Kit (Applied Biosystems), and the amplification was carried out according to the test methods suggested by the manufacturing company. With mRNA used as template, T7 promoter sequence was synthesized with first strand cDNA. Next, the RNA was dissolved using DNA polymerase and RNase H, and a single-stranded cDNA was synthesized using 3' Adaptor. Double-stranded cDNA serving as the template was synthesized using Taq DNA polymerase and primers by adaptor. After that, antisense RNA (cRNA) was synthesized and amplified using T7 RNA polymerase in vitro transcription (IVT) and then purified using antisense cRNA purification beads. The purified antisense RNA was used as the template for reverse transcription to thus produce the single-stranded cDNA in a sensing direction. Next, the sense cDNA was fragmented and biotin-labeled with Terminal deoxynucleotidyl transferase (TdT) using GeneChip® WT Terminal Labeling kit (Affymetrix). Approximately 5.5 $\mu$g of labeled DNA target was subjected to microarray analysis according to the manual of the GeneChip® Human Gene 2.0 ST Array (Affymetrix) platform.

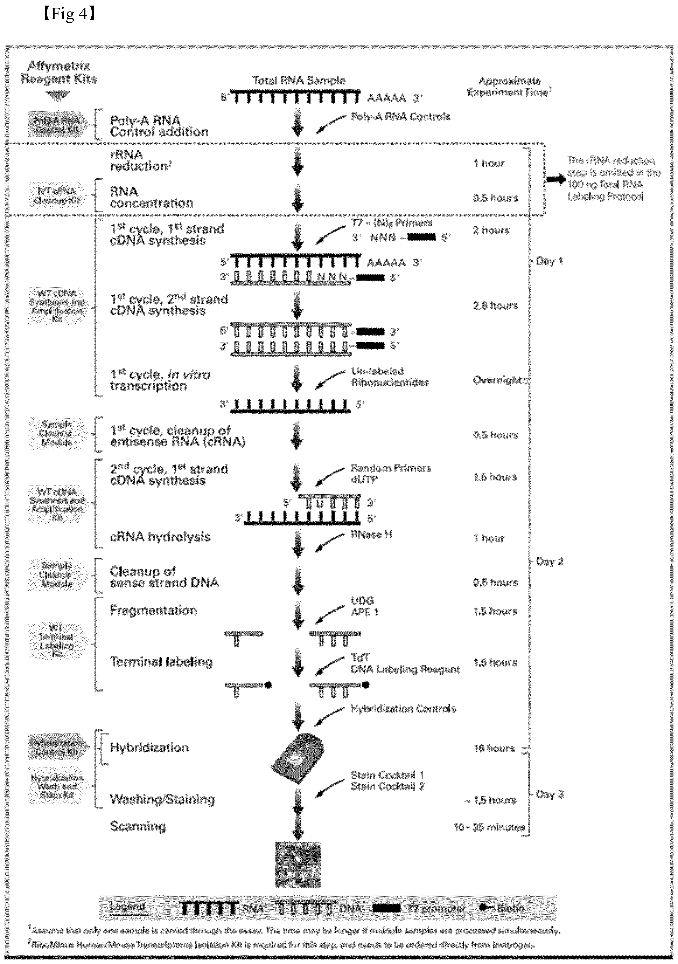

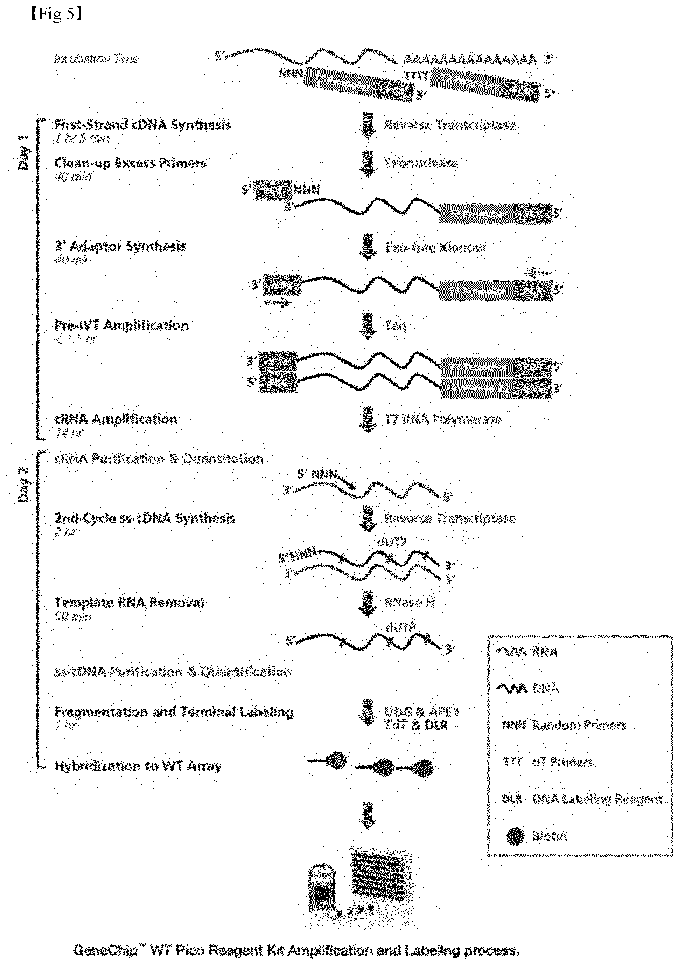

FIGs. 4 and 5 show the microarray procedure introduced in the manual of the platform. Table 3 shows the summary of the RNA microarray analysis protocols of the platform.

[Table 3]

| Sample type | Total RNA |
|---|---|
| Platform | GeneChip® Human Gene 2.0 ST Array |
| cDNA synthesize | cDNA was synthesized using the GeneChip WT (Whole Transcript) Amplification kit as described by the manufacturer. |
| Label protocol | The sense cDNA was then fragmented and biotin-labeled with TdT (terminal deoxynucleotidyl transferase) using the GeneChip WT Terminal labeling kit |
| Hybridization protocol | Approximately 5.5 $\mu$g of labeled DNA target was hybridized to the Affymetrix GeneChip Array at 45°C for 16hour |
| Scan protocol | Hybridized arrays were washed and stained on a GeneChip Fluidics Station 450 and scanned on a GCS3000 Scanner (Affymetrix). |

(continued)

| Sample type | Total RNA |
|---|---|
| Data processing | Array data export processing and analysis was performed using Affymetrix® GeneChip Command Console® Software (AGCC) |

After that, the entire data were summarized and normalized using Robust Multi-Average (RMA) of Affymetrix® Power Tools (APT), and the results were summarized to gene levels and subjected to differentially expressed gene (DEG) analysis. The statistical significance of the data was analyzed using independent t-test and variations of fold change, and false discovery rate (FDR) was adjusted in p value using Benjamini-Hochberg algorithm. DEG set was subjected to Hierarchical cluster analysis using linkage and Euclidean distance. Further, all data analysis and visualization work for checking gene expression differences were carried out using R 4.0.0 program.

**Selection of skin aging biomarker candidate through bioinformatics analysis and significant correlation analysis**

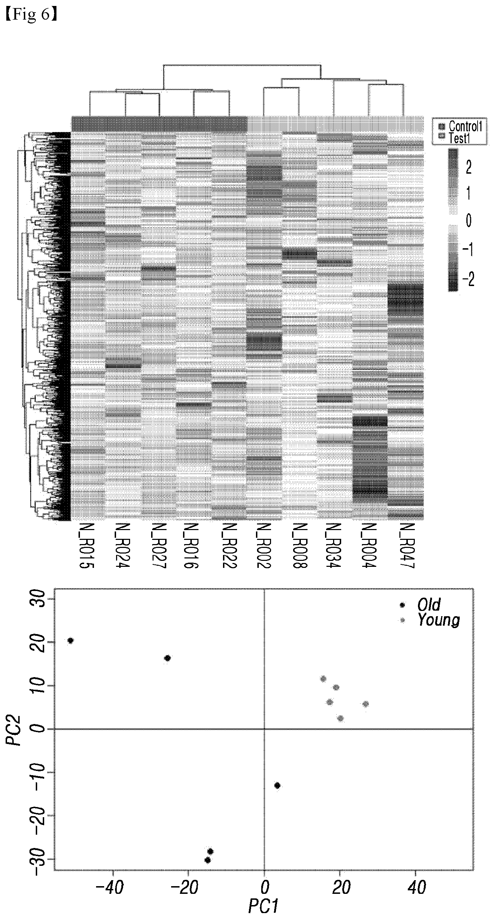

After the RNA microarray test was conducted, so as to check about forty thousand gene expression differences between the test group and the control group, gene alignment was performed using R 4.0.0 program. FIG. 6 shows gene alignment results. The gene alignment as shown in FIG. 6 is called heatmap analysis. The heatmap analysis is a term made by combining heat and map and represents various information expressed with colors in the form of heat distribution on an image. In the heatmap analysis described in the present invention, red colors mean gene expression values become high, and blue colors mean gene expression values become low. When hundreds of genes were analyzed, it was checked in FIG. 6 that there were specific differences between a plurality of gene expression values in the test group and the control group.

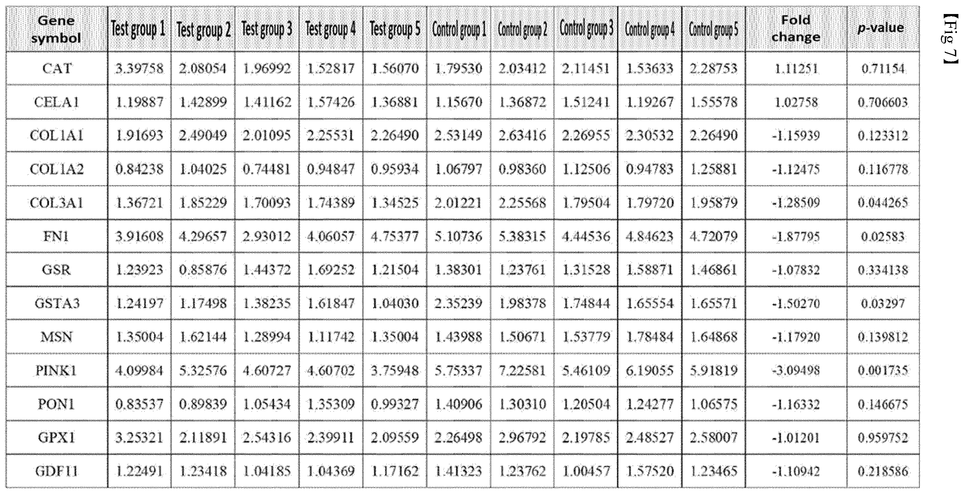

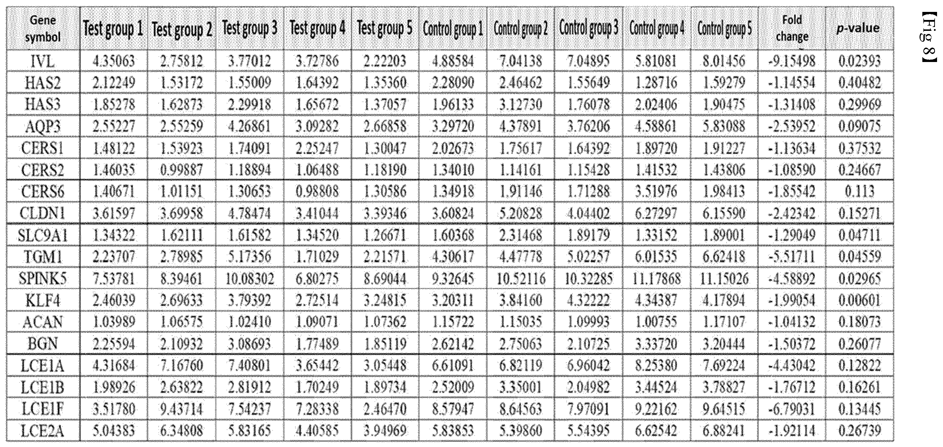

Through document analysis, after that, the inventors selected the genes related to collagen or elasticity among the skin aging factors (See FIG. 7), and they selected the genes related to skin barrier function or water synthesis among the skin aging factors (See FIG. 8). The inventors summarized the gene expression values checked through the microarray test results for the genes related to the skin aging, through the document analysis, on the tables as shown in the above figures.

In the test group in which the skin aging was developed and the control group in which the skin aging was not developed yet, the differentiation in the gene expression values obtained by performing the microarray had to exist, so that the values might be useful as biomarkers related to the skin aging. To determine this, the inventors made use of fold changes and p-values. The fold changes are values that represent the measured values in the test group were higher or lower several times than those of the control group, and the p-values are values between the test group and the control group that represent statistically significant differences in statistical processing. The normalized signal values (expression values) of the respective samples obtained through the RNA microarray tests are represented in the tables of FIGs. 7 and 8, and the fold changes are values obtained by calculating 1:1 expression differences using the normalized signal values between the test group and the control group. The p-values are the values between 0 and 1 that represent the degrees of data coexisting with null hypothesis, and if the p-values are less than or equal to 0.05, generally, the null hypothesis is rejected.

As a result, it was checked that the genes among the skin aging genes related to collagen/elasticity, which had the fold changes of 1.2 times or -1.2 times or the p-values less than or equal to 0.05, were COL3A1, FN1, GSTA3, and PINK1, and the genes among the skin aging genes related to skin barrier function/water synthesis, which had the fold changes of 1.2 times or -1.2 times or the p-values less than or equal to 0.05, were IVL, HAS3, AQP3, CERS6, CLDN1, SLC9A1, TGM1, SPINK5, KLF4, BGN, LCE1A, LCE1B, LCE1F, and LCE2A.

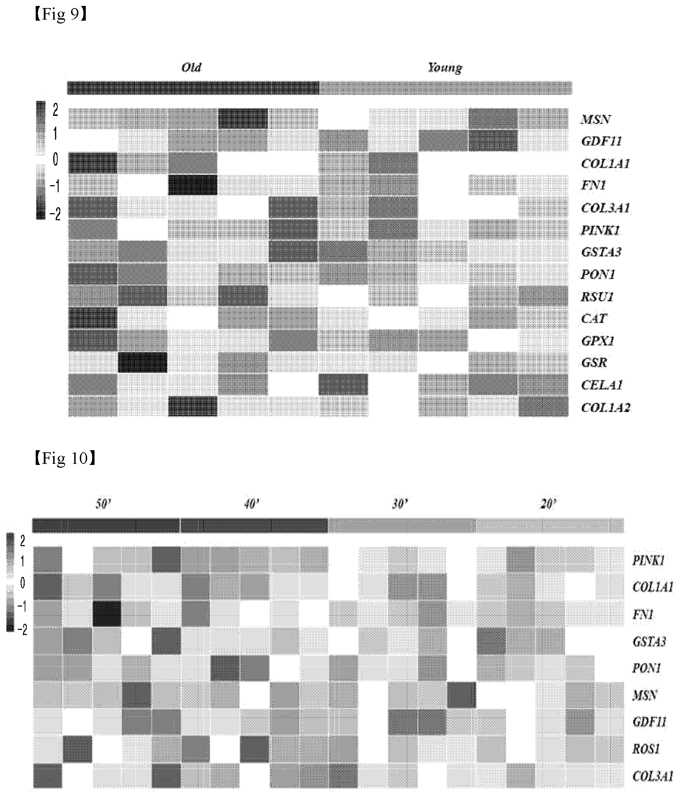

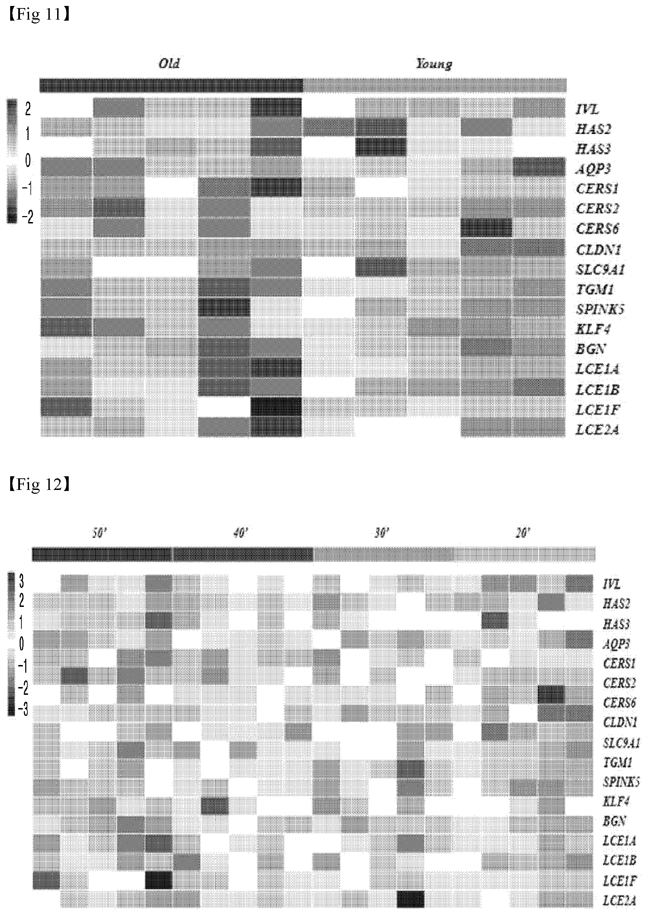

FIGs. 9 to 12 show heatmap analysis results so as to visually compare and analyze the expression levels of the skin aging biomarker candidates selected through the RNA microarray analysis. FIG. 9 is a heatmap showing the gene expressions of the collagen/elasticity-related skin aging biomarker candidates using the RNA microarray analysis results for the five test group subjects in the fifties or more and the five control group subjects in the twenties, and FIG. 10 is a heatmap showing the gene expressions of the collagen/elasticity-related skin aging biomarker candidates using the RNA microarray analysis results for total 20 subjects including five subjects by twenties, thirties, forties, and fifties. FIG. 11 is a heatmap showing the gene expressions of the skin barrier function/water synthesis-related skin aging biomarker candidates using the RNA microarray analysis results for the five test group subjects in the fifties or more and the five control group subjects in the twenties, and FIG. 12 is a heatmap showing the gene expressions of the skin barrier function/water synthesis-related skin aging biomarker candidates using the RNA microarray analysis results for total 20 subjects including five subjects by twenties, thirties, forties, and fifties.

In the same manner as in FIG. 6, in the heatmap analysis as shown in FIGs. 9 to 12, red colors mean gene expression values become high, and blue colors mean gene expression values become low. From the test results as shown in FIGs. 9 to 12, it was visually checked that the specific gene expression values selected through the document analysis in the test group were higher with red colors than those in the control group.

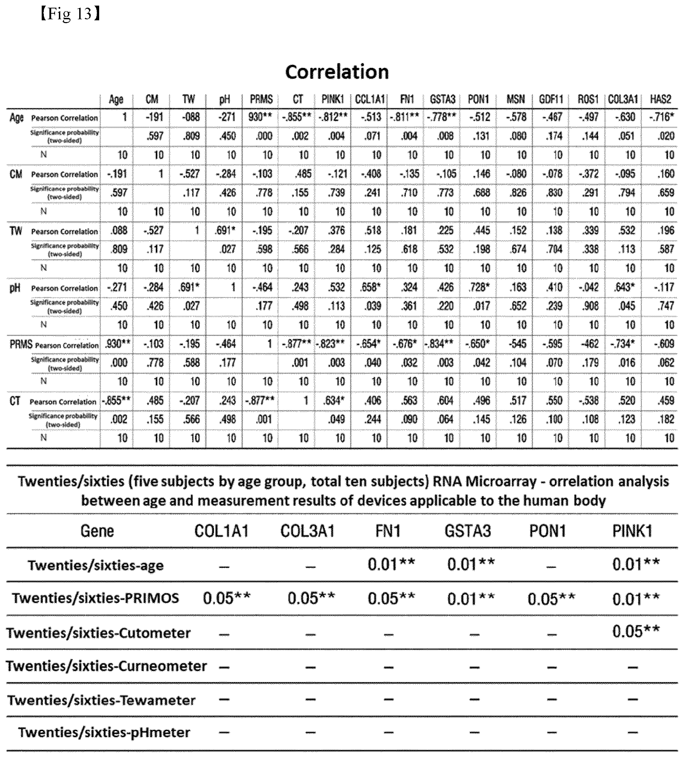

To check the correlation between the skin aging biomarker candidates selected through the RNA microarray analysis and the measurement results of the devices (PRIMOS, Cutometer, Corneometer, Tewameter, and Skin-pHmeter) applicable to the human body, bivariate correlation analysis was conducted. The results are shown in FIGs. 13 to 20.

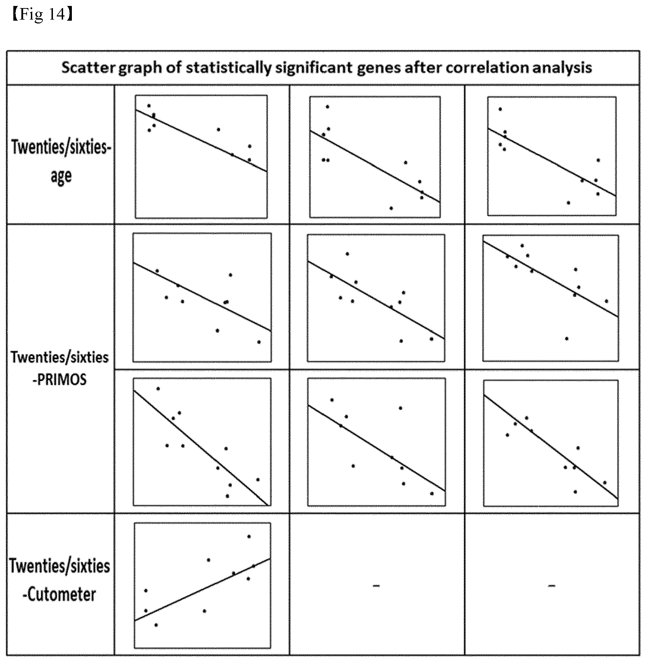

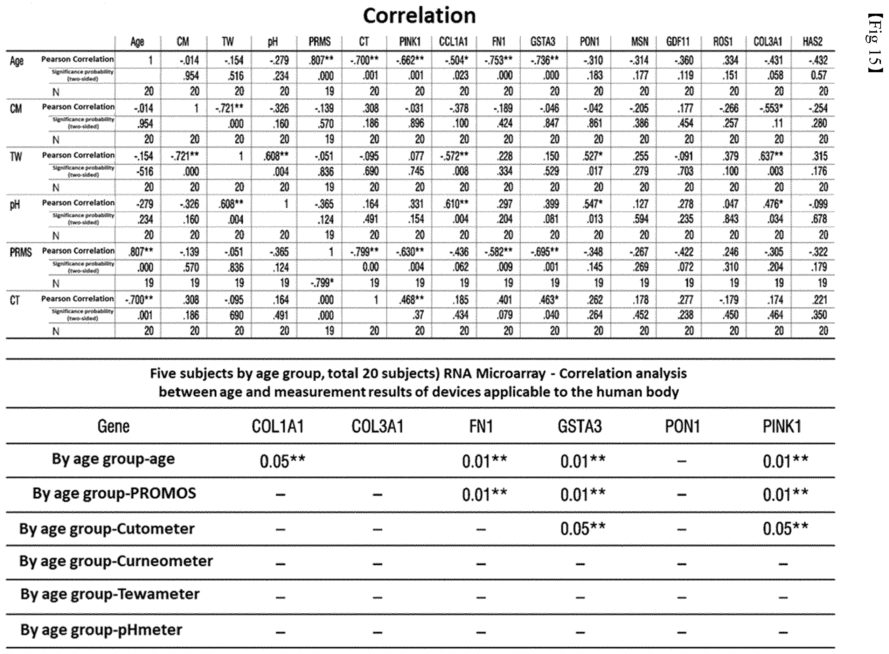

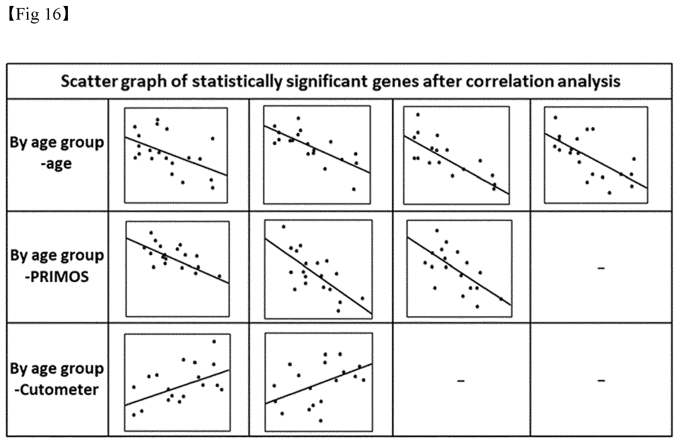

FIGs. 13 and 14 show the correlation between collagen/elasticity-related skin aging biomarker candidates selected through the RNA microarray analysis for five test subjects who are in the fifties or more and five control subjects who are in the twenties and the age/the devices applicable to the human body, and FIGs. 15 and 16 show the correlation between collagen/elasticity-related skin aging biomarker candidates selected through the RNA microarray analysis for total 20 subjects including five subjects by age group and the age/the devices applicable to the human body.

As a result, it was checked that the FN1, GSTA3, and PINK1 genes of the RNA microarray analysis results for total 10 subjects including the five test subjects who are in the fifties or more and the five control subjects who are in the twenties had statistically significant negative correlation with the age of the subjects, the COL1A1, COL3A1, FN1, GSTA3, PON1, and PINK1 genes had statistically significant negative correlation with the measurement results of the PRIMOS device measuring the wrinkles around the eyes, and the PINK1 gene had statistically significant positive correlation with the measurement results of the Cutometer device measuring skin elasticity. In FIG. 13, the respective genes are omitted, but the explanation of FIG. 13 is given in order from the left side of top of FIG. 13. In specific, the results of the FN1, GSTA3, and PINK1 genes having the statistically significant negative correlation with the age of the subjects are shown in order from the left side of a first row of FIG. 13, the COL1A1, COL3A1, FN1, GSTA3, PON1, and PINK1 genes having the statistically significant negative correlation with the measurement results of the PRIMOS device measuring the wrinkles around the eyes are shown in order from the left side of the middle row of FIG. 13, and the PINK1 gene having the statistically significant positive correlation with the measurement results of the Cutometer device measuring skin elasticity is shown in the last row of FIG. 14. In the scatter graph showing the correlation among the plurality of genes, as shown in FIG. 14, the genes are not represented in the graph, but the above-mentioned correlation will be applied to the graph in the same manner as above. That is, the correlation will be described in order from top of the left side of the graph.

Further, it was checked that the COL1A1, FN1, GSTA3, and PINK1 genes of the RNA microarray analysis results in total 20 subjects including the five subjects who are in the twenties, thirties, forties, and fifties by age group had statistically significant negative correlation with the age of the subjects, the FN1, GSTA3, and PINK1 genes had statistically significant negative correlation with the measurement results of the PRIMOS device measuring the wrinkles around the eyes, and the GSTA3 and PINK1 genes had statistically significant positive correlation with the measurement results of the Cutometer device measuring skin elasticity.

When the RNA microarray analysis results of the subjects and the measurement results of the devices applicable to the human body were totally analyzed, accordingly, total six genes, COL1A1, COL3A1, FN1, GSTA3, PON1, and PINK1 were selected as the collagen/elasticity-related skin aging biomarkers.

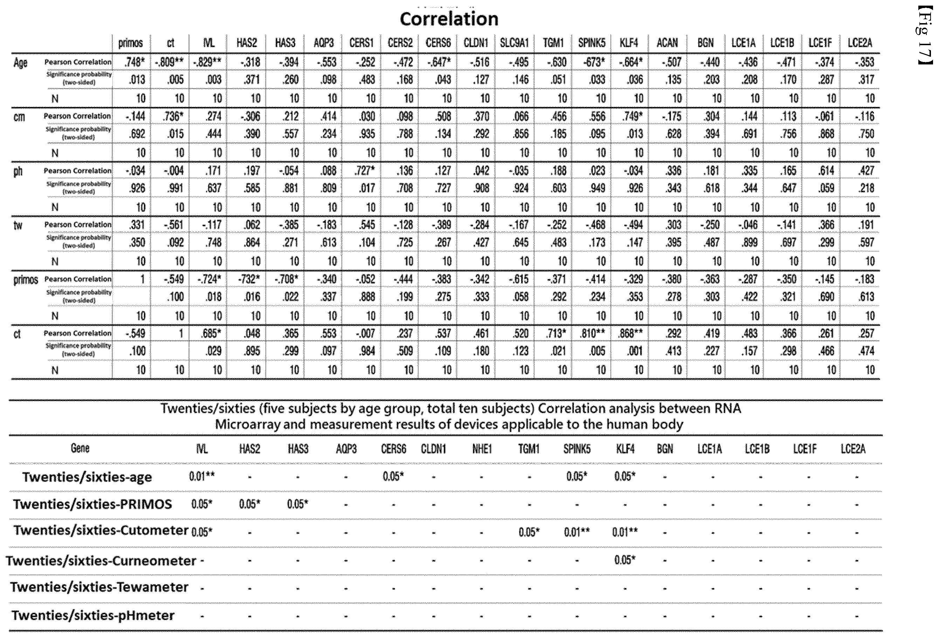

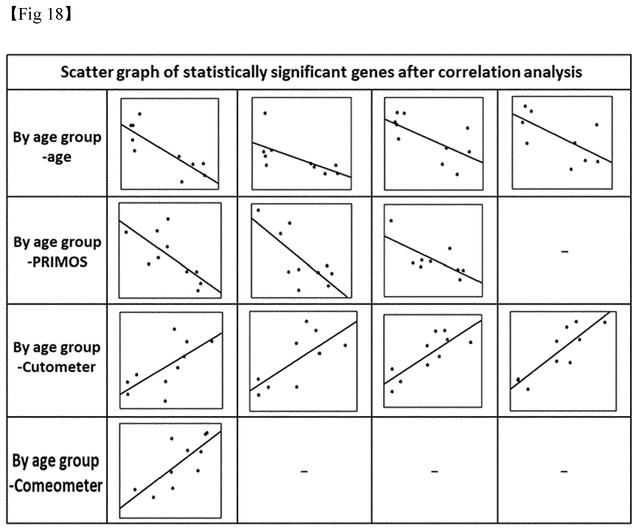

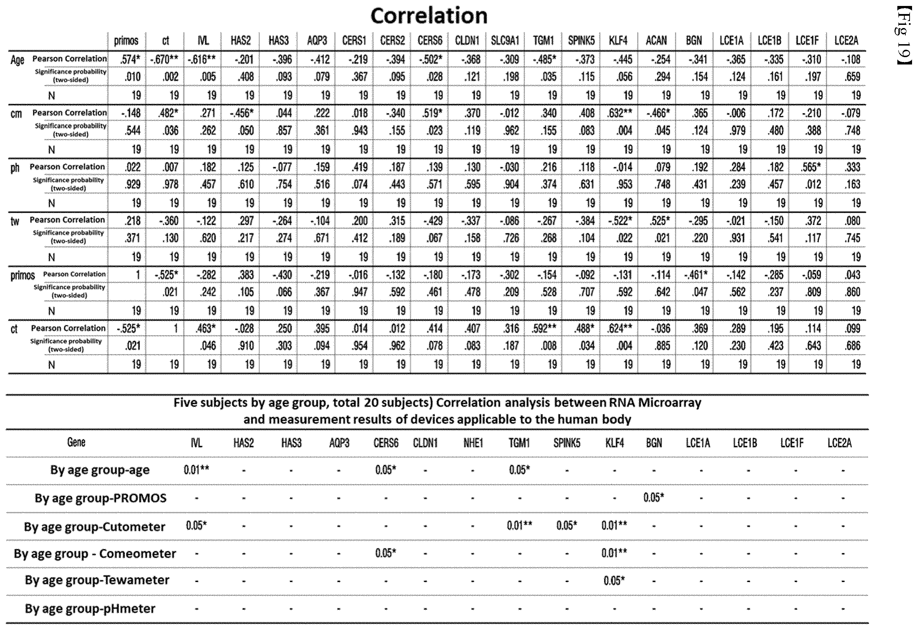

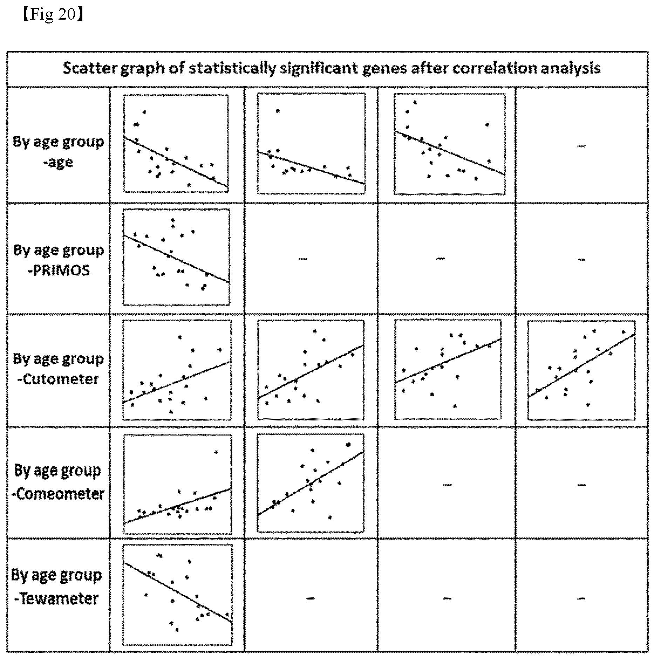

FIGs. 17 and 18 show the correlation between skin barrier function/water content-related skin aging biomarker candidates selected through the RNA microarray analysis in five test subjects who are in the fifties or more and five control subjects who are in the twenties and the age/the devices applicable to the human body, and FIGs. 19 and 20 show the correlation between skin barrier function/water content-related skin aging biomarker candidates selected through the RNA microarray analysis in total 20 subjects including five subjects who are in the twenties, thirties, forties, and fifties by age group and the age/the devices applicable to the human body.

As a result, it was checked that the IVL, CERS6, SPINK5, and KLF4 genes of the RNA microarray analysis results for total 10 subjects including the five test subjects who are in the fifties or more and the five control subjects who are in the twenties had statistically significant negative correlation with the age of the subjects, the IVL, HAS2, and HAS3 genes had statistically significant negative correlation with the measurement results of the PRIMOS device measuring the wrinkles around the eyes, and the IVL, TGM1, SPINK5, and KLF4 genes had statistically significant positive correlation with the measurement results of the Cutometer device measuring skin elasticity. Further, it was checked that the KLF4 gene had statistically significant positive correlation with the measurement results of the Corneometer device measuring water content.

Further, it was checked that the IVL, CERS6, and TGM1 genes of the RNA microarray analysis results for total 20 subjects including the five subjects who are in the twenties, thirties, forties, and fifties by age group had statistically significant negative correlation with the age of the subjects, the BGN gene had statistically significant negative correlation with the measurement results of the PRIMOS device measuring the wrinkles around the eyes, and the IVL, TGM1, SPINK5, and KLF4 genes had statistically significant positive correlation with the measurement results of the Cutometer device measuring skin elasticity. Further, it was checked that the KLF4 gene had statistically significant negative correlation with the measurement results of the Tewameter device measuring transepidermal water loss.

When the RNA microarray analysis results of the subjects and the measurement results of the devices applicable to the human body were totally analyzed, accordingly, total 15 genes, IVL, HAS2, HAS3, AQP3, CERS6, CLDN1, SLC9A1, TGM1, SPINK5, KLF4, LCE1A, LCE1B, LCE1F, LCE2A, and BGN were selected as the skin barrier function/water content-related skin aging biomarkers.

**Selection of collagen/elasticity-related and skin barrier function/water content-related skin aging biomarker through skin aging efficacy evaluation**

So as to verify whether the collagen/elasticity-related and skin barrier function/water content-related skin aging biomarkers selected in the above serve to measure and diagnose a degree of skin aging, to screen effective ingredients related to skin aging or anti-aging agents, to evaluate the skin aging before and after the use of general cosmetics, functional cosmetics, medical devices, medicines, and the like, and to verify whether they are used for the purpose of animal replacement tests, skin aging efficacy evaluation was carried out.

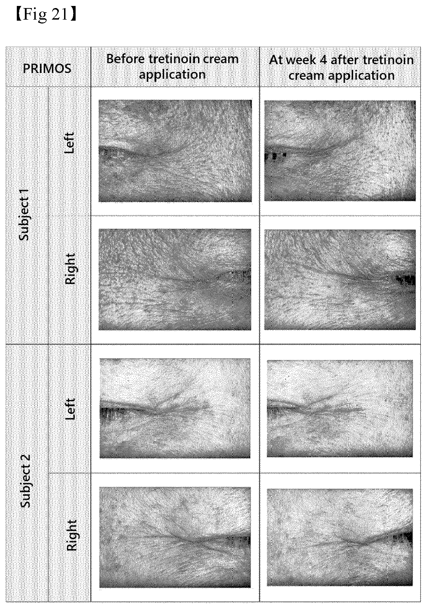

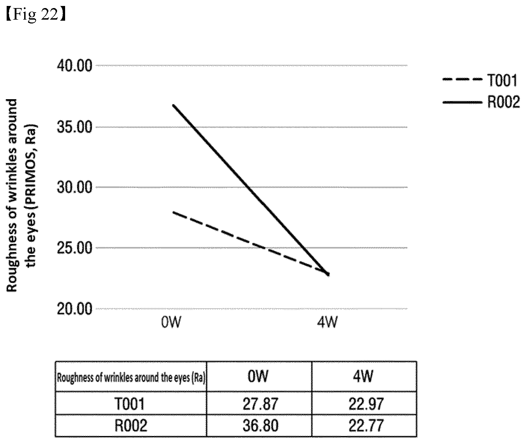

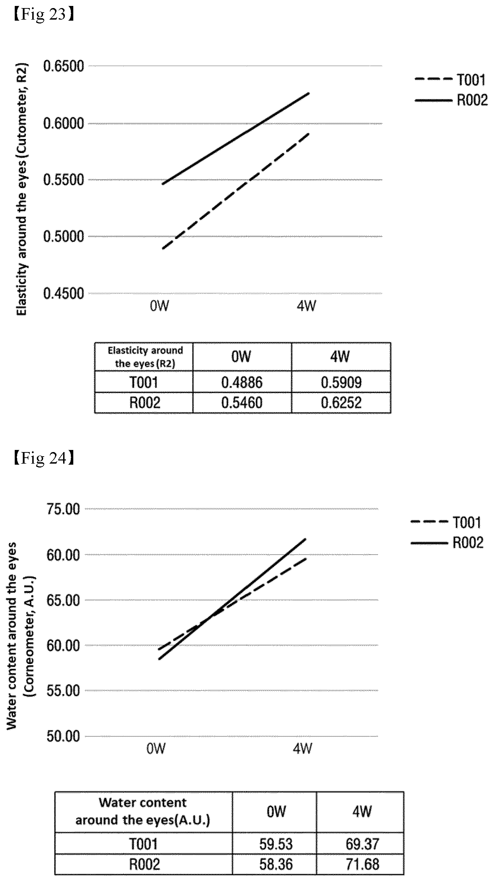

In specific, a tretinoin cream (Stieva-A® cream, 0.025%) was applied to the areas around the eyes of two subjects who are in the sixties or more for four weeks, and using the PRIMOS device, high-resolution photographs were taken (See FIG. 21) and roughness of the wrinkles around the eyes was measured (See Table 4 and FIG. 22) at week 0 and week 4. Further, skin elasticity was measured (See Table 5 and FIG. 23) using the Cutometer device, water content was measured (See Table 6 and FIG. 24) using the Corneometer device, and after the microneedle patch was attached to the areas around the eyes for 15 minutes, a skin specimen was collected to extract RNA therefrom and the RNA microarray was conducted.

[Table 4]

| Wrinkles around the eyes (Ra) | At week 0 | At week 4 |
|---|---|---|
| Subject 1 | 27.87 ± 2.67 | 22.97 ± 0.81 |
| Subject 2 | 36.80 ± 0.70 | 22.77 ± 2.76 |

[Table 5]

| Skin elasticity (R2) | At week 0 | At week 4 |
|---|---|---|
| Subject 1 | 0.4886 ± 0.0158 | 0.5909 ± 0.0245 |
| Subject 2 | 0.5460 ± 0.0430 | 0.6252 ± 0.0123 |

[Table 6]

| Water content (A.U.) | At week 0 | At week 4 |
|---|---|---|
| Subject 1 | 59.53 ± 2.29 | 69.37 ± 2.93 |
| Subject 2 | 58.36 ± 5.83 | 71.68 ± 4.66 |

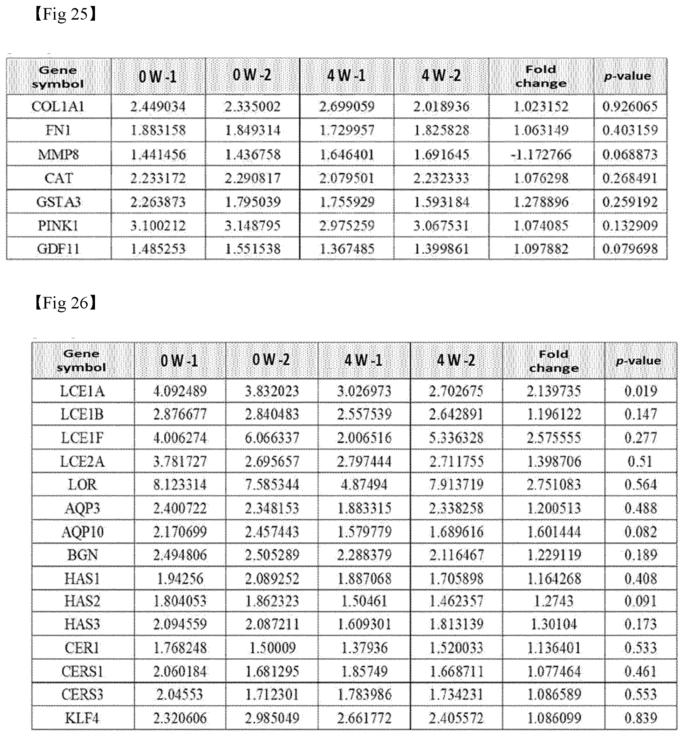

After the RNA microarray test was conducted, so as to check gene expression differences according to the skin aging efficacy evaluation, gene alignment was performed using R 4.0.0 program. After that, among the collagen/elasticity-related genes selected through the document analysis and the collagen/elasticity-related skin aging biomarkers (COL1A1, COL3A1, FN1, GSTA3, PON1, and PINK1), the genes having the same tendency as one another were selected and thus listed in the table of FIG. 25. Further, among the skin barrier function/water synthesis-related genes selected through the document analysis and the skin barrier function/water synthesis-related skin aging biomarkers (IVL, HAS2, HAS3, AQP3, CERS6, CLDN1, SLC9A1, TGM1, SPINK5, KLF4, LCE1A, LCE1B, LCE1F, LCE2A, and BGN), the genes having the same pattern as one another were selected and thus listed in the table of FIG. 26.

It was checked that the gene among the skin aging genes related to collagen/elasticity, as listed in the table of FIG. 25, which had the fold changes of 1.2 times or -1.2 times or the p-values less than or equal to 0.05, was GSTA3, and the genes among the skin aging genes related to skin barrier function/water synthesis, as listed in the table of FIG. 26, which had the fold changes of 1.2 times or -1.2 times or the p-values less than or equal to 0.05, were LEC1A, LCE1F, LCE2A, LOR, AQP3, AQP10, BGN, HAS2, and HAS3.

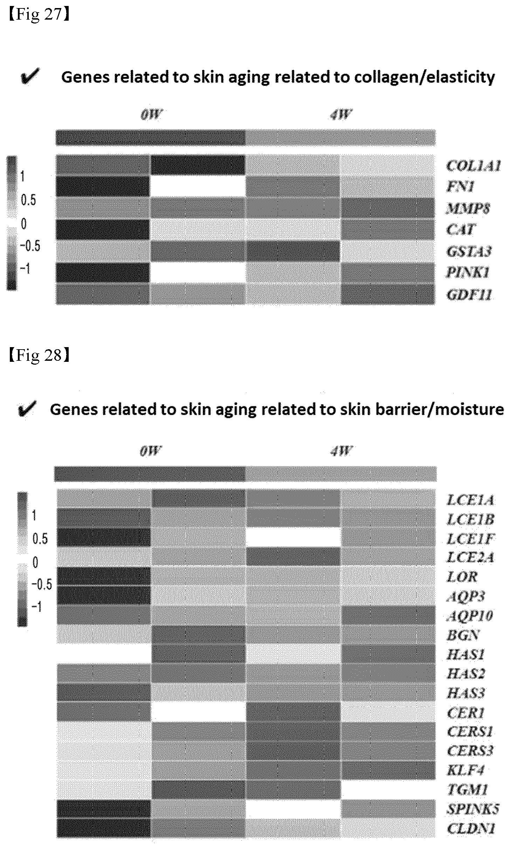

FIGs. 27 and 28 show heatmap analysis results so as to visually compare and analyze the expression levels of the skin aging efficacy evaluation biomarker candidates selected through the RNA microarray analysis. FIG. 27 shows a heatmap representing the expression of the collagen/elasticity-related skin aging efficacy evaluation biomarker candidates using the RNA microarray analysis results for the subjects who are subjected to the skin aging efficacy evaluation, and FIG. 28 shows a heatmap representing the expression of the skin barrier function/water synthesis-related skin aging efficacy evaluation biomarker candidates.

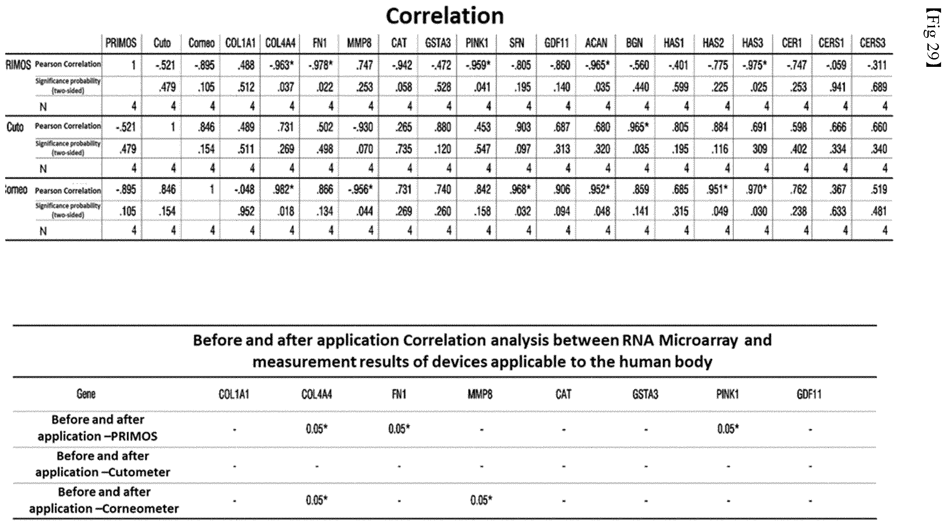

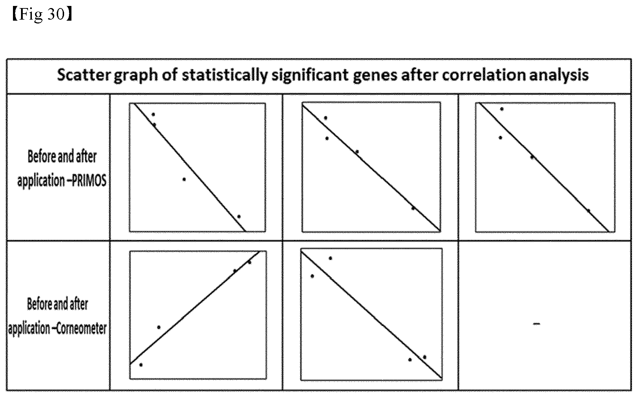

To check the correlation between the skin aging biomarker candidates selected through the skin aging efficacy evaluation RNA microarray analysis and the devices (PRIMOS, Cutometer, and Corneometer) applicable to the human body, bivariate correlation analysis was conducted by the inventors. The results are shown in FIGs. 29 to 32. FIGs. 29 and 30 show the correlation between collagen/elasticity-related skin aging biomarker candidates selected through the RNA microarray analysis for the subjects who are subjected to the skin aging efficacy evaluation and the devices applicable to the human body. As a result, it was checked that after the tretinoin cream was applied for 4 weeks, when compared to the week 0, the collagen/elasticity-related skin aging biomarkers, COL4A4, FN1, and PINK1 genes had statistically significant negative correlation with the measurement results of the PRIMOS device measuring wrinkles around the eyes, and the COL4A4 and MMP8 genes had statistically significant positive or negative correlation with the measurement results of the Corneometer device measuring water content. When the skin aging and the skin aging efficacy evaluation were totally analyzed, accordingly, total eight genes, COL1A1, COL3A1, FN1, GSTA3, PON1, and PINK1 genes as the skin aging biomarkers and the COL4A4 and MMP8 genes were selected as the collagen/elasticity-related skin aging efficacy evaluation biomarkers. Further, in view of accurate and effective skin condition diagnosis, the inventors found that it was advantageous when the COL1A1, COL3A1, FN1, and PINK1 genes among the genes were included in the biomarker group.

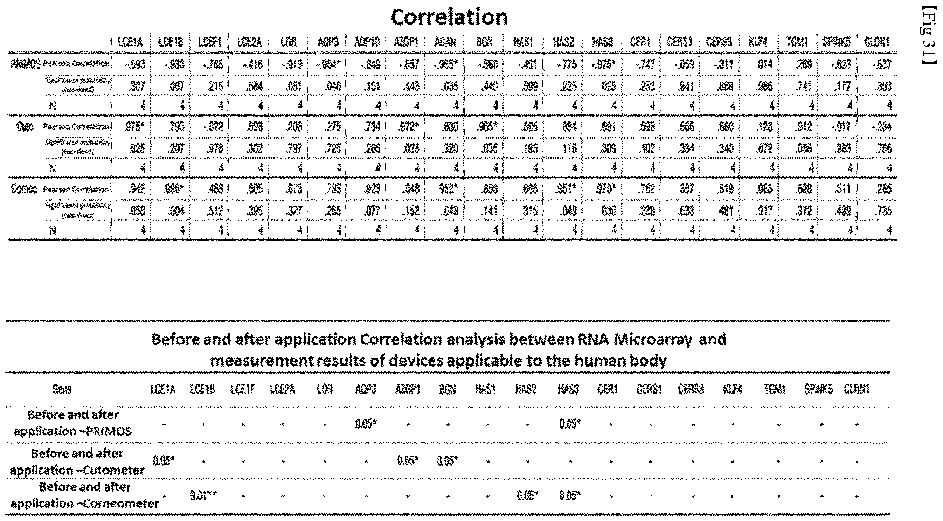

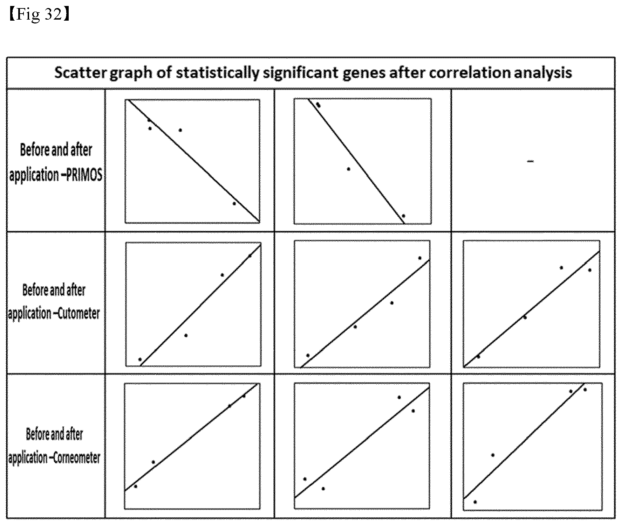

FIGs. 31 and 32 show the correlation between skin barrier function/water synthesis-related skin aging biomarker candidates selected through the RNA microarray analysis for the subjects who were subjected to the skin aging efficacy evaluation and the devices applicable to the human body. As a result, it was checked that after the tretinoin cream was applied for 4 weeks, when compared to week 0, the skin barrier function/water synthesis-related skin aging biomarkers, AQP3 and HAS3 genes had statistically significant negative correlation with the measurement results of the PRIMOS device measuring wrinkles around the eyes, the LCE1A, AZGP1, and BGN genes had statistically significant positive correlation with the measurement results of the Cutometer measuring skin elasticity, and the LCE1B, HAS2, and HAS3 genes had statistically significant positive correlation with the measurement results of the Corneometer device measuring water content.

When the skin aging and the skin aging efficacy evaluation were totally analyzed, accordingly, total 16 genes, the IVL, HAS2, HAS3, AQP3, CERS6, CLDN1, SLC9A1, TGM1, SPINK5, KLF4, LCE1A, LCE1B, LCE1F, LCE2A, and BGN genes as the skin aging biomarkers and the AZGP1 gene were selected as the skin barrier function/water synthesis-related skin aging efficacy evaluation biomarkers. Further, in view of accurate and effective skin condition diagnosis, the inventors found that it was advantageous when the IVL, HAS3, AQP3, BGN, and AZGP1 genes among the genes were included in the biomarker group.

In summary, the COL1A1, COL3A1, FN1, GSTA3, PON1, PINK1, COL4A4, and MMP8 genes were selected as the collagen/elasticity-related skin aging biomarkers, and the genes IVL, HAS2, HAS3, AQP3, CERS6, CLDN1, SLC9A1, TGM1, SPINK5, KLF4, LCE1A, LCE1B, LCE1F, LCE2A, BGN, and AZGP1 genes were selected as the skin barrier function/water synthesis-related skin aging biomarkers. As a result, the selected biomarkers may be used in a skin aging diagnosis method or kit, in developing and screening skin aging inducing or inhibiting materials and anti-aging agents, in providing skin type information of individuals, in developing customized cosmetics, and in evaluating animal replacement skin aging efficacy.

**<Skin hydration>**

**Selection of subject according to skin hydration (water content/water loss/skin pH), specialty history taking, evaluation through device applicable to the human body, and skin specimen collection**

The subjects waited under constant temperature and humidity condition for 30 minutes after they washed their face, and next, the survey and specialty history taking for the subjects were conducted. After that, water content, transepidermal water loss, and skin pH were measured around the cheeks of each subject. In specific, the water content was measured using Corneometer® CM825, the transepidermal water loss using Tewameter® TM300, and the skin pH using Skin-pHmeter PH905. The water content was quantified with arbitrary unit (A.U.) values, the transepidermal water loss with $g/h/m^2$ values, and the skin pH with pH values.

The physical findings of the skin hydration of the subjects were obtained by the visual determination of a dermatologist on the basis of a document (Int J Dermatol. 2017 Yoshida-Amano et al).

Table 7 shows the skin hydration degree evaluation results for five control subjects who did not have dry skin) and five test subjects who had dry skin, which were selected among the total subjects, based on the measurement results of the above-mentioned three devices applicable to the human body.

[Table 7]

|  | Test group (dry) | Control group |
|---|---|---|
| The number of samples (subjects) | 5 | 5 |
| Age (average $\pm$ SD) | 32.60 $\pm$ 6.99 | 44.80 $\pm$ 20.89 |
| Physical findings (score) | 1.20 $\pm$ 0.84 | 0.60 $\pm$ 0.55 |
| Water content (A.U.) | 38.41 $\pm$ 8.78 | 65.81 $\pm$ 5.97 |
| Transepidermal water loss (g/h/m$^2$) | 24.12 $\pm$ 3.92 | 10.69 $\pm$ 1.57 |
| Skin acidity (pH) | 5.42 $\pm$ 0.43 | 4.72 $\pm$ 0.30 |

Further, after the microneedle patch was attached to each subject's face for 15 minutes, a skin specimen was collected.

**Collection of RNA from skin specimen**

The skin specimens collected from the subjects were provided to RNeasy® Plus Mini Kit (Qiagen), and high purity RNA was extracted using the kit. Next, quality control (QC) of RNA samples was checked using Nanodrop and 2100 Bioanalyzer devices, and then, RNA microarray analysis was conducted.

**RNA microarray analysis**

The samples were subjected to flexible and high sensitive pre-treatment using GeneChip® WT Pico Kit (Applied Biosystems) through which a small amount of total RNA (100 pg) separated by the above-mentioned method was amplified to cRNA. The amplified RNA was subjected to microarray analysis according to the manual of GeneChip® Human Gene 2.0 ST Array (Affymetrix) platform. The microarray procedure introduced in the manual of platform was shown in FIGs. 4 and 5. The inventors conducted the microarray in the same procedure. The RNA microarray analysis protocols of the platform were the same as in Table 3.

After that, the entire data were summarized and normalized using Robust Multi-Average (RMA) of Affymetrix® Power Tools (APT), and the results were summarized to gene levels and subjected to differentially expressed gene (DEG) analysis. The statistical significance of the data was analyzed using independent t-test and variations of fold change, and false discovery rate (FDR) was adjusted in p value using Benjamini-Hochberg algorithm. DEG set was subjected to Hierarchical cluster analysis using linkage and Euclidean distance. Further, all data analysis and visualization work for checking gene expression differences were carried out using R 4.0.0 program.

**Selection of skin hydration (water content/water loss/skin pH) biomarker candidate through bioinformatics analysis and significant correlation analysis**

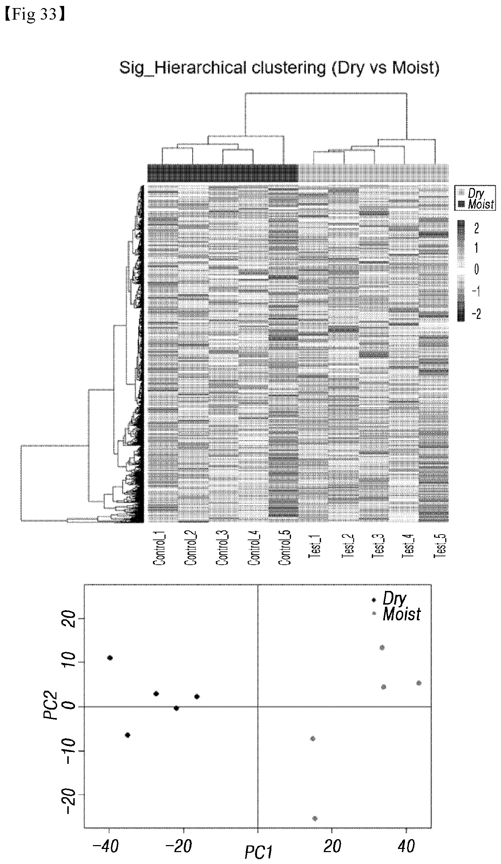

After the RNA microarray test was conducted, so as to check about forty thousand gene expression differences between the test group and the control group, gene alignment was performed using R 4.0.0 program. FIG. 33 shows gene alignment results. The gene alignment as shown in FIG. 33 is called heatmap analysis. The heatmap analysis is a term made by combining heat and map and represents various information expressed with colors in the form of heat distribution on an image. In the heatmap analysis described in the present invention, red colors mean gene expression values become high, and blue colors mean gene expression values become low. When hundreds of genes were analyzed, it was checked in FIG. 33 that there were specific differences between a plurality of gene expression values in the test group and the control group.

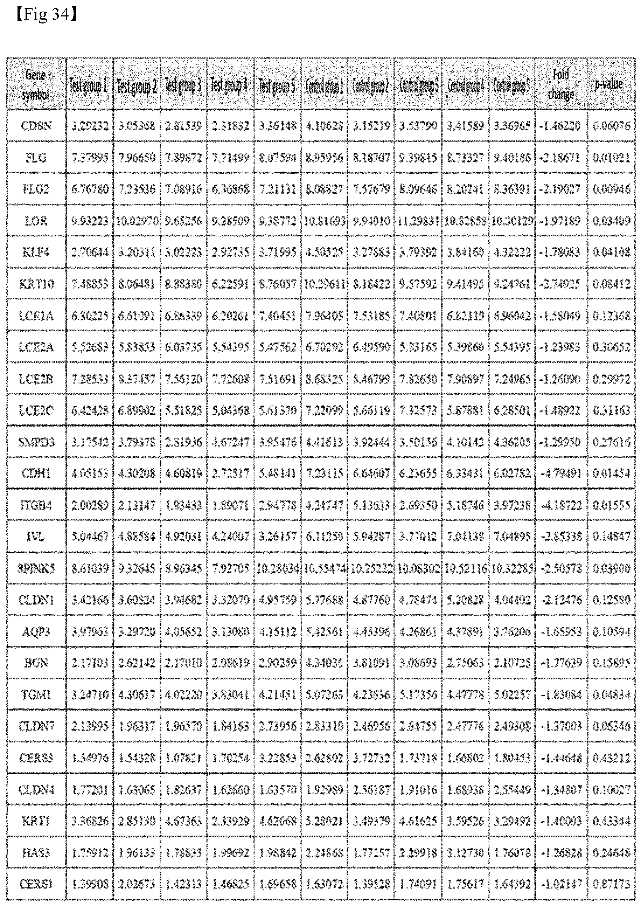

Through document analysis, after that, the inventors selected the genes related to skin hydration (water content/water loss/skin pH) (See FIG. 34), and they summarized the gene expression values checked through the microarray test results for the genes related to the skin hydration, through the document analysis, in the table of the above figure.

In the test group in which a degree of skin hydration was good and the control group in which a degree of skin hydration was not good, the differentiation in the gene expression values obtained by performing the microarray had to exist, so that the values might be useful as biomarkers related to the degree of skin hydration. To determine this, the inventors made use of fold changes and p-values. The fold changes are values that represent the measured values in the test group were higher or lower several times than those of the control group, and the p-values are values between the test group and the control group that represent statistically significant differences in statistical processing. The normalized signal values (expression values) of the respective samples obtained through the RNA microarray tests are represented in the table of FIG. 34, and the fold changes are values obtained by calculating 1:1 expression differences using the normalized signal values between the test group and the control group. The p-values are the values between 0 and 1 that represent the degrees of data coexisting with null hypothesis, and if the p-values are less than or equal to 0.05, generally, the null hypothesis is rejected.

As a result, it was checked that the genes related to the skin hydration (water content/water loss/skin pH), which had the fold changes of 1.2 times or -1.2 times or the p-values less than or equal to 0.05, were CDSN, FLG, FLG2, LOR, KLF4, KRT10, LCE1A, LCE2A, LCE2B, LCE2C, SMPD3, CDH1, ITGB4, IVL, SPINK5, CLDN1, AQP3, BGN, HAS3, TGM1, CLDN7, CERS3, CLDN4, and KRT1.

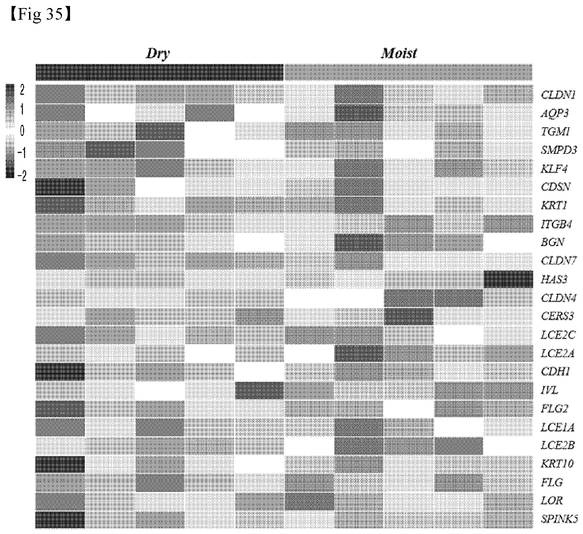

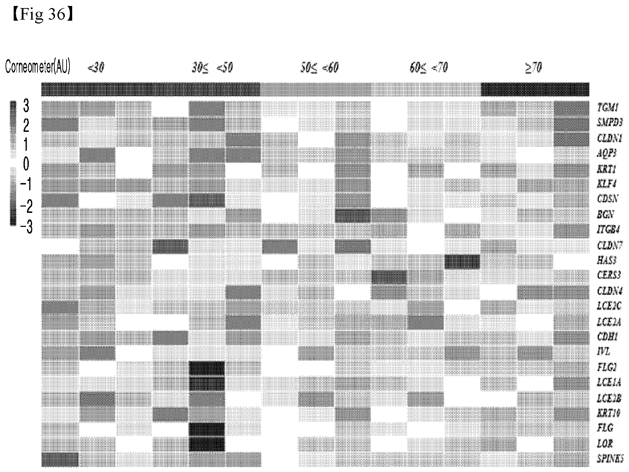

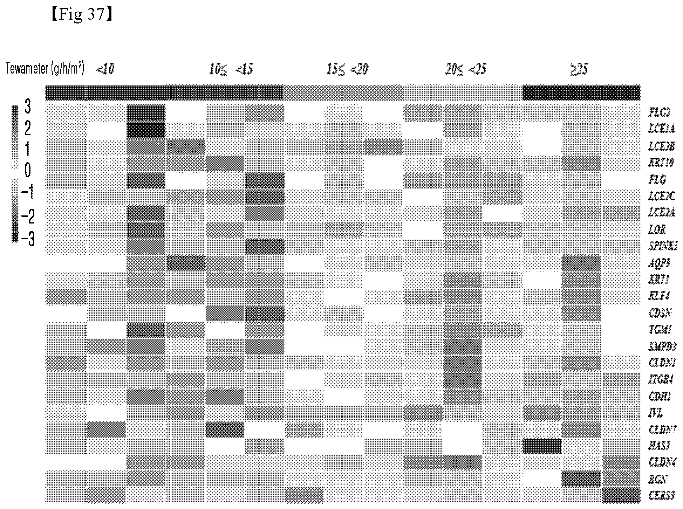

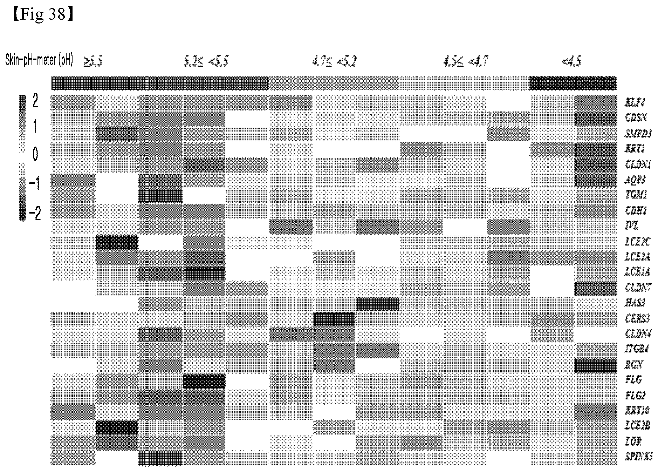

FIGs. 35 to 38 show heatmap analysis results so as to visually compare and analyze the expression levels of the skin hydration biomarker candidates selected through the RNA microarray analysis. FIG. 35 is a heatmap showing the gene expression of the skin hydration biomarker candidates using the RNA microarray analysis results for total ten subjects including five test group subjects who had dry skin and the five control group subjects who had moist skin, and FIG. 36 is a heatmap showing the gene expression of the skin hydration biomarker candidates using the RNA microarray analysis results for total 15 subjects obtained by dividing the water content result values by section on the basis of only the measurement results of the Corneometer. FIG. 37 is a heatmap showing the gene expression of the skin hydration biomarker candidates using the RNA microarray analysis results for total 15 subjects obtained by dividing the transepidermal water loss result values by section on the basis of only the measurement results of the Tewameter. FIG. 38 is a heatmap showing the gene expression of the skin hydration biomarker candidates using the RNA microarray analysis results for total 13 subjects obtained by dividing the skin pH result values by section on the basis of only the measurement results of the Skin-pHmeter.

In the same manner as in FIG. 33, in the heatmap analysis as shown in FIGs. 35 to 38, red colors mean gene expression values become high, and blue colors mean gene expression values become low. From the test results as shown in FIGs. 35 to 38, it was visually checked that the specific gene expression values selected through the document analysis for the dry test group were lower with blue colors than those in the moist control group.

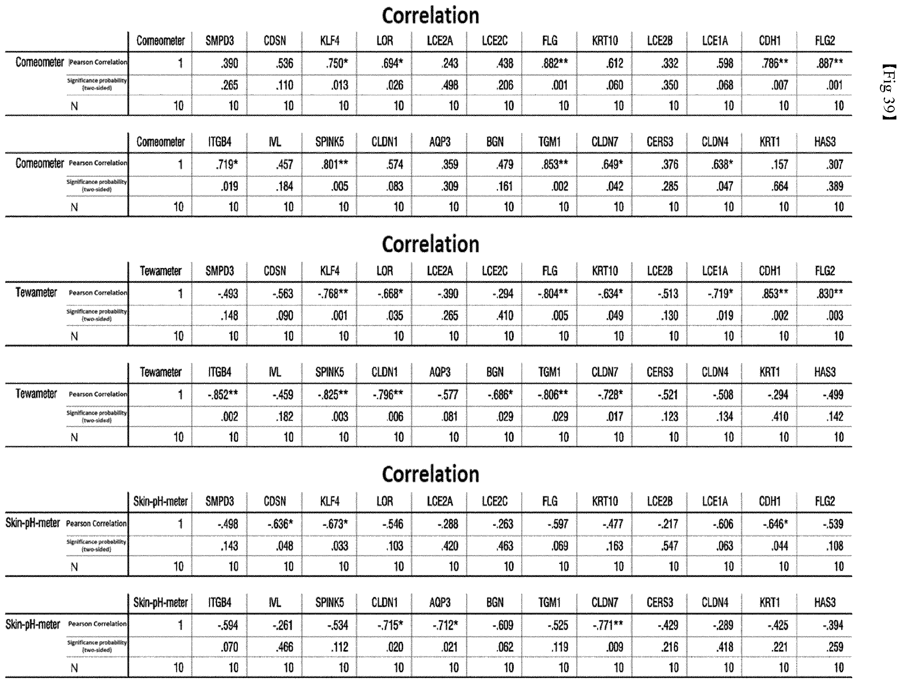

To check the correlation between the skin hydration biomarker candidates selected through the RNA microarray analysis and the measurement results of the devices (Corneometer, Tewameter, and Skin-pHmeter) applicable to the human body, bivariate correlation analysis was conducted. The results are shown in FIGs. 39 to 45.

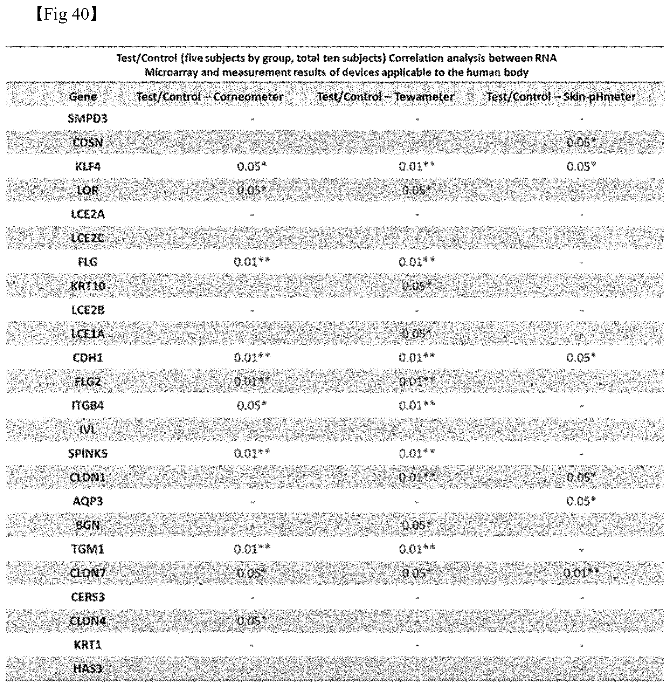

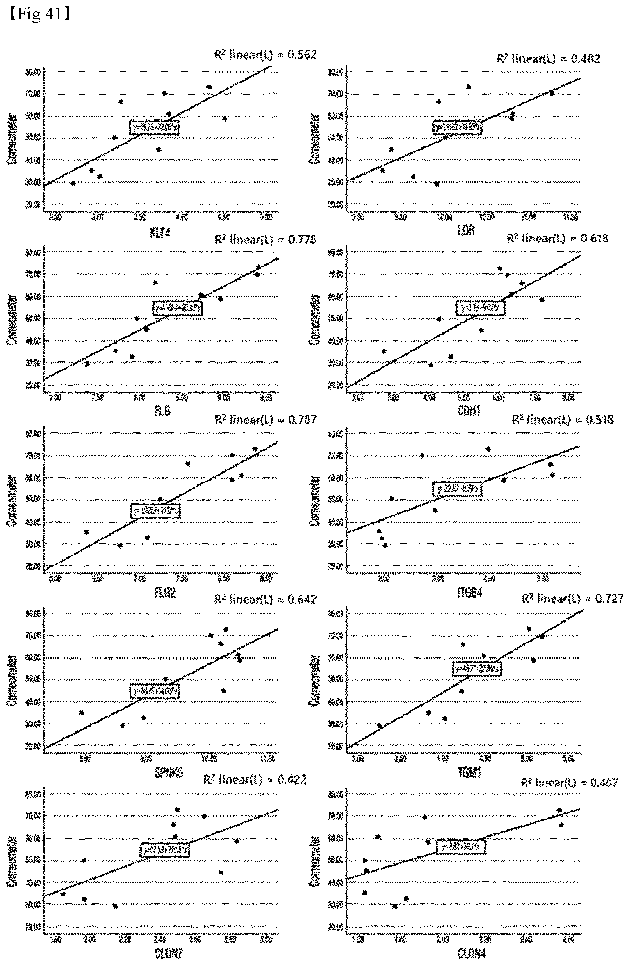

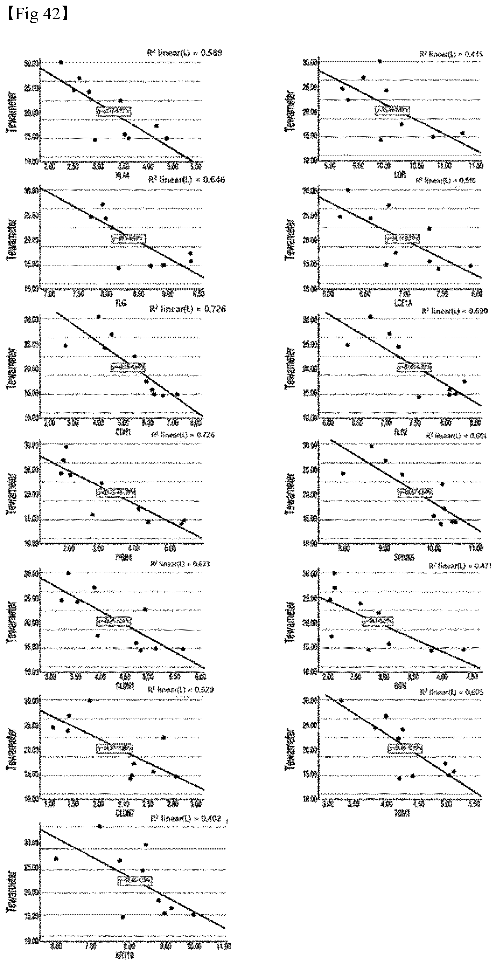

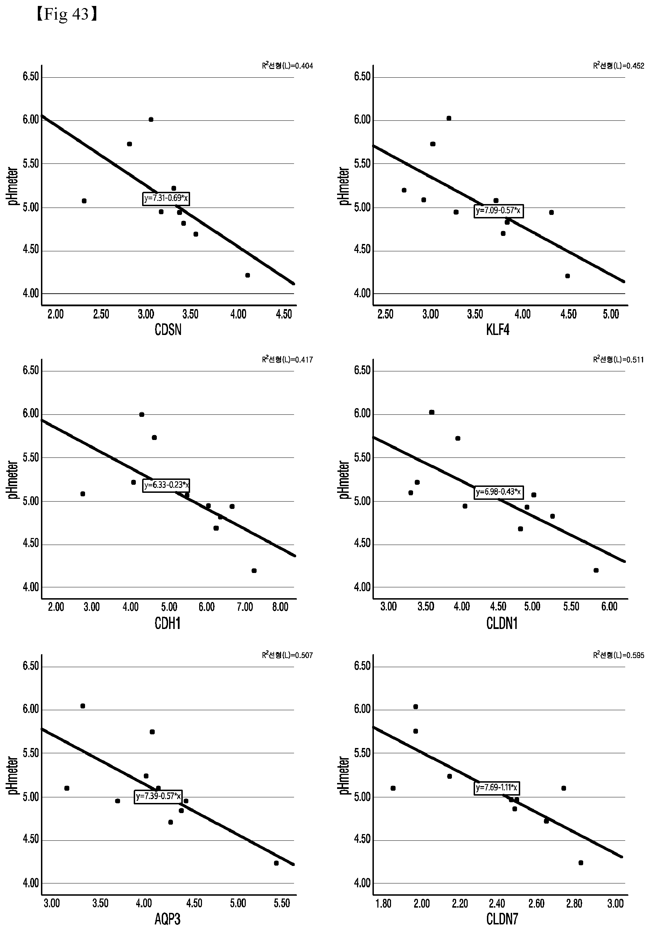

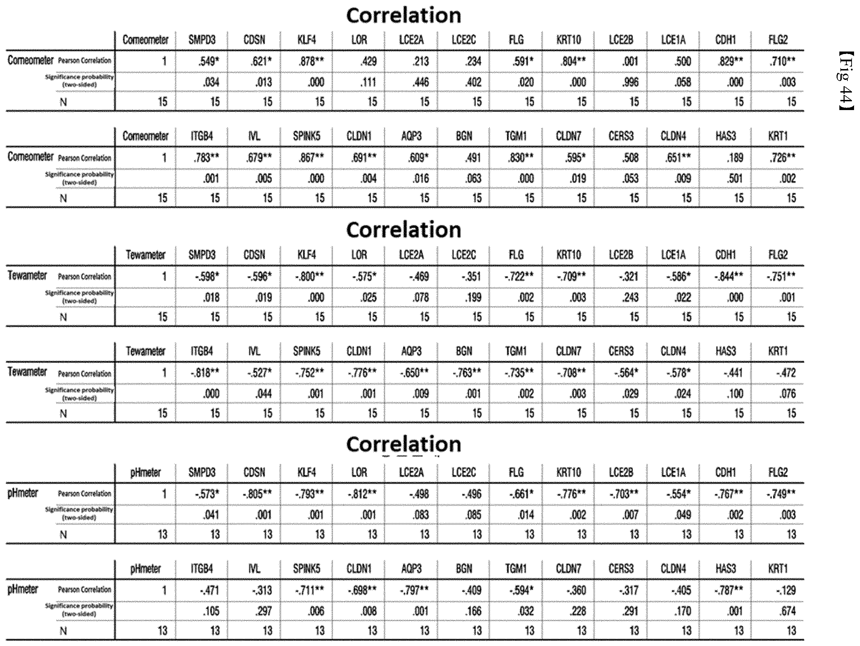

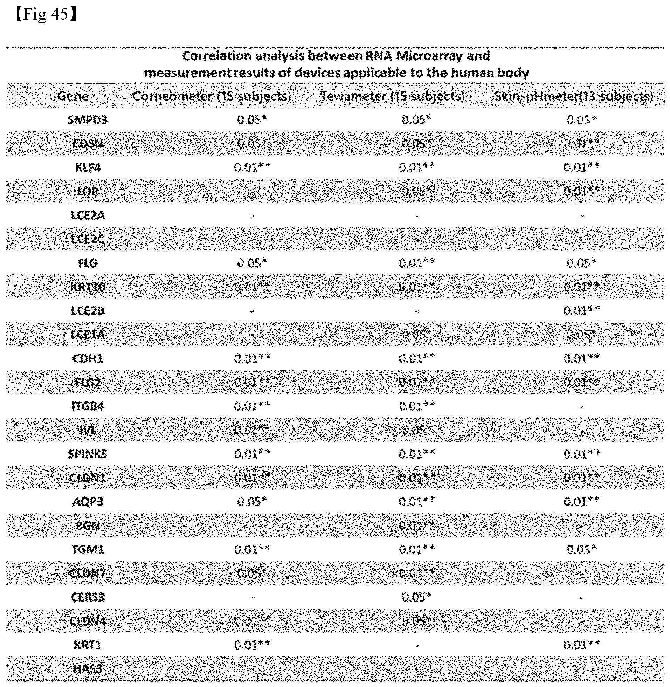

FIG. 39 shows the correlation analysis results between the skin hydration biomarker candidate genes and the measurement results of the devices applicable to the human body. FIG. 40 shows the summary of the analysis values of FIG. 39. FIG. 41 shows scatter graphs of the genes having the significant correlation with the measurement values of the Corneometer. From the graphs, it was checked that FLG, FLG2, LOR, KLF4, CDH1, ITGB4, SPINK5, TGM1, CLDN7, and CLDN4 genes had statistically significant positive correlation with the measurement results of the Corneometer measuring water content. FIG. 42 shows scatter graphs of the genes having the significant correlation with the measurement values of the Tewameter. From the graphs, it was checked that FLG, FLG2, LOR, KLF4, KRT10, LCE1A, CDH1, ITGB4, SPINK5, CLDN1, BGN, TGM1, and CLDN7 genes had statistically significant negative correlation with the measurement results of the Tewameter measuring transepidermal water loss. FIG. 43 shows scatter graphs of the genes having the significant correlation with the measurement values of the Skin-pHmeter. From the graphs, it was checked that CDSN, KLF4, CDH1, CLDN1, AQP3, and CLDN7 genes had statistically significant negative correlation with the measurement results of the Skin-pHmeter measuring skin pH. FIGs. 44 and 45 show the correlation analysis between the skin hydration biomarker candidate genes and the measurement results of the devices (Corneometer, Tewameter, and Skin-pHmeter) applicable to the human body, based on the results as shown in FIGs. 36, 37, and 38. As a result, it was checked that the SMPD3, CDSN, KLF4, FLG, KRT10, CDH1, FLG2, ITGB4, IVL, SPINK5, CLDN1, AQP3, TGM1, CLDN7, CLDN4, and KRT1 genes had statistically significant positive correlation with the water content (Corneometer), SMPD3, CDSN, KLF4, LOR, FLG, KRT10, LCE1A, CDH1, FLG2, ITGB4, IVL, SPINK5, CLDN1, AQP3, BGN, TGM1, CLDN7, CERS3, and CLDN4 genes had statistically significant negative correlation with the transepidermal water loss (Tewameter), and the SMPD3, CDSN, KLF4, LOR, FLG, KRT10, LCE2B, LCE1A, CDH1, FLG2, SPINK5, CLDN1, AQP3, TGM1, and KRT1 genes had statistically significant negative correlation with the skin pH (Skin-pHmeter).

**Efficacy verification of skin hydration (water content/water loss/skin pH) biomarker and selection of biomarker candidate**

So as to verify whether the skin hydration (water content/water loss/skin pH) biomarkers selected in the above serve to evaluate a degree of skin hydration (water content/water loss/skin pH), to screen effective ingredients related to the skin hydration (water content/water loss/skin pH) or moisturizing agents, to evaluate the degree of skin hydration before and after the use of general cosmetics, functional cosmetics, medical devices, medicines, and the like, and to verify whether they are used for the purpose of animal replacement tests, skin hydration efficacy evaluation was carried out.

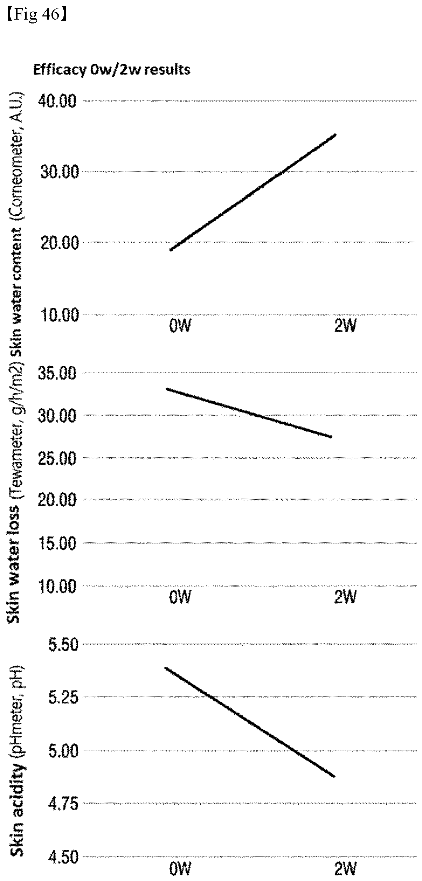

In specific, a Zeroid intensive cream MD was applied for 2 weeks to the face of one test subject (test group) who did not use a moisturizing agent for one month or more and thus had dry skin, and next, water content, transepidermal water loss, and skin pH were measured at week 0 and week 2. Further, after the microneedle patch was attached to the measured portion for 15 minutes, a skin specimen was collected to extract RNA therefrom, and the RNA microarray was conducted. The degree of the dry skin of the subject was better after the Zeroid intensive cream MD was applied for two weeks, when compared to week 0. That is, the water content was increased, the transepidermal water loss was reduced, and the skin pH was decreased. The measurement results are listed in Tables 8 to 10 and FIG. 46.

[Table 8]

|  | At week 0 | At week 2 |
|---|---|---|
| Water content (A.U.) | $18.8 \pm 6.1$ | $35.1 \pm 0.9$ |

[Table 9]

|  | At week 0 | At week 2 |
|---|---|---|
| Transepidermal water loss(g/h/m$^2$) | $33.1 \pm 4.5$ | $27.4 \pm 3.3$ |

[Table 10]

|  | At week 0 | At week 2 |
|---|---|---|
| Skin pH (pH) | $5.4 \pm 0.2$ | $4.9 \pm 0.2$ |

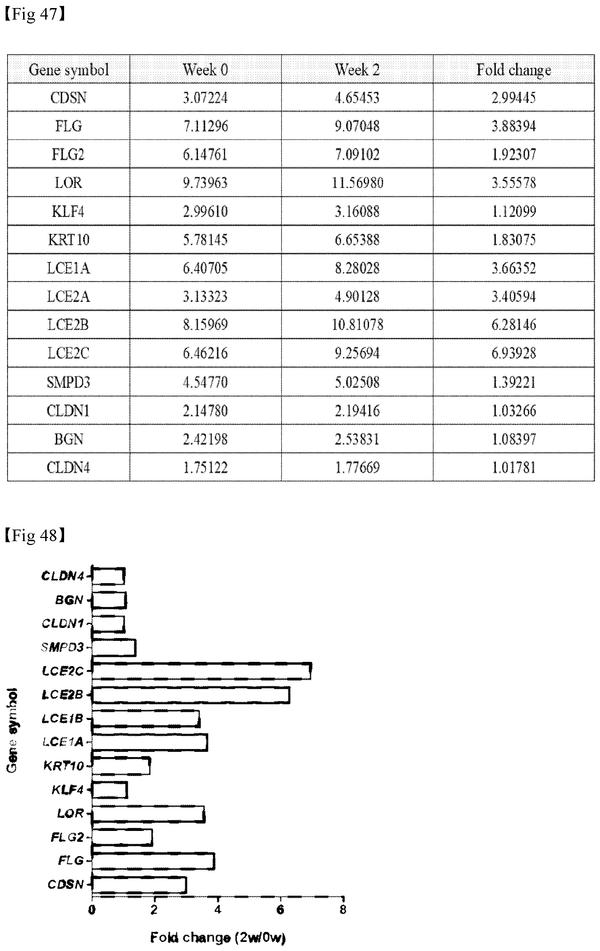

After the RNA microarray test was conducted, so as to check gene expression differences according to the skin hydration efficacy evaluation, gene alignment was performed using R 4.0.0 program. After that, among the skin hydration (water content/water loss/skin pH) biomarkers selected in the above, the genes having the same tendency as one another were selected and thus listed in the table of FIG. 47.

It was checked that the genes among the skin hydration biomarker genes as listed in the table of FIG. 47, which had the fold changes of 1.2 times or -1.2 times or the p-values less than or equal to 0.05, were CDSN, FLG, FLG2, LOR, KRT10, LCE1A, LCE2A, LCE2B, LCE2C, and SMPD3.

FIG. 48 is a graph showing visual comparison and analysis results of the expression levels of the skin hydration efficacy evaluation biomarker candidates selected through the skin hydration efficacy evaluation RNA microarray analysis.

The genes CDSN, FLG, FLG2, LOR, KLF4, KRT10, LCE1A, LCE2A, LCE2B, LCE2C, SMPD3, CDH1, ITGB4, IVL, SPINK5, CLDN1, AQP3, BGN, HAS3, TGM1, CLDN7, CERS3, CLDN4, and KRT1 were finally selected as the skin hydration (water content/water loss/skin pH) biomarkers. Further, in view of accurate and effective skin condition diagnosis, the inventors found that among the genes, it was advantageous when the genes FLG, AQP3, and LOR were included in the biomarker group.

As a result, it will be predicted that the study results of the inventors may be used as very useful tools in a skin hydration evaluation method or kit, in developing and screening of skin hydration inducing or inhibiting materials, in providing skin type information of individuals, in developing customized cosmetics, and in evaluating animal replacement skin hydration efficacy.

**<Skin pigmentation>**

**Selection of subject according to skin pigmentation, specialty history taking, evaluation through device applicable to the human body, and skin specimen collection**

The subjects waited under constant temperature and humidity condition for 30 minutes after they washed their face, and next, the survey and specialty history taking for the subjects were conducted. After that, skin colors and melanin were measured using devices applicable to the human body. In specific, the skin colors were measured using Spectrophotometer, and the melanin using Mexameter. The skin colors were analyzed with ITA° values, and the melanin with melanin index values.

The physical findings of the skin colors of the subjects were obtained by the visual determination of a dermatologist on the basis of Fitzpatrick scale.

Table 11 shows standards on which total subjects are divided into a test group and a control group. The test group was constituted of subjects who had Fitzpatrick type V with the ITA° values lower than 28 and dark skin colors, and the control group was constituted of subjects who had Fitzpatrick type II with the ITA° values higher than 41 and bright skin colors. After the selection of the test and control groups, skin pigmentation was measured using the devices applicable to the human body.

[Table 11]

|  | Test group | Control group |
|---|---|---|
| The number of samples (subjects) | 5 | 5 |
| Age (average ± SD) | 47.80 ± 12.85 | 36.00 ± 9.95 |
| Physical findings (Type) | 4.0 ± 0.0 | 3.2 ± 0.4 |
| Skin color (ITA°) | 23.49 ± 4.33 | 46.50 ± 3.03 |
| Melanin (Melanin index) | 203.00 ± 24.48 | 132.20 ± 15.31 |

Further, after the microneedle patch was attached to each subject's face for 15 minutes, a skin specimen was collected.

**Collection of RNA from skin specimen**

The skin specimens collected from the subjects were provided to RNeasy® Plus Mini Kit (Qiagen), and high purity RNA was extracted using the kit. Next, quality control (QC) of RNA samples was checked using Nanodrop and 2100 Bioanalyzer devices, and then, RNA microarray analysis was conducted.

**RNA microarray analysis**

The samples were subjected to flexible and high sensitive pre-treatment using GeneChip® WT Pico Kit (Applied Biosystems) through which a small amount of total RNA (100 pg) separated by the above-mentioned method was amplified to cRNA. The amplified RNA was subjected to microarray analysis according to the manual of GeneChip® Human Gene 2.0 ST Array (Affymetrix) platform. The microarray procedure introduced in the manual of platform was shown in FIGs. 4 and 5. The inventors conducted the microarray in the same procedure. The RNA microarray analysis protocols of the platform were the same as in Table 3.

After that, the entire data were summarized and normalized using Robust Multi-Average (RMA) of Affymetrix® Power Tools (APT), and the results were summarized to gene levels and subjected to differentially expressed gene (DEG) analysis. The statistical significance of the data was analyzed using independent t-test and variations of fold change, and false discovery rate (FDR) was adjusted in p value using Benjamini-Hochberg algorithm. DEG set was subjected to Hierarchical cluster analysis using linkage and Euclidean distance. Further, all data analysis and visualization work for checking gene expression differences were carried out using R 4.0.0 program.

**Selection of skin pigmentation biomarker candidate through bioinformatics analysis and significant correlation analysis**

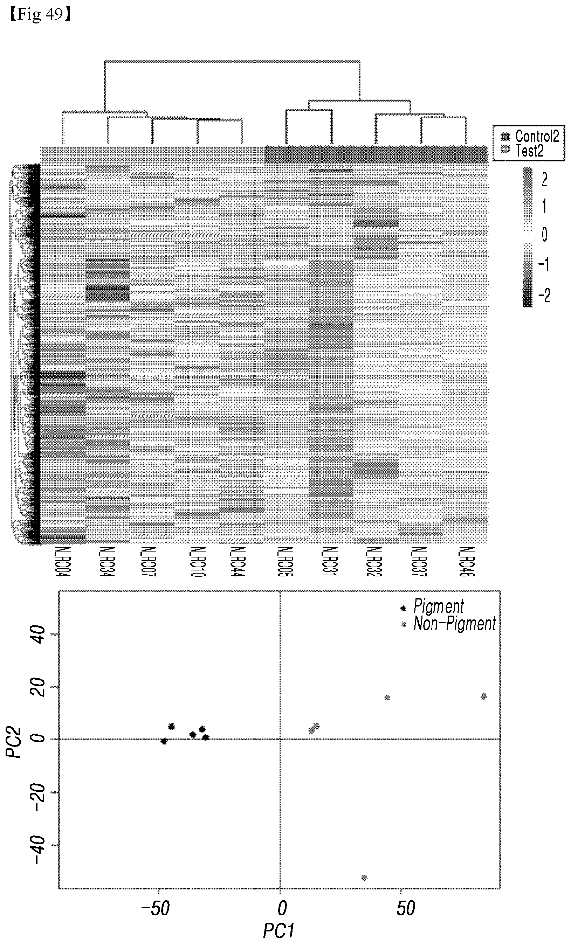

After the RNA microarray test was conducted, so as to check about forty thousand gene expression differences between the test group and the control group, gene alignment was performed using R 4.0.0 program. FIG. 49 shows gene alignment results. The gene alignment as shown in FIG. 49 is called heatmap analysis. The heatmap analysis is a term made by combining heat and map and represents various information expressed with colors in the form of heat distribution on an image. In the heatmap analysis described in the present invention, red colors mean gene expression values become high, and blue colors mean gene expression values become low. When hundreds of genes were analyzed, it was checked in FIG. 49 that there were specific differences between a plurality of gene expression values in the test group and the control group.

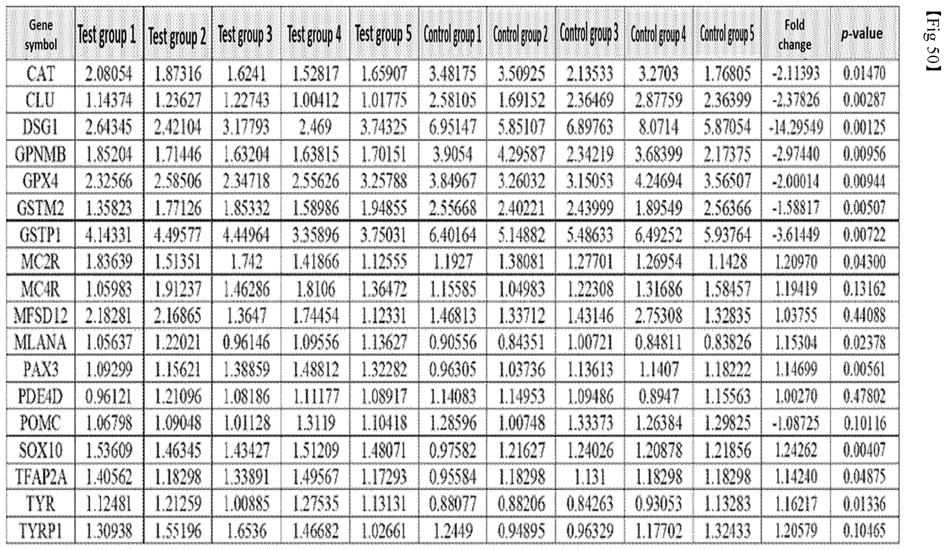

Through document analysis, after that, the inventors selected the genes related to skin pigmentation and melanogenesis mechanism (See FIG. 50), and they conducted the microarray test of the genes related to the skin pigmentation checked through the document analysis. The gene expression values checked through the microarray test results were listed in the table of FIG. 50.

In the test group in which a degree of skin pigmentation was serious and the control group in which a degree of skin pigmentation was not serious, the differentiation in the gene expression values obtained by performing the microarray had to exist, so that the values might be useful as biomarkers related to the degree of skin pigmentation. To determine this, the inventors made use of fold changes and p-values. The fold changes are values that represent the measured values in the test group were higher or lower several times than those of the control group, and the p-values are values between the test group and the control group that represent statistically significant differences in statistical processing. The normalized signal values (expression values) of the respective samples obtained through the RNA microarray tests are represented in the table of FIG. 50, and the fold changes are values obtained by calculating 1:1 expression differences using the normalized signal values between the test group and the control group. The p-values are the values between 0 and 1 that represent the degrees of data coexisting with null hypothesis, and if the p-values are less than or equal to 0.05, generally, the null hypothesis is rejected.

As a result, it was checked that the genes related to the skin pigmentation, which had the fold changes of 1.2 times or -1.2 times or the p-values less than or equal to 0.05, were CAT, CLU, DSG1, GPNMB, GPX4, GSTM2, GSTP1, MC2R, MLANA, PAX3, SOX10, TFAP2A, TYR, and TYRP1.

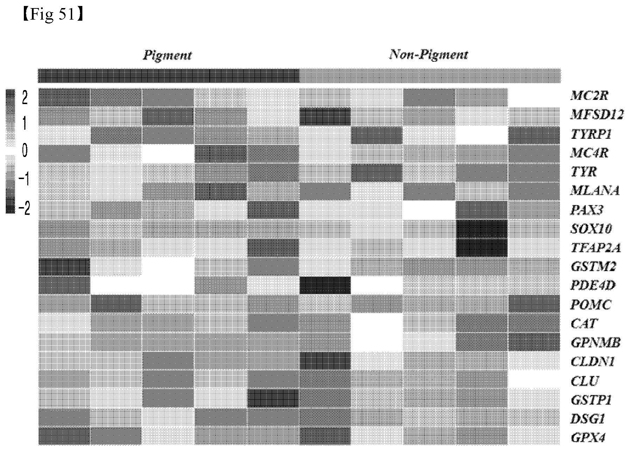

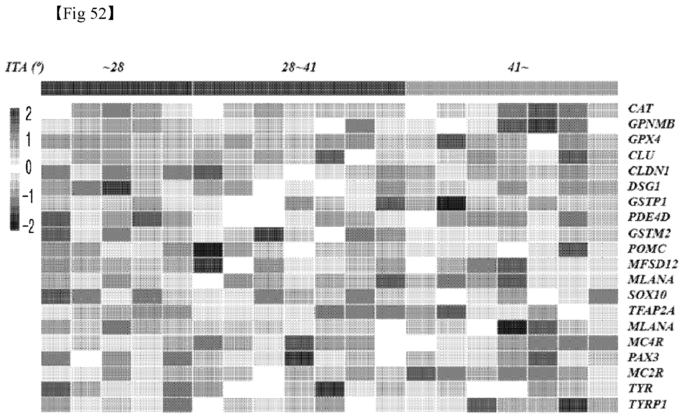

FIGs. 51 and 52 show heatmap analysis results so as to visually compare and analyze the expression levels of the skin pigmentation biomarker candidates selected through the RNA microarray analysis. FIG. 51 shows the heatmap analysis results for total ten subjects including five test group subjects who had the ITA° values less than or equal to 28 and five control group subjects who had the ITA° values greater than or equal to 41, and FIG. 52 is the heatmap showing the gene expressions of the skin pigmentation biomarker candidates using the RNA microarray analysis results of total 19 subjects including five subjects who had the ITA° values less than or equal to 28, seven subjects who had the ITA° values of 20 to 40, and seven subjects who had the ITA° values greater than or equal to 41.

In the same manner as in FIG. 49, in the heatmap analysis as shown in FIGs. 51 and 52, red colors mean gene expression values become high, and blue colors mean gene expression values become low. From the test results as shown in FIGs. 51 and 52, it was visually checked that the specific gene expression values selected through the document analysis in the test group were higher with red colors than those in the control group.

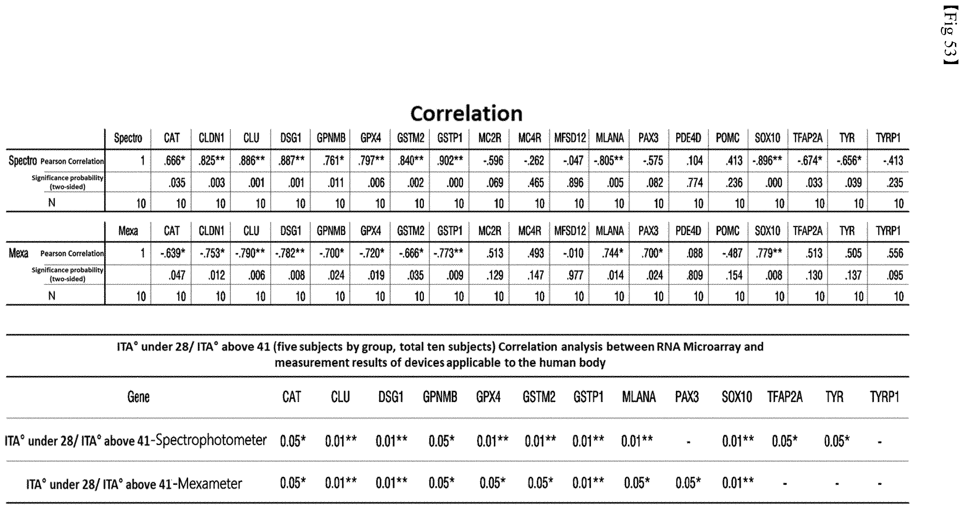

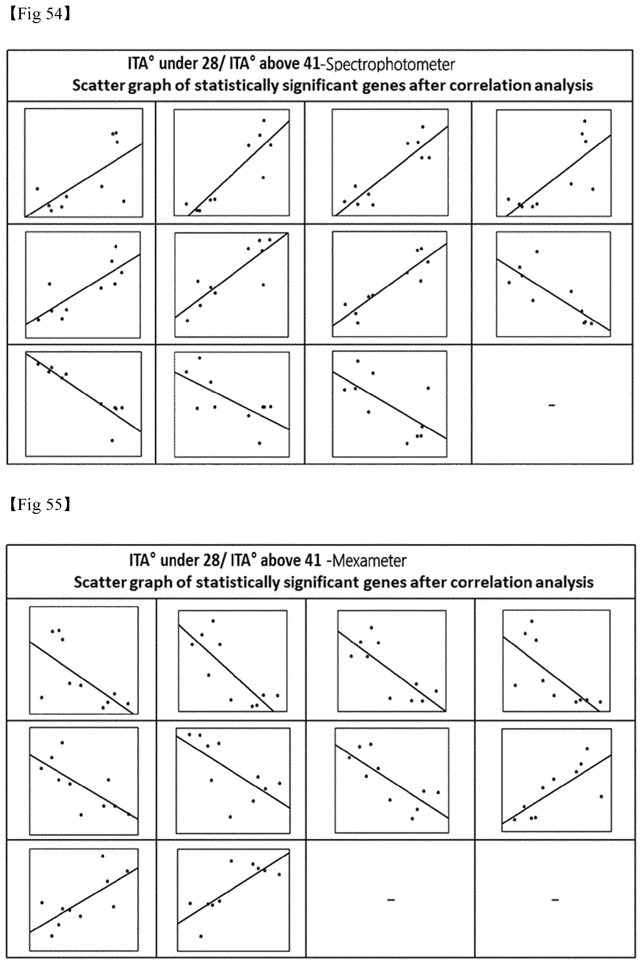

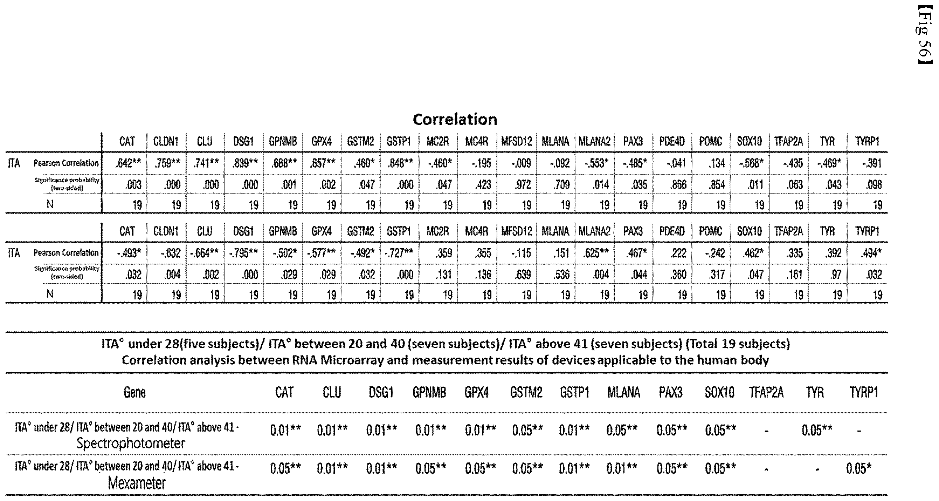

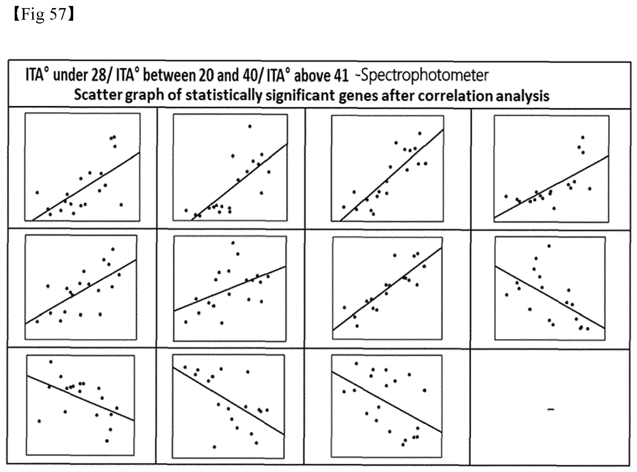

To check the correlation between the skin pigmentation biomarker candidate genes CAT, CLU, DSG1, GPNMB, GPX4, GSTM2, GSTP1, MC2R, MLANA, PAX3, SOX10, TFAP2A, TYR, and TYRP1 selected through the RNA microarray analysis and the measurement results of the devices (Spectrophotometer and Mexameter) applicable to the human body, bivariate correlation analysis was conducted. The results are shown in FIGs. 53 to 58.

FIGs. 53 to 55 shows the correlation analysis results between the skin pigmentation biomarker candidates and the measurement results of the devices applicable to the human body after the total ten subjects including the five test group subjects who had the ITA° values less than or equal to 28 and the five control group subjects who had the ITA° values greater than or equal to 41 were selected and subjected to the RNA microarray analysis. FIGs. 53 to 55 shows the correlation analysis results between the skin pigmentation biomarker candidates and the measurement results of the devices applicable to the human body after the total 19 subjects including the five subjects who had the ITA° values less than or equal to 28, the seven subjects who had the ITA° values of 20 to 40, and the seven subjects who had the ITA° values greater than or equal to 41 were selected and subjected to the RNA microarray analysis.

As a result, it was checked that CAT, CLU, DSG1, GPNMB, GPX4, GSTM2, GSTP1, MLANA, SOX10, TFAP2A, and TYR genes in the RNA microarray analysis results for the total ten subjects including the five test group subjects who had the ITA° values less than or equal to 28 and the five control group subjects who had the ITA° values greater than or equal to 41 had statistically significant positive or negative correlation with the measurement results of the Spectrophotometer measuring skin colors and that CAT, CLU, DSG1, GPNMB, GPX4, GSTM2, GSTP1, MLANA, PAX3, and SOX10 genes had statistically significant negative or positive correlation with the measurement results of the Mexameter measuring melanin around the eyes.

Further, it was checked that CAT, CLU, DSG1, GPNMB, GPX4, GSTM2, GSTP1, MLANA, PAX3, SOX10, and TYR genes in the RNA microarray analysis results for the total 19 subjects including the five subjects who had the ITA° values less than or equal to 28, the seven subjects who had the ITA° values of 20 to 40, and the seven subjects who had the ITA° values greater than or equal to 41 had statistically significant positive or negative correlation with the measurement results of the Spectrophotometer measuring skin colors and that CAT, CLU, DSG1, GPNMB, GPX4, GSTM2, GSTP1, MLANA, PAX3, SOX10, and TYRP1 genes had statistically significant negative or positive correlation with the measurement results of the Mexameter measuring the melanin around the eyes.

When the RNA microarray analysis results of the subjects and the measurement results of the devices applicable to the human body were totally analyzed, accordingly, total 13 genes, CAT, CLU, DSG1, GPNMB, GPX4, GSTM2, GSTP1, MLANA, PAX3, SOX10, TFAP2A, TYR, and TYRP1 genes were selected as the skin pigmentation biomarkers.

**Selection of skin pigmentation biomarker through skin whitening efficacy evaluation**

So as to verify whether the skin pigmentation biomarkers selected in the above serve to evaluate and diagnose a degree of skin pigmentation, to screen effective ingredients related to the skin pigmentation or whitening or whitening agents, to evaluate the degree of skin pigmentation or whitening before and after the use of general cosmetics, functional cosmetics, medical devices, medicines, and the like, and to verify whether they are used for the purpose of animal replacement tests, skin whitening efficacy evaluation was carried out.

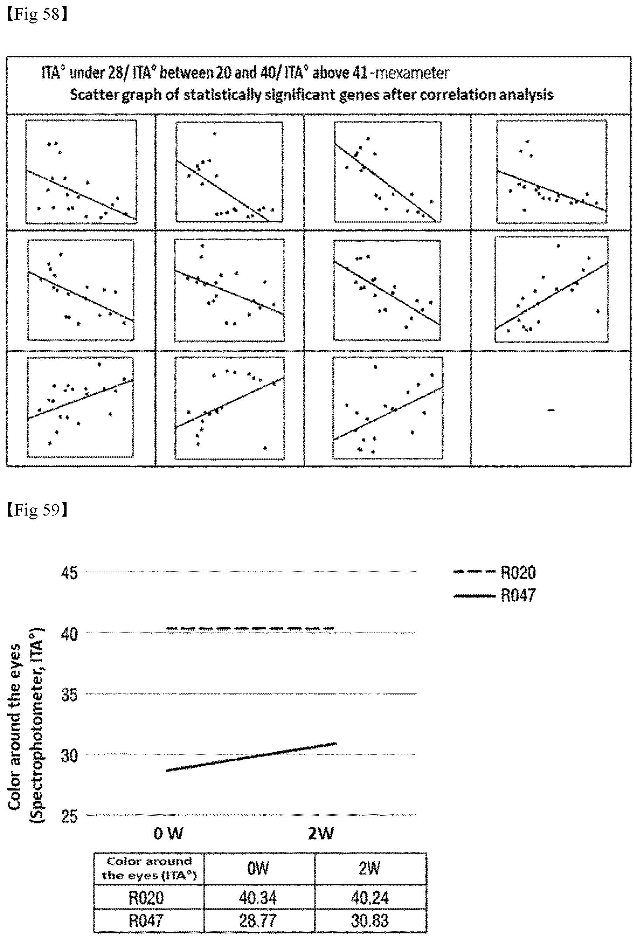

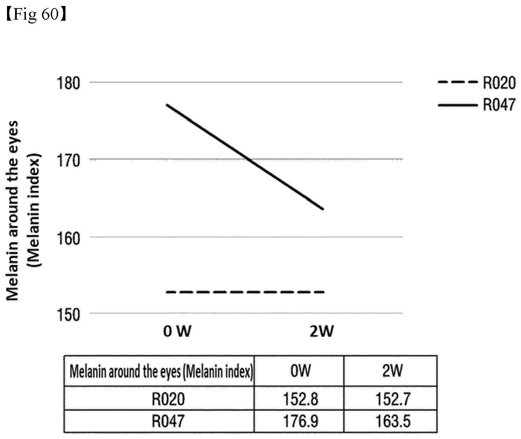

In specific, a Trilustra cream (Kolmar Korea) was applied for two weeks to the area around the eyes of two test subjects who had liver spots around the eyes. At week 0 and week 2, next, a skin color was measured using the Spectrophotometer device (See Table 12 and FIG. 59) and melanin around the eyes was measured using the Mexameter device (See Table 13 and FIG. 60). Further, after the microneedle patch was attached to the measured portion for 15 minutes, a skin specimen was collected to extract RNA therefrom, and the RNA microarray was conducted.

[Table 12]

| Skin color (ITA°) | At week 0 | At week 2 |
| --- | --- | --- |
| Subject 1 | 40.34 ± 1.24 | 40.24 ± 1.26 |
| Subject 2 | 28.77 ± 1.47 | 30.83 ± 1.02 |

[Table 13]

| Melanin(Melanin index) | At week 0 | At week 2 |
| --- | --- | --- |
| Subject 1 | 152.8 ± 15.9 | 152.7 ± 8.1 |
| Subject 2 | 176.9 ± 25.4 | 163.5 ± 24.1 |

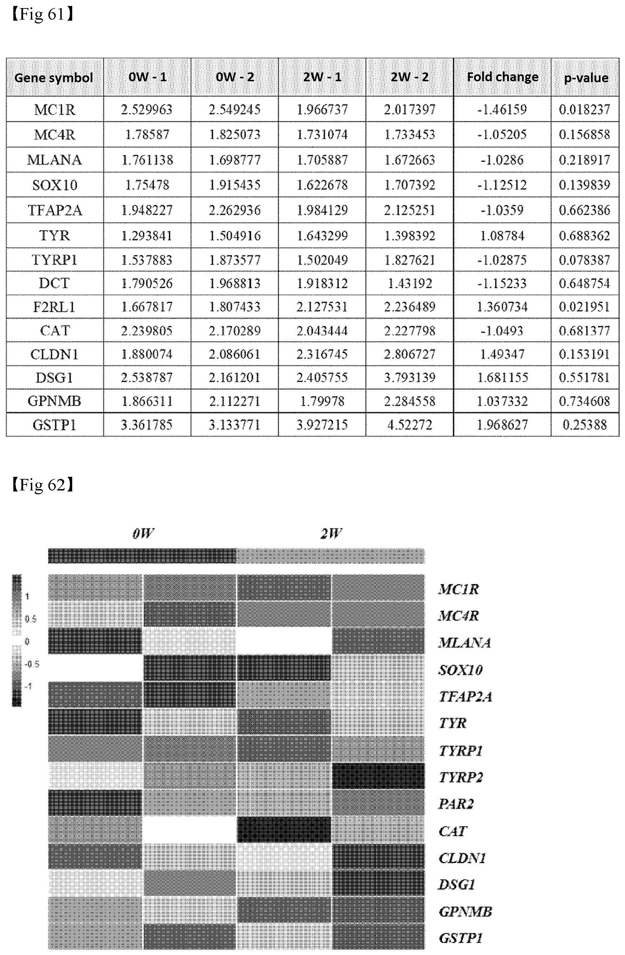

After the RNA microarray test was conducted, so as to check gene expression differences according to the skin whitening efficacy evaluation, gene alignment was performed using R 4.0.0 program. After that, among the genes related to the skin pigmentation or melanogenesis selected through the document analysis and the skin pigmentation biomarkers (CAT, CLU, DSG1, GPNMB, GPX4, GSTM2, GSTP1, MLANA, PAX3, SOX10, TFAP2A, TYR, and TYRP1) selected in the above, the genes having the same tendency as one another were selected and thus listed. FIG. 61 is a table showing the selected results, in which the skin whitening efficacy biomarker candidates are listed.

It was checked that the genes among the genes related to the skin pigmentation or melanogenesis selected through the document analysis and the above skin pigmentation genes, which had the fold changes of 1.2 times or -1.2 times or the p-values less than or equal to 0.05, were MC1R, F2RL1, CLDN1, DSG1, and GSTP1.

FIG. 62 shows heatmap analysis results to visually compare and analyze the expression levels of the skin whitening efficacy evaluation biomarker candidates selected through the skin whitening efficacy evaluation RNA microarray analysis.

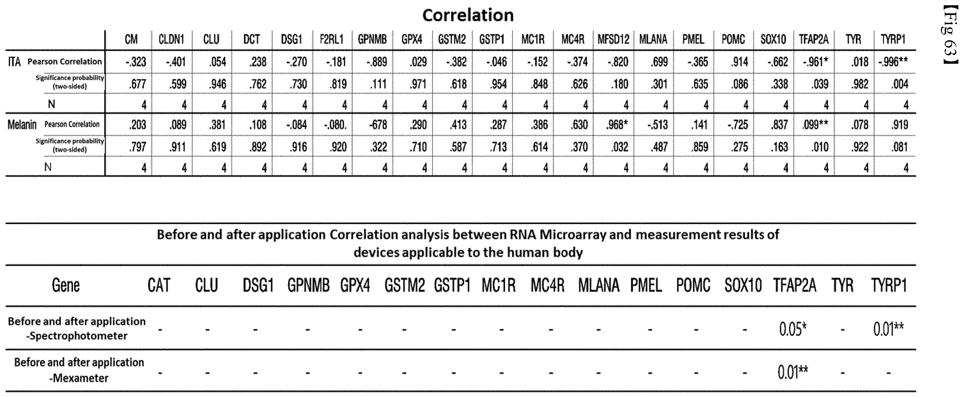

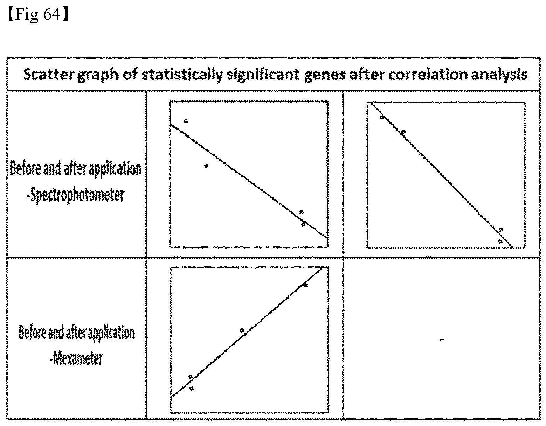

To check the correlation between the skin whitening biomarker candidates selected through the skin whitening efficacy evaluation RNA microarray analysis and the devices (Spectophotometer and Mexameter) applicable to the human body, bivariate correlation analysis was conducted by the inventors. FIGs. 63 and 64 show the analysis results of the correlation between the skin whitening biomarker candidates selected through the RNA microarray analysis for the subjects who are subjected to the skin whitening efficacy evaluation and the devices applicable to the human body. Referring to the analysis results of FIGs. 63 and 64, it was checked that after the Trilustra cream (a combined material of tretinoin, hydroquinone, and steroid) was applied for 2 weeks, when compared to week 0, the TFAP2A and TYRP1 genes had statistically significant negative correlation with the measurement results of the Spectrophotometer device measuring skin color. Further, it was checked that the TFAP2A gene had statistically significant positive correlation with the measurement results of the Mexameter device measuring melanin around the eyes.

When the results of the skin pigmentation and skin whitening efficacy evaluation conducted by the inventors were totally analyzed, accordingly, total 16 genes, the CAT, CLU, DSG1, GPNMB, GPX4, GSTM2, GSTP1, MLANA, PAX3, SOX10, TFAP2A, TYR, TYRP1, MC1R, F2RL1, and CLDN1 were selected as the skin pigmentation biomarkers in the widest range thereof. Further, in view of accurate and effective skin condition diagnosis, the inventors found that it was advantageous when the MLANA, TYRP1, and GPNMB genes among the genes were included in the biomarker group.

If such selection results are utilized, any one or two or more genes among the 16 RNA genes may be used as the biomarkers, so that the skin pigmentation or whitening evaluation may be performed quantitatively. Further, it will be predicted that the study results of the inventors may be used as very useful tools in a skin pigmentation evaluation method or kit, in developing and screening of skin pigmentation inducing or inhibiting materials, in providing skin type information of individuals, in developing customized cosmetics, and in evaluating animal replacement skin pigmentation efficacy.

**<Skin sebum>**

**Selection of subject according to skin sebum, specialty history taking, evaluation through device applicable to the human body, and skin specimen collection**

The subjects waited under constant temperature and humidity condition for 30 minutes after they washed their face, and next, the survey and specialty history taking for the subjects were conducted. After that, skin sebum around both sides of the nose was measured. In specific, the skin sebum was measured using Sebumeter® SM815 and analyzed with $\mu$g/cm$^2$ values.

The physical findings of the skin sebum of the subjects were obtained by the visual determination of a dermatologist on the basis of a document (Reprod Biol Endocrinol. 2017 Homburg *et al*).

Table 14 shows the skin hydration degree evaluation results for five control subjects who had oily skin and five test subjects who had non-oily skin, which were selected among the total subjects.

[Table 14]

|  | Test group (Oily) | Control group (Non-oily) |
|---|---|---|
| The number of samples (subjects) | 5 | 5 |
| Age (average $\pm$ SD) | 38.40 $\pm$ 12.93 | 37.60 $\pm$ 18.15 |
| Physical findings (Score) | 1.80 $\pm$ 0.84 | 1.40 $\pm$ 0.55 |
| Skin sebum content ($\mu$g/cm$^2$) | 223.70 $\pm$ 65.96 | 53.90 $\pm$ 33.52 |

Further, after the microneedle patch was attached to each subject's face for 15 minutes, a skin specimen was collected.

**Collection of RNA from skin specimen**

The skin specimens collected from the subjects were provided to RNeasy® Plus Mini Kit (Qiagen), and high purity RNA was extracted using the kit. Next, quality control (QC) of RNA samples was checked using Nanodrop and 2100 Bioanalyzer devices, and then, RNA microarray analysis was conducted.

**RNA microarray analysis**

The samples were subjected to flexible and high sensitive pre-treatment using GeneChip® WT Pico Kit (Applied Biosystems) through which a small amount of total RNA (100 pg) separated by the above-mentioned method was amplified to cRNA. The amplified RNA was subjected to microarray analysis according to the manual of GeneChip® Human Gene 2.0 ST Array (Affymetrix) platform. The microarray procedure introduced in the manual of platform was shown in FIGs. 4 and 5. The inventors conducted the microarray in the same procedure. The RNA microarray analysis protocols of the platform were the same as in Table 3.

After that, the entire data were summarized and normalized using Robust Multi-Average (RMA) of Affymetrix® Power Tools (APT), and the results were summarized to gene levels and subjected to differentially expressed gene (DEG) analysis. The statistical significance of the data was analyzed using independent t-test and variations of fold change, and false discovery rate (FDR) was adjusted in p value using Benjamini-Hochberg algorithm. DEG set was subjected to Hierarchical cluster analysis using linkage and Euclidean distance. Further, all data analysis and visualization work for checking gene expression differences were carried out using R 4.0.0 program.

**Selection of skin sebum biomarker candidate through bioinformatics analysis and significant correlation analysis**

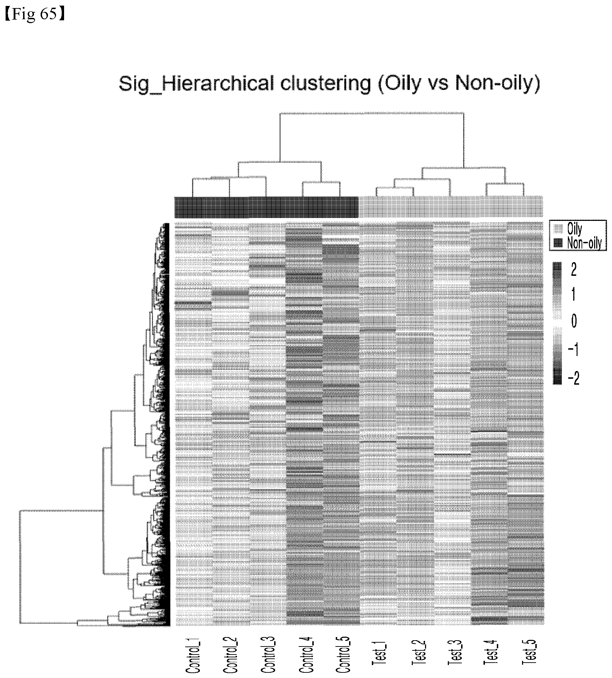

After the RNA microarray test was conducted, so as to check about forty thousand gene expression differences between the test group and the control group, gene alignment was performed using R 4.0.0 program. FIG. 65 shows gene alignment results. The gene alignment as shown in FIG. 65 is called heatmap analysis. The heatmap analysis is a term made by combining heat and map and represents various information expressed with colors in the form of heat distribution on an image. In the heatmap analysis described in the present invention, red colors mean gene expression values become high, and blue colors mean gene expression values become low. When hundreds of genes were analyzed, it was checked in FIG. 65 that there were specific differences between a plurality of gene expression values in the test group and the control group.

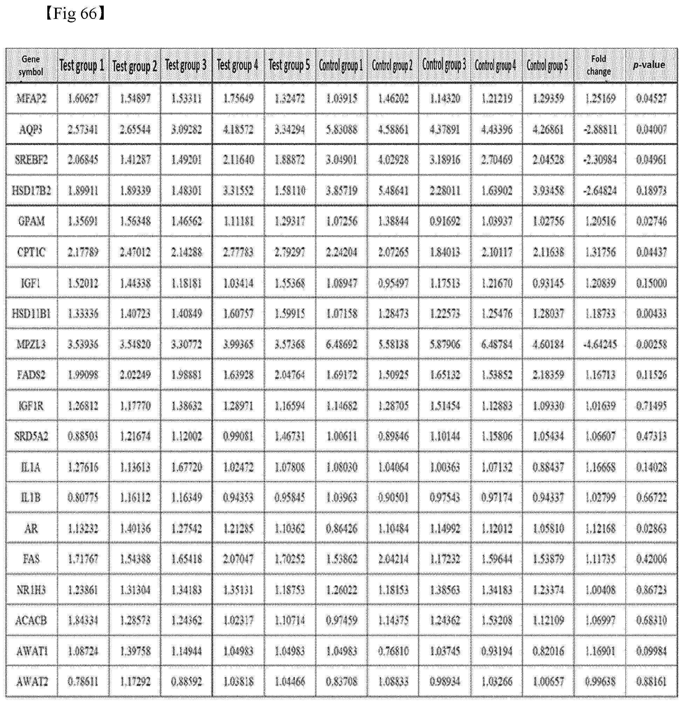

Through document analysis, after that, the inventors selected the genes related to skin sebum (See FIG. 66), and they conducted the microarray test of the genes related to the skin sebum, which were checked through the document analysis. The gene expression values checked through the microarray test results are summarized in the table of FIG. 66.

In the test group in which a degree of skin sebum was high and the control group in which a degree of skin sebum was not high, the differentiation in the gene expression values obtained by performing the microarray had to exist, so that the values might be useful as biomarkers related to the degree of skin sebum. To determine this, the inventors made use of fold changes and p-values. The fold changes are values that represent the measured values in the test group were higher or lower several times than those of the control group, and the p-values are values between the test group and the control group that represent statistically significant differences in statistical processing. The normalized signal values (expression values) of the respective samples obtained through the RNA microarray tests are represented in the table of FIG. 66, and the fold changes are values obtained by calculating 1:1 expression differences using the normalized signal values between the test group and the control group. The p-values are the values between 0 and 1 that represent the degrees of data coexisting with null hypothesis, and if the p-values are less than or equal to 0.05, generally, the null hypothesis is rejected.

As a result, it was checked that the genes related to the skin sebum, which had the fold changes of 1.2 times or -1.2 times or the p-values less than or equal to 0.05, were MFAP2, IGF1, HSD11B1, GPAM, CPT1C, AR, MPZL3, AQP3, SREBF2, and HSD17B2.

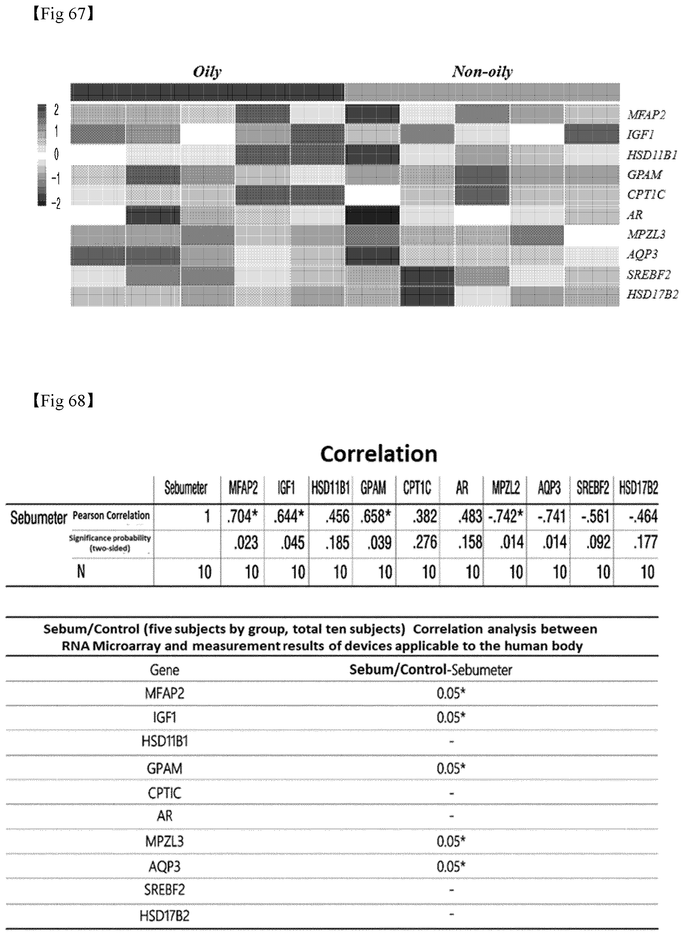

FIG. 67 shows heatmap analysis results so as to visually compare and analyze the expression levels of the skin sebum biomarker candidates selected through the RNA microarray analysis. In the same manner as in FIG. 65, in the heatmap analysis as shown in FIG. 67, red colors mean gene expression values become high, and blue colors mean gene expression values become low. From the test results as shown in FIG. 67, it was visually checked that the specific gene expression values selected through the document analysis in the test group were higher with the red colors and lower with the blue colors than those in the control group.

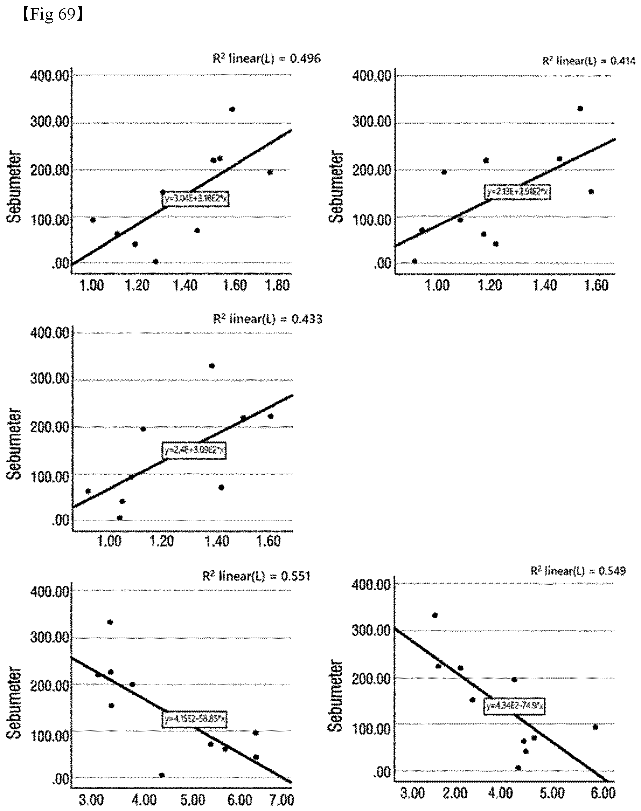

To check the correlation between the skin sebum biomarker candidates, MFAP2, IGF1, HSD11B1, GPAM, CPT1C, AR, MPZL3, AQP3, SREBF2, and HSD17B2 selected through the RNA microarray analysis and the measurement results of the device (Sebumeter) applicable to the human body, bivariate correlation analysis was conducted. The results are shown in FIGs. 68 and 69.

As the bivariate correlation analysis results as shown in FIGs. 68 and 69, the inventors checked that the MFAP2, GPAM, and IGF1 genes had statistically significant positive correlation with the skin sebum content (the measurement results of the Sebumeter) and the MPZL3 and AQP3 genes had statistically significant negative correlation with the skin sebum content (the measurement results of the Sebumeter).

When the results of the skin sebum evaluation conducted by the inventors were totally analyzed, accordingly, total 10 genes, the MFAP2, AQP3, SREBF2, HSD17B2, GPAM, CPT1C, AR, IGF1, HSD11B1, and MPZL3 were selected as the skin sebum biomarkers in the widest range thereof. Further, in view of accurate and effective skin condition diagnosis, the inventors found that it was advantageous when the MPZL3, GPAM, and IGF1 genes among the genes were included in the biomarker group.

If such selection results are utilized, any one or two or more genes among the 10 RNA genes may be used as the biomarkers, so that the skin sebum evaluation may be performed quantitatively. Further, it will be predicted that the study results of the inventors may be used as very useful tools in a skin sebum evaluation method or kit, in developing and screening skin sebum inducing or inhibiting materials, in providing skin type information of individuals, in developing customized cosmetics, and in evaluating animal replacement skin pigmentation efficacy.

**<Rosacea skin>**

**Selection of subject according to rosacea skin, specialty history taking, evaluation through device applicable to the human body, lactic acid sting test, and skin specimen collection**

The subjects waited under constant temperature and humidity condition for 30 minutes after they washed their face, and next, the survey and specialty history taking for the subjects were conducted. After that, the cheeks of each subject were measured using devices applicable to the human body, and subjective irritation was evaluated through lactic acid sting test.

In specific, erythema dose was measured using Mexameter® MX 18 and analyzed with erythema index (EI) values. Further, skin redness was measured using Spectrophotometer (CM-700d) and analyzed with a* values. In addition, subjective irritation was evaluated using "5% lactic acid sting test" according to Frosch & Kligman method (1977). 50 $\mu\ell$ of lactic acid (5%) and 50 $\mu\ell$ of DW were dispensed on nasolabial folds, and in 0 minute, 2.5 minutes, 5 minutes, and 8 minutes, stinging, burning, and itching were graded directly by subjects according to intensity using a 4-point scale (where 0: no stinging, 1: slight stinging, 2: moderate stinging, and 3: severe stinging). The higher the numerical values of the sting intensity were, the more the subjects were sensitive. In the tests of the present invention, a difference value between lactic acid (5%) and DW was obtained and analyzed, and the expression of calculating the difference value was given as follows.

$$\text{Score} = (\text{Total lactic acid value} - \text{Total DW value})/12(4 \text{ Sections}*3 \text{ Items})$$

Table 15 shows the selection results of the subjects in which among total subjects, five control subjects had low erythema dose and redness and did not have any subjective irritation and five test subjects had high erythema dose and redness and did not have any subjective irritation.

[Table 15]

|  | Test group (rosacea) | Control group (control) |
|---|---|---|
| The number of samples (subjects) | 5 | 5 |
| Age (average $\pm$ SD) | 33.80 $\pm$ 6.94 | 39.00 $\pm$ 11.20 |
| Subjective irritation (score) | 0.20 $\pm$ 0.14 | 0.20 $\pm$ 0.25 |
| Erythema dose (EI) | 402.14 $\pm$ 50.13 | 250.48 $\pm$ 21.10 |
| Redness (a* value) | 12.99 $\pm$ 0.66 | 9.84 $\pm$ 0.59 |

Further, after the microneedle patch was attached to each subject's face for 15 minutes, a skin specimen was collected.

**Collection of RNA from skin specimen**

The skin specimens collected from the subjects were provided to RNeasy® Plus Mini Kit (Qiagen), and high purity RNA was extracted using the kit. Next, quality control (QC) of RNA samples was checked using Nanodrop and 2100 Bioanalyzer devices, and then, RNA microarray analysis was conducted.

**RNA microarray analysis**

The samples were subjected to flexible and high sensitive pre-treatment using GeneChip® WT Pico Kit (Applied Biosystems) through which a small amount of total RNA (100 pg) separated by the above-mentioned method was amplified to cRNA. The amplified RNA was subjected to microarray analysis according to the manual of GeneChip® Human Gene 2.0 ST Array (Affymetrix) platform. The microarray procedure introduced in the manual of platform was shown in FIGs. 4 and 5. The inventors conducted the microarray in the same procedure. The RNA microarray analysis protocols of the platform were the same as in Table 3.

After that, the entire data were summarized and normalized using Robust Multi-Average (RMA) of Affymetrix® Power Tools (APT), and the results were summarized to gene levels and subjected to differentially expressed gene (DEG) analysis. The statistical significance of the data was analyzed using independent t-test and variations of fold change, and false discovery rate (FDR) was adjusted in p value using Benjamini-Hochberg algorithm. DEG set was subjected to Hierarchical cluster analysis using linkage and Euclidean distance. Further, all data analysis and visualization work for checking gene expression differences were carried out using R 4.0.0 program.

**Selection of rosacea skin biomarker candidate through bioinformatics analysis and significant correlation analysis**

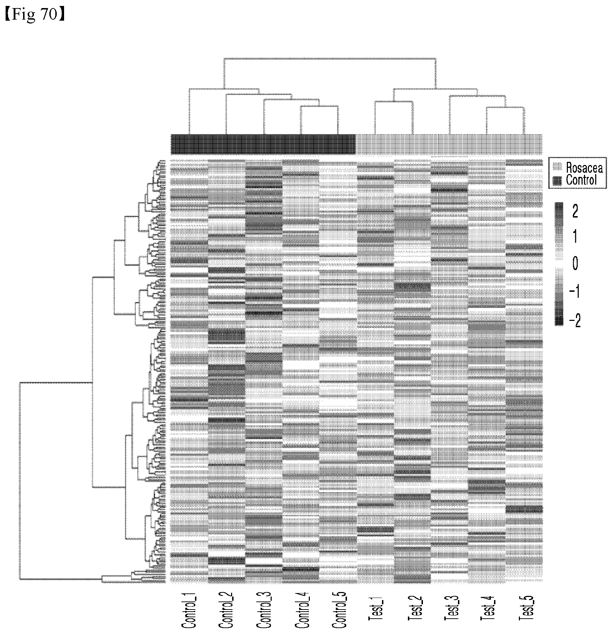

After the RNA microarray test was conducted, so as to check about forty thousand gene expression differences between the test group and the control group, gene alignment was performed using R 4.0.0 program. FIG. 70 shows gene alignment results. The gene alignment as shown in FIG. 70 is called heatmap analysis. The heatmap analysis is a term made by combining heat and map and represents various information expressed with colors in the form of heat distribution on an image. In the heatmap analysis described in the present invention, red colors mean gene expression values become high, and blue colors mean gene expression values become low. When hundreds of genes were analyzed, it was checked in FIG. 70 that there were specific differences between a plurality of gene expression values in the test group and the control group.

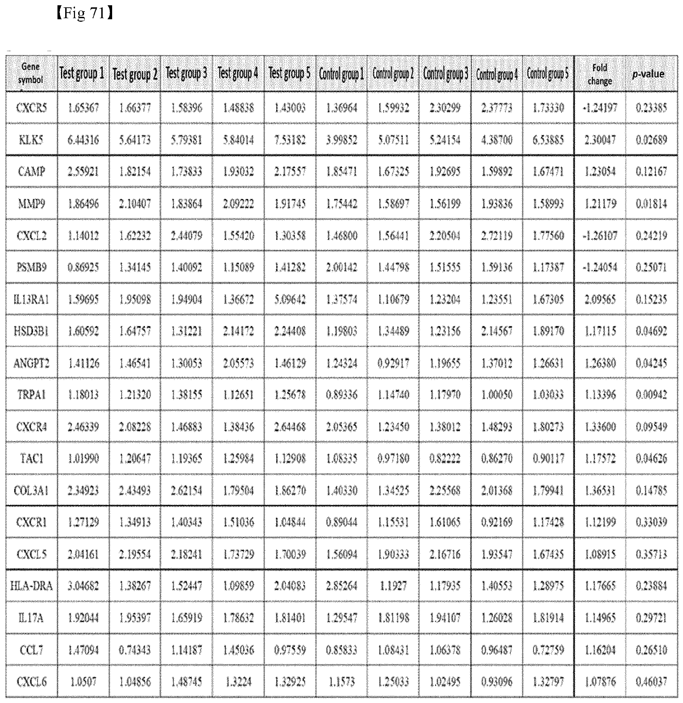

Through document analysis, after that, the inventors selected the genes related to rosacea skin (See FIG. 71), and they conducted the microarray test for the genes related to the rosacea skin, which were checked through the document analysis. The gene expression values checked through the microarray test results are summarized in the table of FIG. 71.

In the test group in which a degree of rosacea sensitivity was high and the control group in which a degree of rosacea sensitivity was not high, the differentiation in the gene expression values obtained by performing the microarray had to exist, so that the values might be useful as biomarkers related to the rosacea skin. To determine this, the inventors made use of fold changes and p-values. The fold changes are values that represent the measured values in the test group were higher or lower several times than those of the control group, and the p-values are values between the test group and the control group that represent statistically significant differences in statistical processing. The normalized signal values (expression values) of the respective samples obtained through the RNA microarray tests are represented in the table of FIG. 71, and the fold changes are values obtained by calculating 1:1 expression differences using the normalized signal values between the test group and the control group. The p-values are the values between 0 and 1 that represent the degrees of data coexisting with null hypothesis, and if the p-values are less than or equal to 0.05, generally, the null hypothesis is rejected.

As a result, it was checked that the genes related to the rosacea skin, which had the fold changes of 1.2 times or -1.2 times or the p-values less than or equal to 0.05, were COL3A1, TAC1, KLK5, CAMP, MMP9, TRPA1, IL13RA1, HSD3B1, CXCR4, ANGPT2, CXCL2, CXCR5, and PSMB9.

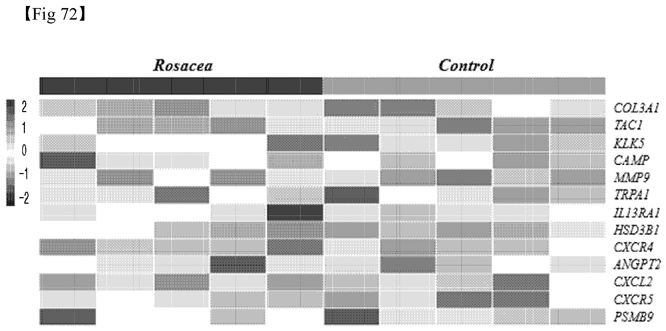

FIG. 72 shows heatmap analysis results so as to visually compare and analyze the expression levels of the rosacea skin biomarker candidates selected through the RNA microarray analysis. In the same manner as in FIG. 70, in the heatmap analysis as shown in FIG. 72, red colors mean gene expression values become high, and blue colors mean gene expression values become low.

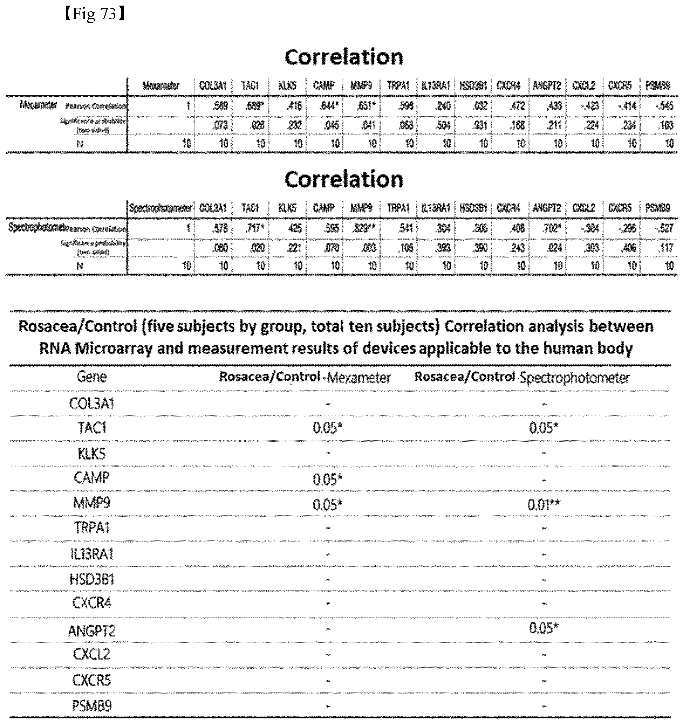

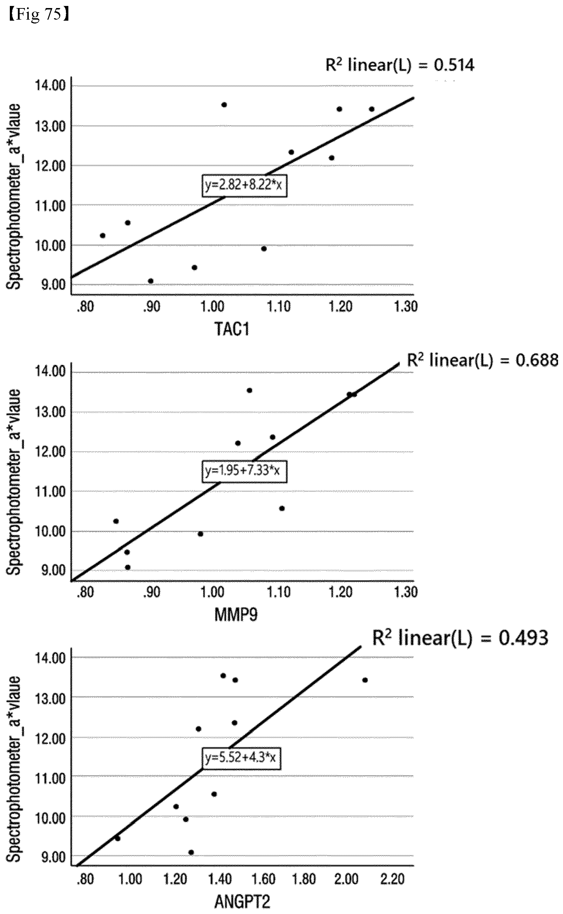

To check the correlation between the rosacea skin biomarker candidates, COL3A1, TAC1, KLK5, CAMP, MMP9, TRPA1, IL13RA1, HSD3B1, CXCR4, ANGPT2, CXCL2, CXCR5, and PSMB9 selected through the RNA microarray analysis and the measurement results of the devices (Mexameter and Spectrophotometer) applicable to the human body, bivariate correlation analysis was conducted. The results are shown in FIGs. 73 and 75.

FIG. 73 is a table showing the correlation values between the rosacea skin biomarker candidate genes and the devices applicable to the human body.

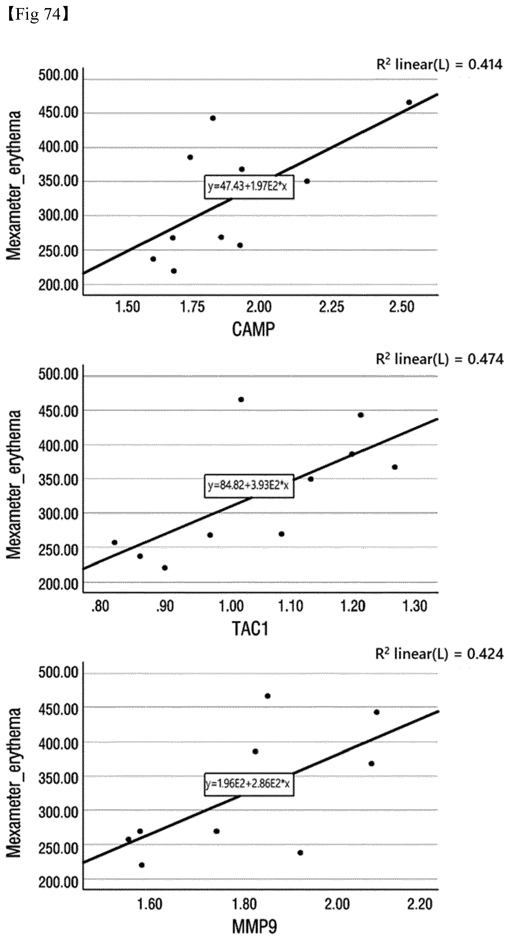

FIG. 74 shows scatter graphs for the genes having the significant correlation with the measurement values of Mexameter. As appreciated from the results of FIG. 74, it was checked that TAC1, CAMP, and MMP9 genes had statistically significant positive correlation with erythema dose (Mexameter).

FIG. 75 shows scatter graphs for the genes having the significant correlation with the measurement values of Spectrophotometer. As appreciated from the results of FIG. 75, it was checked that TAC1, MMP9, and ANGPT2 genes had statistically significant positive correlation with redness (Spectrophotometer, a*value).

When the results of the rosacea skin evaluation conducted by the inventors were totally analyzed, accordingly, total 13 genes COL3A1, TAC1, KLK5, CAMP, MMP9, TRPA1, IL13RA1, HSD3B1, CXCR4, ANGPT2, CXCL2, CXCR5, and PSMB9 were selected as the rosacea skin biomarkers in the widest range thereof. Further, in view of accurate and effective skin condition diagnosis, the inventors found that it was advantageous when the MMP9, TAC1, and CAMP genes among the genes were included in the biomarker group.

If such selection results are utilized, any one or two or more genes among the 13 RNA genes are used as the biomarkers, so that the rosacea skin evaluation may be performed quantitatively. Further, it will be predicted that the study results of the inventors may be used as very useful tools in a rosacea skin evaluation method or kit, in developing and screening of rosacea skin inducing or inhibiting materials, in providing skin type information of individuals, in developing customized cosmetics, and in evaluating animal replacement rosacea skin efficacy.

**\<Stinging skin\>**

**Selection of subject according to stinging skin, specialty history taking, lactic acid sting test, and skin specimen collection**

The subjects waited under constant temperature and humidity condition for 30 minutes after they washed their face, and next, the survey and specialty history taking for the subjects were conducted. After that, subjective irritation evaluation was conducted using lactic acid.

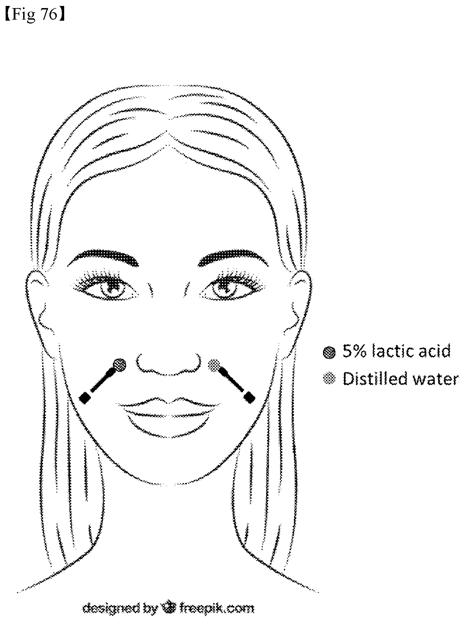

In specific, the subjective irritation was evaluated using "5% lactic acid sting test" according to Frosch & Kligman method (1977). FIG. 76 shows an example of lactic acid sting test. To do this, 50 $\mu\ell$ of lactic acid (5%) and 50 $\mu\ell$ of DW were dispensed on nasolabial folds, and in 0 minute, 2.5 minutes, 5 minutes, and 8 minutes, stinging, burning, and itching were graded directly by subjects according to intensity using a 4-point scale (where 0: no stinging, 1: slight stinging, 2: moderate stinging, and 3: severe stinging). The higher the numerical values of the sting intensity were, the more the subjects were sensitive. In the tests of the present invention, a difference value between lactic acid (5%) and DW was obtained and analyzed, and the expression of calculating the difference value was given as follows.

$$\text{Score} = (\text{Total lactic acid value} - \text{Total DW value})/12(4 \text{ Sections}*3 \text{ Items})$$

Table 16 shows the selection results of the subjects in which among the total subjects, five test subjects (test group) had stinging skin so that they had the lactic acid sting test score greater than 0.5 and five control subjects (control group) did not have any stinging skin so that they had the lactic acid sting test score of 0.

[Table 16] Table 16

|  | Test group | Control group |
|---|---|---|
| The number of samples (subjects) | 5 | 5 |
| Age (average $\pm$ SD) | 35.60 $\pm$ 13.03 | 25.80 $\pm$ 2.59 |
| Lactic acid sting test (score) | 0.65 $\pm$ 0.07 | 0.00 $\pm$ 0.00 |

Further, after the microneedle patch was attached to each subject's face for 15 minutes, a skin specimen was collected.

**Collection of RNA from skin specimen**

The skin specimens collected from the subjects were provided to RNeasy® Plus Mini Kit (Qiagen), and high purity RNA was extracted using the kit. Next, quality control (QC) of RNA samples was checked using Nanodrop and 2100 Bioanalyzer devices, and then, RNA microarray analysis was conducted.

**RNA microarray analysis**

The samples were subjected to flexible and high sensitive pre-treatment using GeneChip® WT Pico Kit (Applied Biosystems) through which a small amount of total RNA (100 pg) separated by the above-mentioned method was amplified to cRNA. The amplified RNA was subjected to microarray analysis according to the manual of GeneChip® Human Gene 2.0 ST Array (Affymetrix) platform. The microarray procedure introduced in the manual of platform was shown in FIGs. 4 and 5. The inventors conducted the microarray in the same procedure. The RNA microarray analysis protocols of the platform were the same as in Table 3.
After that, the entire data were summarized and normalized using Robust Multi-Average (RMA) of Affymetrix® Power Tools (APT), and the results were summarized to gene levels and subjected to differentially expressed gene (DEG) analysis. The statistical significance of the data was analyzed using independent t-test and variations of fold change, and false discovery rate (FDR) was adjusted in p value using Benjamini-Hochberg algorithm. DEG set was subjected to Hierarchical cluster analysis using linkage and Euclidean distance. Further, all data analysis and visualization work for checking gene expression differences were carried out using R 4.0.0 program.

**Selection of stinging skin biomarker candidate through bioinformatics analysis and significant correlation analysis**

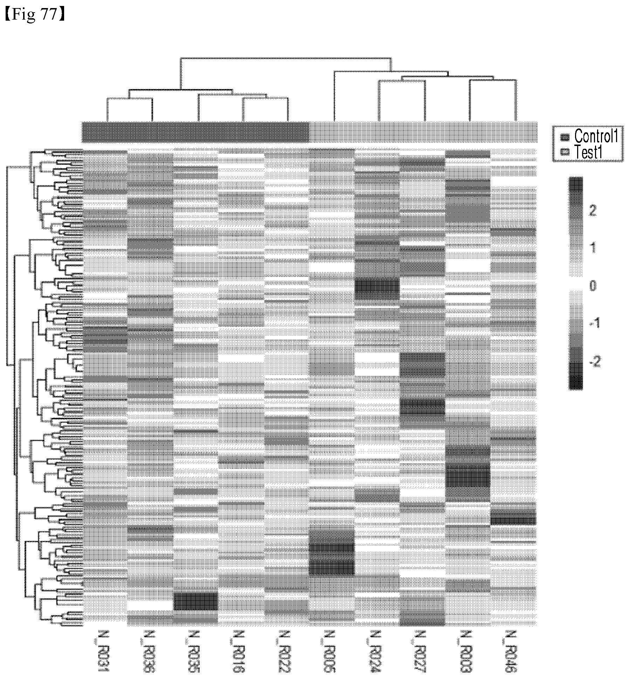

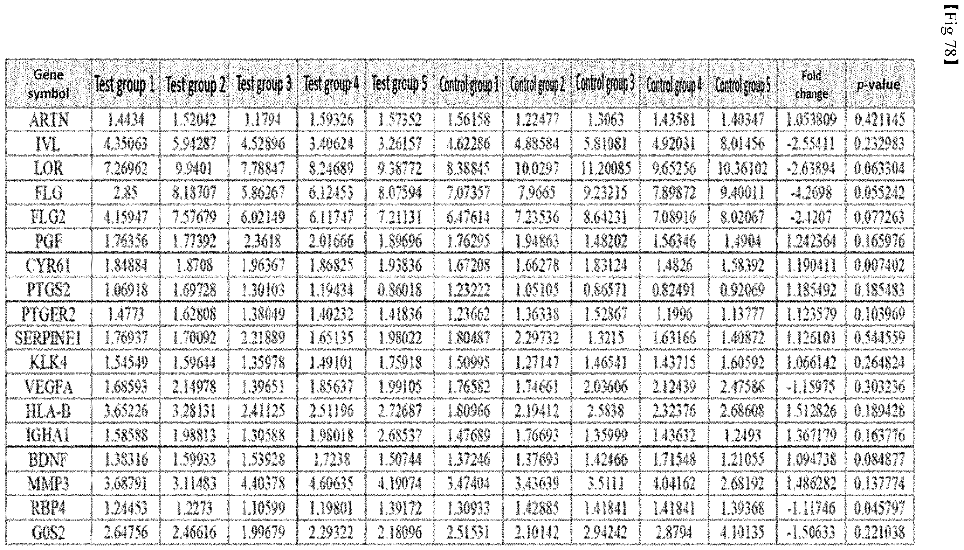

After the RNA microarray test was conducted, so as to check about forty thousand gene expression differences between the test group and the control group, gene alignment was performed using R 4.0.0 program. FIG. 77 shows gene alignment results. The gene alignment as shown in FIG. 77 is called heatmap analysis. The heatmap analysis is a term made by combining heat and map and represents various information expressed with colors in the form of heat distribution on an image. In the heatmap analysis described in the present invention, red colors mean gene expression values become high, and blue colors mean gene expression values become low. When hundreds of genes were analyzed, it was checked in FIG. 77 that there were specific differences between a plurality of gene expression values in the test group and the control group.
Through document analysis, after that, the inventors selected the genes related to stinging skin (See FIG. 78), and they conducted the microarray test for the genes related to the stinging skin, which were checked through the document analysis. The gene expression values checked through the microarray test results are summarized in the table of FIG. 78.

In the test group in which a degree of stinging skin was high and the control group in which a degree of stinging skin was not high, the differentiation in the gene expression values obtained by performing the microarray had to exist, so that the values might be useful as biomarkers related to the stinging skin. To determine this, the inventors made use of fold changes and p-values. The fold changes are values that represent the measured values in the test group were higher or lower several times than those of the control group, and the p-values are values between the test group and the control group that represent statistically significant differences in statistical processing. The normalized signal values (expression values) of the respective samples obtained through the RNA microarray tests are represented in the table of FIG. 78, and the fold changes are values obtained by calculating 1: 1 expression differences using the normalized signal values between the test group and the control group. The p-values are the values between 0 and 1 that represent the degrees of data coexisting with null hypothesis, and if the p-values are less than or equal to 0.05, generally, the null hypothesis is rejected.

As a result, it was checked that the genes related to the stinging skin, which had the fold changes of 1.2 times or -1.2 times or the p-values less than or equal to 0.05, were IVL, LOR, FLG, FLG2, PGF, CYR61, HLA-B, IGHA1, MMP3, RBP4, and G0S2.

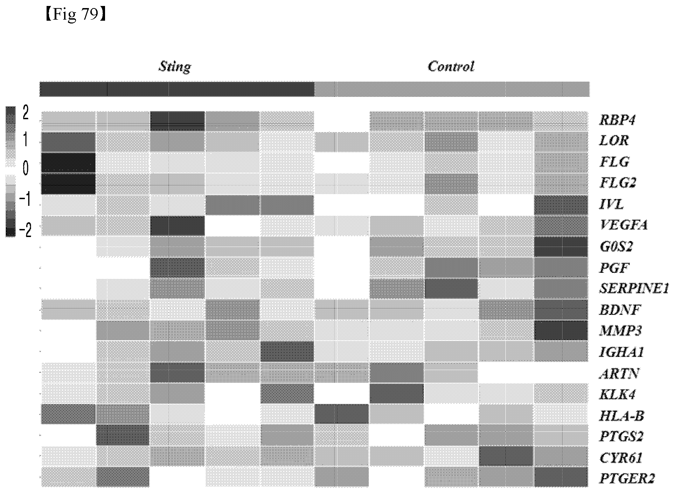

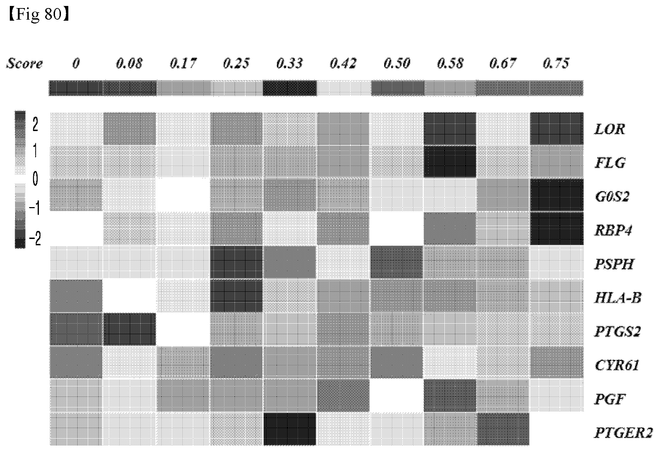

FIGs. 79 and 80 show heatmap analysis results so as to visually compare and analyze the expression levels of the stinging skin biomarker candidates selected through the RNA microarray analysis. FIG. 79 is a heatmap showing the expression of the stinging skin biomarker candidates using the RNA microarray analysis results after the total ten subjects including the five test subjects having the lactic acid sting test score greater than 0.5 and the five control subjects having the score of 0 were selected. FIG. 80 is a heatmap showing the expression of the stinging skin biomarker candidates using the RNA microarray average values by score after the total 33 subjects were sorted by lactic acid sting test score. In the same manner as in FIG. 77, in the heatmap analysis results as shown in FIGs. 79 and 80, red colors mean gene expression values become high, and blue colors mean gene expression values become low.

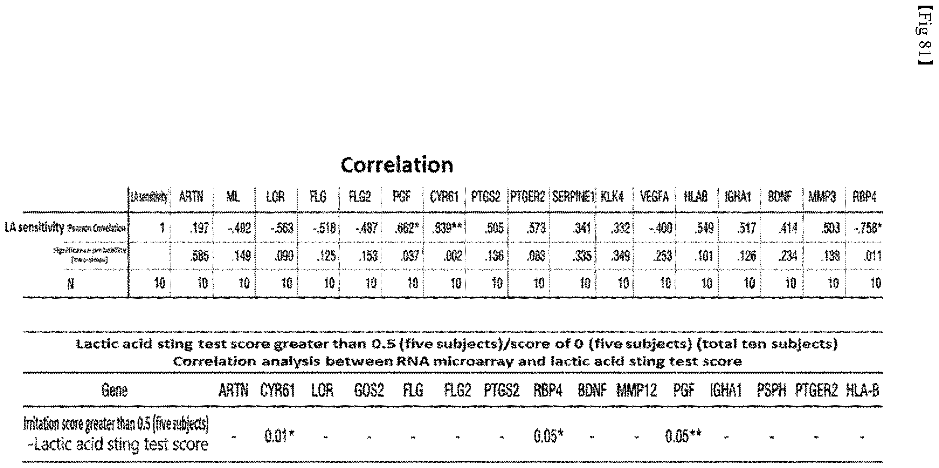

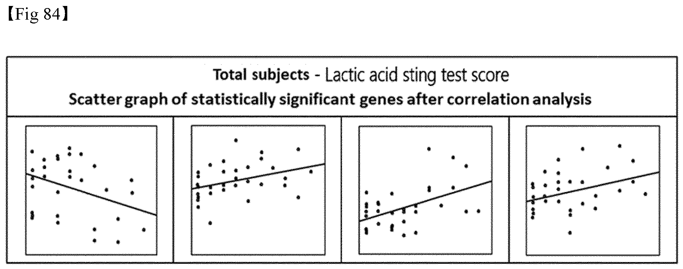

To check the correlations between the stinging skin biomarker candidates, IVL, LOR, FLG, FLG2, PGF, CYR61, HLA-B, IGHA1, MMP3, RBP4, and G0S2 selected through the RNA microarray analysis and the lactic acid sting test scores, bivariate correlation analysis was conducted. The results are shown in FIGs. 81 and 84.

As a result, it was checked that the CYR61, RBP4, and PGF genes of the RNA microarray analysis results for total ten subjects including the five test subjects who had the lactic acid sting test score greater than 0.5 and the five control subjects who had the score of 0 had statistically significant positive or negative correlation with the lactic acid sting test score and the LOR, PTGS2, PGF, and HLA-B genes of the RNA microarray analysis results for total 33 subjects had statistically significant negative or positive correlation with the lactic acid sting test score. When the RNA microarray analysis results of the subjects and the lactic acid sting tests were totally analyzed, accordingly, the inventors selected total 11 genes, IVL, LOR, FLG, FLG2, PGF, CYR61, HLA-B, IGHA1, MMP3, RBP4, and G0S2 as the stinging skin biomarkers. Further, in view of accurate and effective skin condition diagnosis, the inventors found that it was advantageous when the PGF, RBP4, and CYR61 genes among the genes were included in the biomarker group.

If such selection results are utilized, any one or two or more genes among the 11 RNA genes are used as the biomarkers, so that the stinging skin evaluation may be performed quantitatively. Further, it will be predicted that the study results of the inventors may be used as very useful tools in a stinging skin evaluation method or kit, in developing and screening of stinging skin inducing or inhibiting materials, in providing skin type information of individuals, in developing customized cosmetics, and in evaluating animal replacement stinging skin efficacy.

**<Acne cosmetica>**

**Selection of subject according to acne cosmetica, specialty history taking, evaluation through device applicable to the human body, lactic acid sting test, and skin specimen collection**

The subjects waited under constant temperature and humidity condition for 30 minutes after they washed their face, and next, the survey and specialty history taking for the subjects were conducted. After that, skin sebum was measured using a device applicable to the human body, and skin irritation evaluation was conducted using lactic acid sting test.

In specific, the skin sebum was measured using Sebumeter® SM815 and analyzed with $\mu g/cm^2$ values. The skin irritation was evaluated using "5% lactic acid sting test" according to Frosch & Kligman method (1977). To do this, 50 $\mu \ell$ of lactic acid (5%) and 50 $\mu \ell$ of DW were dispensed on nasolabial folds, and on 0 minute, 2.5 minutes, 5 minutes, and 8 minutes, stinging, burning, and itching were graded directly by subjects according to intensity using a 4-point scale (where 0: no stinging, 1: slight stinging, 2: moderate stinging, and 3: severe stinging). The higher the numerical values of the sting intensity were, the more the subjects were sensitive. In the tests of the present invention, a difference value between lactic acid (5%) and DW was obtained and analyzed, and the expression of calculating the difference value was given as follows.

$$\text{Score} = (\text{Total lactic acid value} - \text{Total DW value})/12(4 \text{ Sections}*3 \text{ Items})$$

The physical findings of the acne cosmetica of the subjects were obtained by the visual determination of a dermatologist on the basis of document related thereto (Korean acne severity system, Korean Journal of Dermatology: Vol.24 No.10, 2004), and the physical findings of the skin sebum of the subjects were obtained by the visual determination of a dermatologist on the basis of document related thereto (Reprod Biol Endocrinol. 2017 Homburg et al.).

Among the total subjects, five subjects (test group) who had acne cosmetica according to the visual determination of the dermatologist and five subjects (control group) who had no acne cosmetica and the lactic acid sting test score less than or equal to 0.5 were selected, and the selection results of the test group and the control group were listed in Table 17.

[Table 17]

|  | Test group | Control group |
|---|---|---|
| The number of samples (subjects) | 5 | 5 |
| Age (average ± SD) | 31.40 ± 9.81 | 36.00 ± 11.42 |
| Physical findings (acne) | O | X |
| Physical findings (sebum score) | 2.60 ± 0.89 | 1.40 ± 0.55 |
| Sebumeter ($\mu$g/cm$^2$) | 108.10 ± 59.93 | 75.20 ± 26.04 |
| Lactic acid sting test (score) | 0.35 ± 0.26 | 0.17 ± 0.23 |

Further, after the microneedle patch was attached to each subject's face for 15 minutes, a skin specimen was collected.

**Collection of RNA from skin specimen**

The skin specimens collected from the subjects were provided to RNeasy® Plus Mini Kit (Qiagen), and high purity RNA was extracted using the kit. Next, quality control (QC) of RNA samples was checked using Nanodrop and 2100 Bioanalyzer devices, and then, RNA microarray analysis was conducted.

**RNA microarray analysis**

The samples were subjected to flexible and high sensitive pre-treatment using GeneChip® WT Pico Kit (Applied Biosystems) through which a small amount of total RNA (100 pg) separated by the above-mentioned method was amplified to cRNA. The amplified RNA was subjected to microarray analysis according to the manual of GeneChip® Human Gene 2.0 ST Array (Affymetrix) platform. The microarray procedure introduced in the manual of platform was shown in FIGs. 4 and 5. The inventors conducted the microarray in the same procedure. The RNA microarray analysis protocols of the platform were the same as in Table 3.

After that, the entire data were summarized and normalized using Robust Multi-Average (RMA) of Affymetrix® Power Tools (APT), and the results were summarized to gene levels and subjected to differentially expressed gene (DEG) analysis. The statistical significance of the data was analyzed using independent t-test and variations of fold change, and false discovery rate (FDR) was adjusted in p value using Benjamini-Hochberg algorithm. DEG set was subjected to Hierarchical cluster analysis using linkage and Euclidean distance. Further, all data analysis and visualization work for checking gene expression differences were carried out using R 4.0.0 program.

**Selection of acne biomarker candidate through bioinformatics analysis and significant correlation analysis**

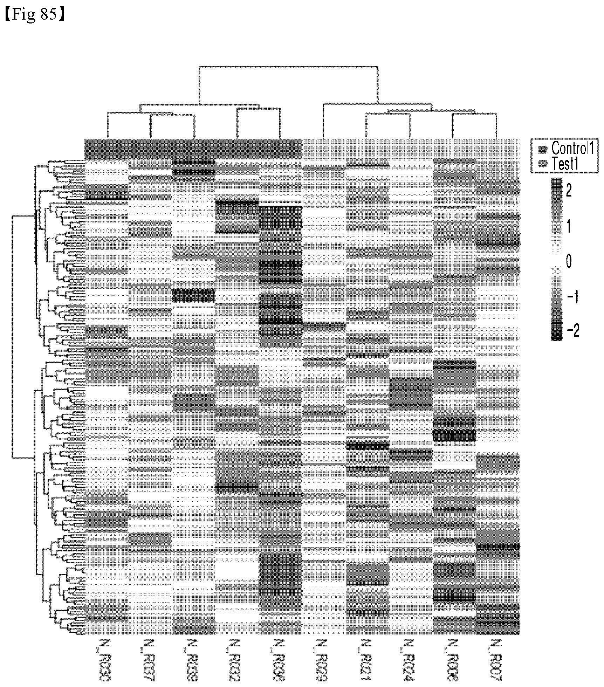

After the RNA microarray test was conducted, so as to check about forty thousand gene expression differences between the test group and the control group, gene alignment was performed using R 4.0.0 program. FIG. 85 shows gene alignment results. The gene alignment as shown in FIG. 85 is called heatmap analysis. The heatmap analysis is a term made by combining heat and map and represents various information expressed with colors in the form of heat distribution on an image. In the heatmap analysis described in the present invention, red colors mean gene expression values become high, and blue colors mean gene expression values become low. When hundreds of genes were analyzed, it was checked in FIG. 85 that there were specific differences between a plurality of gene expression values in the test group and the control group.

Through document analysis, after that, the inventors selected the genes related to acne cosmetica, and they conducted the microarray test of the genes related to the acne cosmetica, which were checked through the document analysis. The gene expression values checked through the microarray test results are summarized in the table of FIG. 85.

In the test group in which a degree of acne cosmetica activity was high and the control group in which a degree of acne cosmetica activity was not high, the differentiation in the gene expression values obtained by performing the microarray had to exist, so that the values might be useful as biomarkers related to acne cosmetica evaluation. To determine this, the inventors made use of fold changes and p-values. The fold changes are values that represent the measured values in the test group were higher or lower several times than those of the control groups, and the p-values are the values between 0 and 1 that represent the degree of data coexisting with null hypothesis. If the p-values are less than or equal to 0.05, the null hypothesis is rejected. The p-values are values between the test groups and the control groups that represent statistically significant differences in statistical processing.

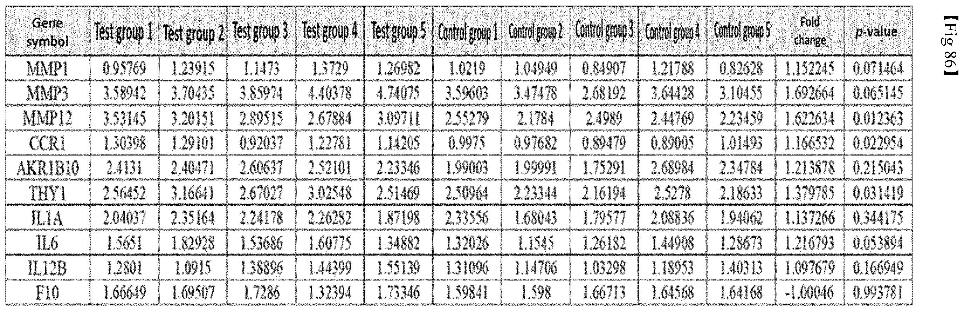

As a result, it was checked that the genes related to the acne cosmetica listed in the table of FIG. 86, which had the fold changes of 1.2 times or -1.2 times or the p-values less than or equal to 0.05, were MMP3, MMP12, CCR1, AKR1B10, THY1, and IL-6.

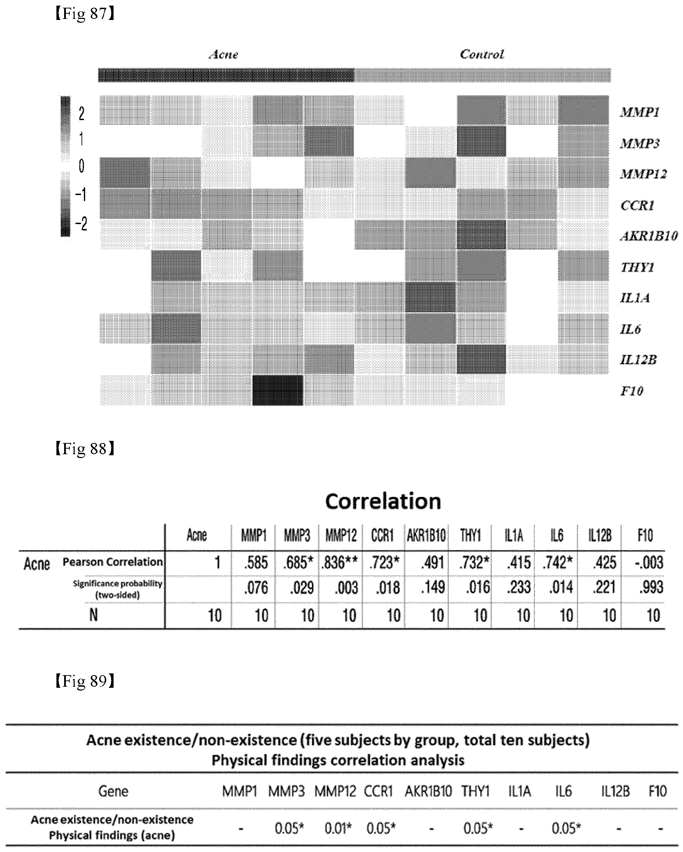

FIG. 87 shows heatmap analysis results so as to visually compare and analyze the expression levels of the acne cosmetica biomarker candidates selected through the RNA microarray analysis after the total ten subjects including the five test subjects having the high acne cosmetica activity and the five control subjects having no acne cosmetica and the lactic acid sting test score less than or equal to 0.5 had been selected according to the physical findings of a dermatologist. In the same manner as in FIG. 84, in the heatmap analysis results as shown in FIG. 87, red colors mean gene expression values become high, and blue colors mean gene expression values become low. From the test results as shown in FIG. 87, it was visually checked that the specific gene expression values selected through the document analysis in the test group were higher with red colors than those in the control group.

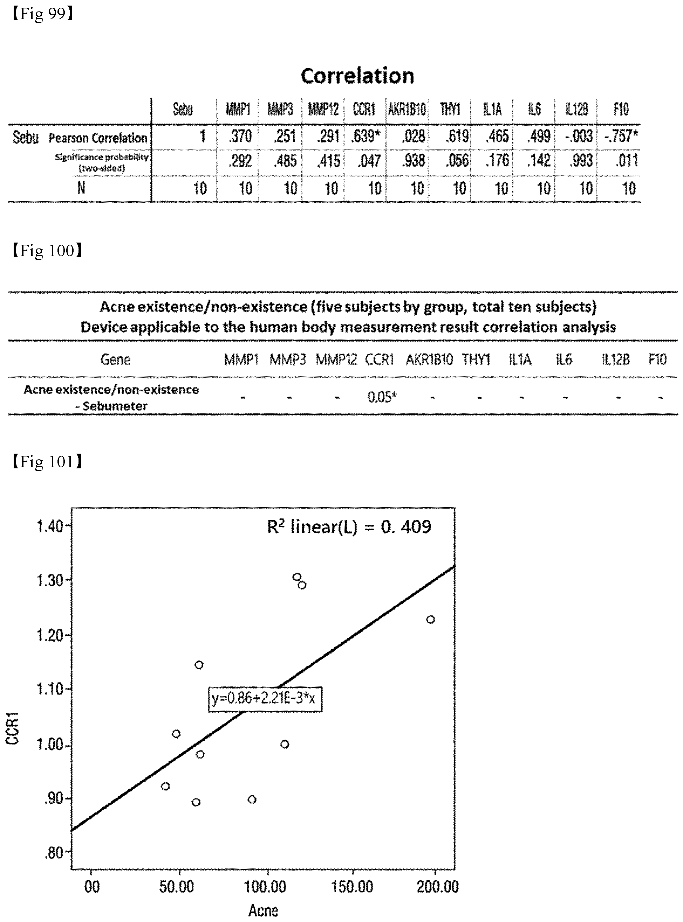

To check the correlations among the acne cosmetica biomarker candidates, MMP3, MMP12, CCR1, AKR1B10, THY1, and IL-6 selected through the RNA microarray analysis, acne cosmetica existence/non-existence and oily skin visual determination according to the physical findings of the dermatologist, and the device (Sebumeter) applicable to the human body, bivariate correlation analysis was conducted. The results are shown in FIGs. 88 and 101.

The inventors analyzed the correlations among the acne cosmetica biomarker candidates selected through the RNA microarray analysis of the total ten subjects including the five test subjects having the acne cosmetica and the five control subjects having no acne cosmetica and the lactic acid sting test score less than or equal to 0.5 according to the physical findings of the dermatologist, the scores (existence: +1, non-existence: 0) of the acne cosmetica existence/non-existence according to the physical findings of the dermatologist, and the measurement results of the device (Sebumeter).

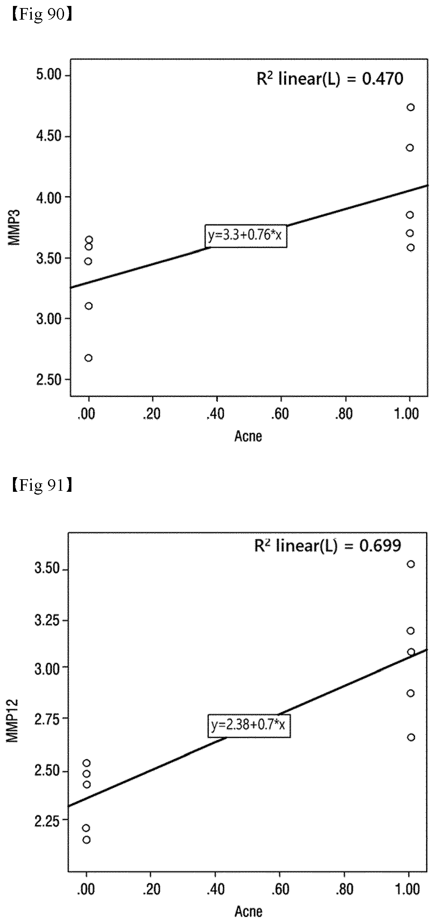

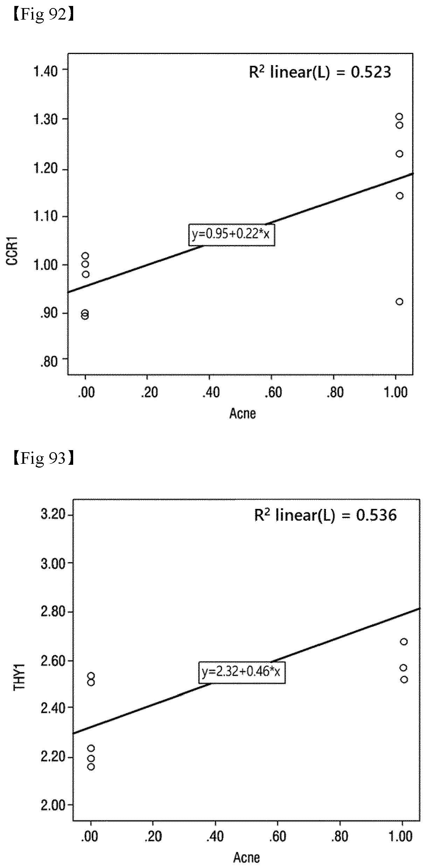

As a result, it was checked that the MMP3, MMP12, CCR1, THY1, and IL6 genes had statistically significant positive correlation with the scores of the acne cosmetica existence/non-existence according to the physical findings of the dermatologist (See FIGs. 88 to 94). FIGs. 88 and 89 are tables showing the correlation analysis results with the physical findings. FIGs. 90 to 94 show scatter graphs for the five genes having the significant positive correlation.

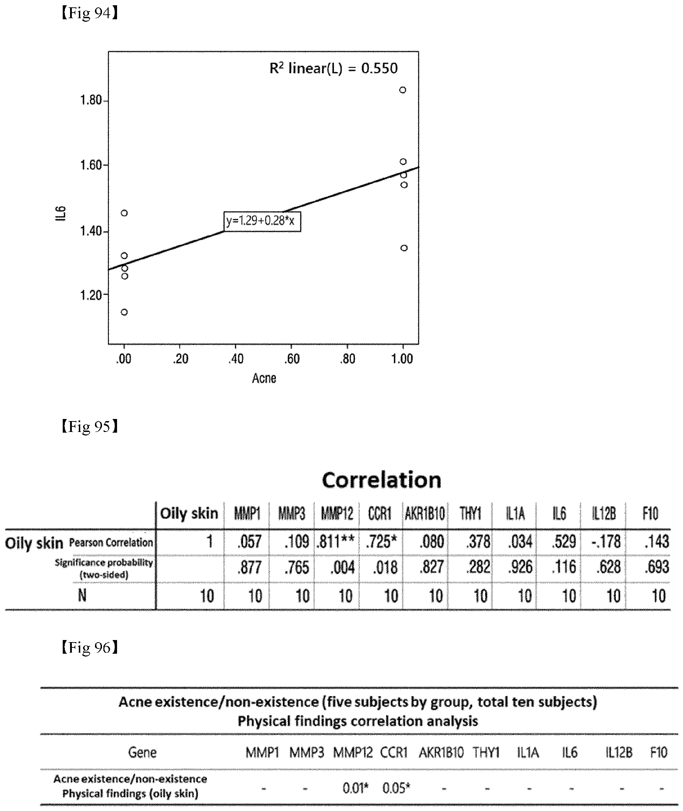

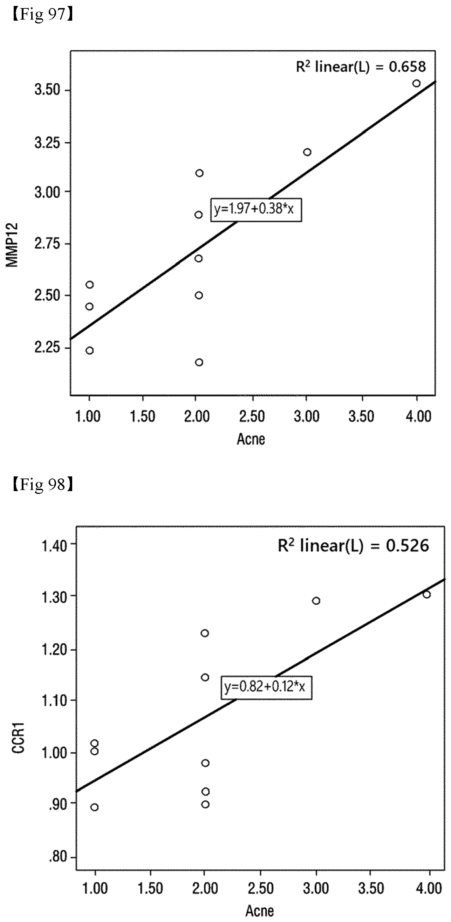

Further, it was checked that the MMP12 and CCR1 genes had statistically significant positive correlation with the sebum scores according to the visual determination of the dermatologist (See FIGs. 95 to 98). FIGs. 95 and 96 are tables showing the correlation analysis results with the sebum score according to the visual determination. FIGs. 97 and 98 show scatter graphs for the two genes having the significant positive correlation.

Furthermore, it was checked that the CCR1 gene had statistically significant positive correlation with the measurement results of the device (Sebumeter) applicable to the human body (See FIGs. 99 to 101). FIGs. 99 and 100 are tables showing the correlation analysis results with the measurement results of the device (Sebumeter) applicable to the human body. FIG. 101 shows a scatter graph for the one gene having the significant positive correlation.

When the results of the acne cosmetica evaluation tests conducted by the inventors were totally analyzed, accordingly, the inventors selected total six genes, MMP3, MMP12, CCR1, AKR1B10, THY1, and IL-6 as the acne cosmetica biomarkers in the widest range thereof. Further, in view of accurate and effective skin condition diagnosis, the inventors found that it was advantageous when the MMP3, MMP12, and CCR1 genes among the genes were included in the biomarker group.

If such selection results are utilized, any one or two or more genes among the six RNA genes are used as the biomarkers, so that acne cosmetica prediction may be performed quantitatively. Further, it will be predicted that the study results of the inventors may be used as very useful tools in an acne cosmetica prediction method or kit, in developing and screening of acne cosmetica inducing or inhibiting materials or treating agents, in providing skin type information of individuals, in developing customized cosmetics, and in evaluating animal replacement acne cosmetica efficacy.

In the above-mentioned embodiments of the present invention, the RNAs were extracted from the skin specimens obtained from the skin of subjects through the microneedles, and they were provided to the RNA microarray quantitative analysis device so that the expression levels of the RNA genes were quantitatively analyzed. However, the present invention cannot be limited to the specific quantification method as mentioned above. Only if the expression levels of the RNA genes are quantitatively analyzed using the skin specimens, they will be within the scope of the present invention. For example, a method for measuring levels of protein, such as ELISA test may be applied. The RNA biomarkers are expressed and produced to thus determine specifically the types of proteins, and through the quantification of the proteins, the expression levels of the RNA genes may be quantified. That is, the quantifying devices or kits used in the present invention may be ELISA, RT-PCR kit, RNA chip, DNA chip, or protein chip kit. The measurement of the levels of protein may be performed using one or more methods selected among ELISA, western blotting, magnet bead-immunoprecipitation, immunohistochemistry, and mass spectrometry. Further, the measurement of the levels of mRNA may be performed using one or more methods selected among competitive RT-PCT, real time RT-PCR, Rnase protection assay (RPA), northern blotting, and DNA chip.

According to the characteristics of the present invention, the non-invasion method using the microneedles is provided to measure and evaluate the skin condition, while utilizing the specific RNA biomarkers, and so as to detect the specific RNA biomarkers, both of the RNA-level test and the protein-level test may be utilized. However, in the case of the application of the RNA-level test, the following advantages may be obtained when compared to the application of the protein-level test. As a technology in which the RNA sample having a relatively smaller amount than the protein sample is amplified is applied, a substantially large number of biomarkers even from the sample having a small amount may be detected efficiently and precisely, which is considered as the advantages of the RNA-level test.

One of differences in practice use between the protein level test and the RNA level test in observing or quantifying the expression levels of the specific biomarkers is in that the RNA level test is capable of observing or quantifying a great number of biomarkers even from a very extreme amount of RNA when compared to the protein level test. So as to simultaneously see the changes of various biomarkers in the protein levels, proteome analysis is proper, but for the proteome analysis, a large number of samples are needed. In specific, if proteins are extracted from skin tissues, the extraction yield is less than or equal to 10%, and accordingly, the number of samples has to be increased by 10 times. In more specific, at least 1 to 2 mg of protein sample is needed for the proteome analysis, but about 0. 15 mg of protein is extracted by the 3 mm punch skin tissue biopsy. That is, when the 3 mm punch skin tissue biopsy is carried out ten times, the proteome analysis can be conducted.

In view of several limitations of the tissue biopsy, however, it is actually hard to try the 3 mm punch skin tissue biopsy ten times. Even in the case of extracting the protein using the microneedle patch, about 0.06 mg of protein is extracted by one microneedle patch, and accordingly, when the microneedle patches greater than or equal to 20 pieces are attached to the skin, the proteome analysis can be conducted, so that it is hard to apply the proteome analysis to the skin for practical use. Even if the protein is extracted from the sample, about 650 biomarkers are extracted through the proteome analysis, and therefore, they just correspond to 1 to 2% of the biomarkers (greater than about forty thousand biomarkers) detected through the RNA microarray analysis, thereby showing low efficacy.

Even if other analysis methods are conducted, they have limitations. Even in the case of the ELISA method as the most sensitive test of the protein analysis methods, 0.06 mg of protein extracted by one microneedle patch is just the amount with which only the expression changes of one biomarker can be checked upon repeated analysis of three times. According to the characteristics of the ELISA method, only one biomarker per one kit is detected, and an interference phenomenon occurs according to solvents. Further, a large number of steps have to be performed by a tester, and big variations may be caused whenever he or she measures the protein levels. Unlike RNA, the protein has a three-dimensional structure and is hard to be amplified, and accordingly, it cannot overcome the limitations in the amount of specimen collected and has difficulties in simultaneously analyzing the changes of the plurality of biomarkers for prognosis and diagnosis of skin type or skin diseases, advanced material treatment efficacy evaluation, and the like.

To solve such problems, according to the present invention, the RNA is applied for the analysis. So as to synthesize specific proteins, the genes participating in the synthesis of the proteins from the DNA existing in a cell nucleus are copied (transferred) to the form of RNA, and the RNA moves to cytoplasm to allow the amino acids corresponding to the nucleic sequences to be connected to one another with the RNA as template, so that the proteins are finally synthesized (translated). If the RNA as the precursor of the protein synthesis and the genes of reflecting the expression amounts of the skin proteins changing according to various environmental factors is utilized for the analysis, the analysis results may have high efficiency.

However, RNA has lower yield and higher instability than protein, and so as to allow RNA to be utilized for analysis, accordingly, studies of setting the RNA to optimal conditions are required. According to the present invention, it is checked that the RNA biomarkers can be reliably checked from an extremely small amount of skin specimen obtained using the microneedle patch. Before the present invention is introduced, as mentioned above, it is generally recognized in the art that RNA has lower yield and higher instability than protein, so that it is impossible that the RNA biomarkers are checked from the extremely small amount of skin specimen obtained using the microneedle patch to thus diagnose a specific skin condition or disease.

The inventor(s) checks for the first time that so as to ensure an adequate small amount of RNA for the analysis, the RNA amplification is performed to efficiently or accurately detect the RNA biomarkers from the extremely small amount of skin specimen obtained using the microneedle patch.

When calculated into the same unit, an amount of protein extracted per one microneedle patch is about 60 ng/$\mu$L, but an amount of RNA is about 10 ng/$\mu$L, which is extracted to 1/6 of the amount of protein extracted. To overcome the extremely smaller amount of RNA than the amount of protein, the RNA amplification is adopted so that the RNA microarray analysis can be conducted. The RNA amplification allows 10 ng of RNA to be amplified to 5500 ng of RNA increased by about 550 times through polymerase reaction principle. Using such amplification, the expression changes of about forty thousand biomarkers can be checked from the skin specimen extracted from one microneedle patch, and the plurality of biomarkers selected to measure, evaluate, or diagnose the respective skin conditions in the above-mentioned embodiments of the present invention can be detected very easily and precisely.

However, if the expression of a plurality of biomarkers, for example, nine protein biomarkers is detected accurately in the method acquiring the skin specimen using the microneedle patch, the detection is impossible even with the amount of skin specimen obtained using one microneedle patch. Accordingly, several microneedle patches have to be used, and a process of acquiring the skin specimen from one microneedle patch has to be performed several times. As mentioned above, if the ELISA method is used, only one biomarker per one kit is detectable, and accordingly, several kits have to be required. Further, the interference phenomenon may occur according to solvents, and a large number of steps have to be performed by the tester, so that big variations may be caused whenever he or she measures the protein levels.

The foregoing description of the embodiments of the invention has been presented for the purpose of illustration; it is not intended to be exhaustive or to limit the invention to the precise forms disclosed. Persons skilled in the relevant art can appreciate that many modifications and variations are possible in light of the above teachings.

Thus, although the invention has been described with respect to specific embodiments, it will be appreciated that the invention is intended to cover all modifications and equivalents within the scope of the following claims.

**Claims**

1.    A minimally invasive kit for diagnosing a skin condition, comprising:

a device for quantifying the expression levels of RNA genes from a specimen extracted from the skin of a subject; and
a microneedle patch comprising a plurality of microneedles having a solid structure and made of a bioidegradable polymer hyaluronic acid,
wherein when the microneedle patch is applied to the skin of the subject and is detached after applied for a predetermined time, the specimen attached to the microneedle surfaces of the microneedle patch from the skin of the subject or an extract extracted from the specimen is quantitatively analyzed by the quantifying device, and the quantifying device quantifies the expression levels of RNA biomarker genes related to collagen/elasticity or the expression levels of RNA biomarker genes related to skin barrier function/water synthesis from the specimen attached to the microneedle surfaces of the microneedle patch from the skin of the subject or the extract extracted from the specimen, such that diagnosis of skin aging is conducted based on the quantified values by the quantifying device, the biomarkers related to the collagen/elasticity comprising one or two genes selected from COL1A1, COL3A1, FN1, GSTA3, PON1, PINK1, COL4A4, and MMP8, and the biomarkers related to the skin barrier function/water synthesis comprising one or two genes selected from IVL, HAS2, HAS3, AQP3, CERS6, CLDN1, SLC9A1, TGM1, SPINK5, KLF4, LCE1A, LCE1B, LCE1F, LCE2A, BGN, and AZGP1.

2.    The minimally invasive kit according to claim 1, wherein the biomarkers related to the collagen/elasticity comprise the COL1A1, COL3A1, FN1, and PINK1 and the biomarkers related to the skin barrier function/water synthesis comprise IVL, HAS3, AQP3, BGN, and AZGP1.

3.    A minimally invasive kit for diagnosing a skin condition, comprising:

a device for quantifying the expression levels of RNA genes from a specimen extracted from the skin of a subject; and
a microneedle patch comprising a plurality of microneedles having a solid structure and made of a bioidegradable polymer hyaluronic acid,
wherein when the microneedle patch is applied to the skin of the subject and is detached after applied for a predetermined time, the specimen attached to the microneedle surfaces of the microneedle patch from the skin of the subject or an extract extracted from the specimen is quantitatively analyzed by the quantifying device, and the quantifying device quantifies the expression levels of RNA biomarker genes related to skin hydration from the specimen attached to the microneedle surfaces of the microneedle patch from the skin of the subject or the extract extracted from the specimen, such that skin hydration evaluation is conducted based on the quantified values by the quantifying device, the biomarkers related to the skin hydration comprising one or two genes selected from CDSN, FLG, FLG2, LOR, KLF4, KRT10, LCE1A, LCE2A, LCE2B, LCE2C, SMPD3, CDH1, ITGB4, IVL, SPINKS, CLDN1, AQP3, BGN, HAS3, TGM1, CLDN7, CERS3, CLDN4, and KRT1.

4. The minimally invasive kit according to claim 3, wherein the biomarkers related to the skin hydration comprise the FLG, AQP3, and LOR.

5. A minimally invasive kit for diagnosing a skin condition, comprising:

a device for quantifying the expression levels of RNA genes from a specimen extracted from the skin of a subject; and
a microneedle patch comprising a plurality of microneedles having a solid structure and made of a bioidegradable polymer hyaluronic acid,
wherein when the microneedle patch is applied to the skin of the subject and is detached after applied for a predetermined time, the specimen attached to the microneedle surfaces of the microneedle patch from the skin of the subject or an extract extracted from the specimen is quantitatively analyzed by the quantifying device, and the quantifying device quantifies the expression levels of RNA biomarker genes related to skin pigmentation from the specimen attached to the microneedle surfaces of the microneedle patch from the skin of the subject or the extract extracted from the specimen, such that skin pigmentation evaluation is conducted based on the quantified values by the quantifying device, the biomarkers related to the skin pigmentation comprising one or two genes selected from CAT, CLU, DSG1, GPNMB, GPX4, GSTM2, GSTP1, MLANA, PAX3, SOX10, TFAP2A, TYR, TYRP1, MC1R, F2RL1, and CLDN1.

6. The minimally invasive kit according to claim 5, wherein the biomarkers related to the skin pigmentation comprise the MLANA, TYRP1, and GPNMB.

7. A minimally invasive kit for diagnosing a skin condition, comprising:

a device for quantifying the expression levels of RNA genes from a specimen extracted from the skin of a subject; and
a microneedle patch comprising a plurality of microneedles having a solid structure and made of a bioidegradable polymer hyaluronic acid,
wherein when the microneedle patch is applied to the skin of the subject and is detached after applied for a predetermined time, the specimen attached to the microneedle surfaces of the microneedle patch from the skin of the subject or an extract extracted from the specimen is quantitatively analyzed by the quantifying device, and the quantifying device quantifies the expression levels of RNA biomarker genes related to skin sebum from the specimen attached to the microneedle surfaces of the microneedle patch from the skin of the subject or the extract extracted from the specimen, such that skin sebum evaluation is conducted based on the quantified values by the quantifying device, the biomarkers related to the skin sebum comprising one or two genes selected from MFAP2, IGF1, HSD11B1, GPAM, CPT1C, AR, MPZL3, AQP3, SREBF2, and HSD17B2.

8. The minimally invasive kit according to claim 7, wherein the biomarkers related to the skin sebum comprise the MPZL3, GPAM, and IGF1.

9. A minimally invasive kit for diagnosing a skin condition, comprising:

a device for quantifying the expression levels of RNA genes from a specimen extracted from the skin of a subject; and
a microneedle patch comprising a plurality of microneedles having a solid structure and made of a bioidegradable polymer hyaluronic acid,
wherein when the microneedle patch is applied to the skin of the subject and is detached after applied for a predetermined time, the specimen attached to the microneedle surfaces of the microneedle patch from the skin of the subject or an extract extracted from the specimen is quantitatively analyzed by the quantifying device, and the quantifying device quantifies the expression levels of RNA biomarker genes related to rosacea skin from the specimen attached to the microneedle surfaces of the microneedle patch from the skin of the subject or the extract extracted from the specimen, such that rosacea skin evaluation is conducted based on the quantified values by the quantifying device, the biomarkers related to the rosacea skin comprising one or two genes selected from COL3A1, TAC1, KLK5, CAMP, MMP9, TRPA1, IL13RA1, HSD3B1, CXCR4, ANGPT2, CXCL2, CXCR5, and PSMB9.

10. The minimally invasive kit according to claim 9, wherein the biomarkers related to the rosacea skin comprise the MMP9, TAC1, and CAMP.

11. A minimally invasive kit for diagnosing a skin condition, comprising:

a device for quantifying the expression levels of RNA genes from a specimen extracted from the skin of a subject; and
a microneedle patch comprising a plurality of microneedles having a solid structure and made of a bioidegradable polymer hyaluronic acid,
wherein when the microneedle patch is applied to the skin of the subject and is detached after applied for a predetermined time, the specimen attached to the microneedle surfaces of the microneedle patch from the skin of the subject or an extract extracted from the specimen is quantitatively analyzed by the quantifying device, and the quantifying device quantifies the expression levels of RNA biomarker genes related to stinging skin from the specimen attached to the microneedle surfaces of the microneedle patch from the skin of the subject or the extract extracted from the specimen, such that stinging skin evaluation is conducted based on the quantified values by the quantifying device, the biomarkers related to the stinging skin comprising one or two genes selected from IVL, LOR, FLG, FLG2, PGF, CYR61, HLA-B, IGHA1, MMP3, RBP4, and G0S2.

12. The minimally invasive kit according to claim 11, wherein the biomarkers related to the stinging skin comprise the PGF, RBP4, and CYR61.

13. A minimally invasive kit for diagnosing a skin condition, comprising:

a device for quantifying the expression levels of RNA genes from a specimen extracted from the skin of a subject; and
a microneedle patch comprising a plurality of microneedles having a solid structure and made of a bioidegradable polymer hyaluronic acid,
wherein when the microneedle patch is applied to the skin of the subject and is detached after applied for a predetermined time, the specimen attached to the microneedle surfaces of the microneedle patch from the skin of the subject or an extract extracted from the specimen is quantitatively analyzed by the quantifying device, and the quantifying device quantifies the expression levels of RNA biomarker genes related to acne cosmetica from the specimen attached to the microneedle surfaces of the microneedle patch from the skin of the subject or the extract extracted from the specimen, such that acne cosmetica prediction is conducted based on the quantified values by the quantifying device, the biomarkers related to the acne cosmetica comprising one or two genes selected from MMP3, MMP12, CCR1, AKR1B10, THY1, and IL-6.

14. The minimally invasive kit according to claim 13, wherein the biomarkers related to the acne cosmetica comprise the MMP3, MMP12, and CCR1.

15. The minimally invasive kit according to any one of claims 1 to 14, further comprising a device for extracting RNA from the skin specimen of the subject so that the RNA acquired by the extracting device is amplified and thus provided to the quantifying device.

【Fig 1】

【Fig 2】

【Fig 3】

【Fig 4】

【Fig 5】

GeneChip™ WT Pico Reagent Kit Amplification and Labeling process.

【Fig 6】

【Fig 7】

| Gene symbol | Test group 1 | Test group 2 | Test group 3 | Test group 4 | Test group 5 | Control group 1 | Control group 2 | Control group 3 | Control group 4 | Control group 5 | Fold change | p-value |
|---|---|---|---|---|---|---|---|---|---|---|---|---|
| CAT | 3.39758 | 2.08054 | 1.96992 | 1.52817 | 1.56070 | 1.79530 | 2.03412 | 2.11451 | 1.53633 | 2.28753 | 1.11251 | 0.71154 |
| CELA1 | 1.19887 | 1.42899 | 1.41162 | 1.57426 | 1.36881 | 1.15670 | 1.36872 | 1.51241 | 1.19267 | 1.55578 | 1.02758 | 0.706603 |
| COL1A1 | 1.91693 | 2.49049 | 2.01095 | 2.25531 | 2.26490 | 2.53149 | 2.63416 | 2.26955 | 2.30532 | 2.26490 | -1.15939 | 0.123312 |
| COL1A2 | 0.84238 | 1.04025 | 0.74481 | 0.94847 | 0.95934 | 1.06797 | 0.98360 | 1.12506 | 0.94783 | 1.25881 | -1.12475 | 0.116778 |
| COL3A1 | 1.36721 | 1.85229 | 1.70093 | 1.74389 | 1.34525 | 2.01221 | 2.25568 | 1.79504 | 1.79720 | 1.95879 | -1.28509 | 0.044265 |
| FN1 | 3.91608 | 4.29657 | 2.93012 | 4.06057 | 4.75377 | 5.10736 | 5.38315 | 4.44536 | 4.84623 | 4.72079 | -1.87795 | 0.02583 |
| GSR | 1.23923 | 0.85876 | 1.44372 | 1.69252 | 1.21504 | 1.38301 | 1.23761 | 1.31528 | 1.58871 | 1.46861 | -1.07832 | 0.334138 |
| GSTA3 | 1.24197 | 1.17498 | 1.38235 | 1.61847 | 1.04030 | 2.35239 | 1.98378 | 1.74844 | 1.65554 | 1.65571 | -1.50270 | 0.03297 |
| MSN | 1.35004 | 1.62144 | 1.28994 | 1.11742 | 1.35004 | 1.43988 | 1.50671 | 1.53779 | 1.78484 | 1.64868 | -1.17920 | 0.139812 |
| PINK1 | 4.09984 | 5.32576 | 4.60727 | 4.60702 | 3.75948 | 5.75337 | 7.22581 | 5.46109 | 6.19055 | 5.91819 | -3.09498 | 0.001735 |
| PON1 | 0.83537 | 0.89839 | 1.05434 | 1.35309 | 0.99327 | 1.40906 | 1.30310 | 1.20504 | 1.24277 | 1.06575 | -1.16332 | 0.146675 |
| GPX1 | 3.25321 | 2.11891 | 2.54316 | 2.39911 | 2.09559 | 2.26498 | 2.96792 | 2.19785 | 2.48527 | 2.58007 | -1.01201 | 0.959752 |
| GDF11 | 1.22491 | 1.23418 | 1.04185 | 1.04369 | 1.17162 | 1.41323 | 1.23762 | 1.00457 | 1.57520 | 1.23465 | -1.10942 | 0.218586 |

[Fig 8]

EP 4 194 565 A1

| Gene symbol | Test group 1 | Test group 2 | Test group 3 | Test group 4 | Test group 5 | Control group 1 | Control group 2 | Control group 3 | Control group 4 | Control group 5 | Fold change | p-value |
|---|---|---|---|---|---|---|---|---|---|---|---|---|
| IVL | 4.35063 | 2.75812 | 3.77012 | 3.72786 | 2.22203 | 4.88584 | 7.04138 | 7.04895 | 5.81081 | 8.01456 | -9.15498 | 0.02393 |
| HAS2 | 2.12249 | 1.53172 | 1.55009 | 1.64392 | 1.35360 | 2.28090 | 2.46462 | 1.55649 | 1.28716 | 1.59279 | -1.14554 | 0.40482 |
| HAS3 | 1.85278 | 1.62873 | 2.29918 | 1.65672 | 1.37057 | 1.96133 | 3.12730 | 1.76078 | 2.02406 | 1.90475 | -1.31408 | 0.29969 |
| AQP3 | 2.55227 | 2.55259 | 4.26861 | 3.09282 | 2.66858 | 3.29720 | 4.37891 | 3.76206 | 4.58861 | 5.83088 | -2.53952 | 0.09075 |
| CERS1 | 1.48122 | 1.53923 | 1.74091 | 2.25247 | 1.30047 | 2.02673 | 1.75617 | 1.64392 | 1.89720 | 1.91227 | -1.13634 | 0.37532 |
| CERS2 | 1.46035 | 0.99887 | 1.18894 | 1.06488 | 1.18190 | 1.34010 | 1.14161 | 1.15428 | 1.41532 | 1.43806 | -1.08590 | 0.24667 |
| CERS6 | 1.40671 | 1.01151 | 1.30653 | 0.98808 | 1.30586 | 1.34918 | 1.91146 | 1.71288 | 3.51976 | 1.98413 | -1.85542 | 0.113 |
| CLDN1 | 3.61597 | 3.69958 | 4.78474 | 3.41044 | 3.39346 | 3.60824 | 5.20828 | 4.04402 | 6.27297 | 6.15590 | -2.42342 | 0.15271 |
| SLC9A1 | 1.34322 | 1.62111 | 1.61582 | 1.34520 | 1.26671 | 1.60368 | 2.31468 | 1.89179 | 1.33152 | 1.89001 | -1.29049 | 0.04711 |
| TGM1 | 2.23707 | 2.78985 | 5.17356 | 1.71029 | 2.21571 | 4.30617 | 4.47778 | 5.02257 | 6.01535 | 6.62418 | -5.51711 | 0.04559 |
| SPINK5 | 7.53781 | 8.39461 | 10.08302 | 6.80275 | 8.69044 | 9.32645 | 10.52116 | 10.32285 | 11.17868 | 11.15026 | -4.58892 | 0.02965 |
| KLF4 | 2.46039 | 2.69633 | 3.79392 | 2.72514 | 3.24815 | 3.20311 | 3.84160 | 4.32222 | 4.34387 | 4.17894 | -1.99054 | 0.00601 |
| ACAN | 1.03989 | 1.06575 | 1.02410 | 1.09071 | 1.07362 | 1.15722 | 1.15035 | 1.09993 | 1.00755 | 1.17107 | -1.04132 | 0.18073 |
| BGN | 2.25594 | 2.10932 | 3.08693 | 1.77489 | 1.85119 | 2.62142 | 2.75063 | 2.10725 | 3.33720 | 3.20444 | -1.50372 | 0.26077 |
| LCE1A | 4.31684 | 7.16760 | 7.40801 | 3.65442 | 3.05448 | 6.61091 | 6.82119 | 6.96042 | 8.25380 | 7.69224 | -4.43042 | 0.12822 |
| LCE1B | 1.98926 | 2.63822 | 2.81912 | 1.70249 | 1.89734 | 2.52009 | 3.35001 | 2.04982 | 3.44524 | 3.78827 | -1.76712 | 0.16261 |
| LCE1F | 3.51780 | 9.43714 | 7.54237 | 7.28338 | 2.46470 | 8.57947 | 8.64563 | 7.97091 | 9.22162 | 9.64515 | -6.79031 | 0.13445 |
| LCE2A | 5.04383 | 6.34808 | 5.83165 | 4.40585 | 3.94969 | 5.83853 | 5.39860 | 5.54395 | 6.62542 | 6.88241 | -1.92114 | 0.26739 |

【Fig 9】

【Fig 10】

【Fig 11】

【Fig 12】

【Fig 13】

# Correlation

| | | Age | CM | TW | pH | PRMS | CT | PINK1 | CCL1A1 | FN1 | GSTA3 | PON1 | MSN | GDF11 | ROS1 | COL3A1 | HAS2 |
|---|---|---|---|---|---|---|---|---|---|---|---|---|---|---|---|---|---|
| Age | Pearson Correlation | 1 | -191 | -088 | -271 | 930** | -.855** | -.812** | -.513 | -.811** | -.778** | -.512 | -.578 | -.467 | -.497 | -.630 | -.716* |
| | Significance probability (two-sided) | | .597 | .809 | .450 | .000 | .002 | .004 | .071 | .004 | .008 | .131 | .080 | .174 | .144 | .051 | .020 |
| | N | 10 | 10 | 10 | 10 | 10 | 10 | 10 | 10 | 10 | 10 | 10 | 10 | 10 | 10 | 10 | 10 |
| CM | Pearson Correlation | -.191 | 1 | -.527 | -.284 | -.103 | .485 | -.121 | -.408 | -.135 | -.105 | .146 | -.080 | -.078 | -.372 | -.095 | .160 |
| | Significance probability (two-sided) | .597 | | .117 | .426 | .778 | .155 | .739 | .241 | .710 | .773 | .688 | .826 | .830 | .291 | .794 | .659 |
| | N | 10 | 10 | 10 | 10 | 10 | 10 | 10 | 10 | 10 | 10 | 10 | 10 | 10 | 10 | 10 | 10 |
| TW | Pearson Correlation | .088 | -.527 | 1 | .691* | -.195 | -.207 | .376 | .518 | .181 | .225 | .445 | .152 | .138 | .339 | .532 | .196 |
| | Significance probability (two-sided) | .809 | .117 | | .027 | .598 | .566 | .284 | .125 | .618 | .532 | .198 | .674 | .704 | .338 | .113 | .587 |
| | N | 10 | 10 | 10 | 10 | 10 | 10 | 10 | 10 | 10 | 10 | 10 | 10 | 10 | 10 | 10 | 10 |
| pH | Pearson Correlation | -.271 | -.284 | .691* | 1 | -.464 | .243 | .532 | .658* | .324 | .426 | .728* | .163 | .410 | -.042 | .643* | -.117 |
| | Significance probability (two-sided) | .450 | .426 | .027 | | .177 | .498 | .113 | .039 | .361 | .220 | .017 | .652 | .239 | .908 | .045 | .747 |
| | N | 10 | 10 | 10 | 10 | 10 | 10 | 10 | 10 | 10 | 10 | 10 | 10 | 10 | 10 | 10 | 10 |
| PRMS | Pearson Correlation | .930** | -.103 | -.195 | -.464 | 1 | -.877** | -.823** | -.654* | -.676* | -.834** | -.650* | -.545 | -.595 | -.462 | -.734* | -.609 |
| | Significance probability (two-sided) | .000 | .778 | .588 | .177 | | .001 | .003 | .040 | .032 | .003 | .042 | .104 | .070 | .179 | .016 | .062 |
| | N | 10 | 10 | 10 | 10 | 10 | 10 | 10 | 10 | 10 | 10 | 10 | 10 | 10 | 10 | 10 | 10 |
| CT | Pearson Correlation | -.855** | .485 | -.207 | .243 | -.877** | 1 | .634* | .406 | .563 | .604 | .496 | .517 | .550 | -.538 | .520 | .459 |
| | Significance probability (two-sided) | .002 | .155 | .566 | .498 | .001 | | .049 | .244 | .090 | .064 | .145 | .126 | .100 | .108 | .123 | .182 |
| | N | 10 | 10 | 10 | 10 | 10 | 10 | 10 | 10 | 10 | 10 | 10 | 10 | 10 | 10 | 10 | 10 |

Twenties/sixties (five subjects by age group, total ten subjects) RNA Microarray - orrelation analysis between age and measurement results of devices applicable to the human body

| Gene | COL1A1 | COL3A1 | FN1 | GSTA3 | PON1 | PINK1 |
|---|---|---|---|---|---|---|
| Twenties/sixties-age | — | — | 0.01** | 0.01** | — | 0.01** |
| Twenties/sixties-PRIMOS | 0.05** | 0.05** | 0.05** | 0.01** | 0.05** | 0.01** |
| Twenties/sixties-Cutometer | — | — | — | — | — | 0.05** |
| Twenties/sixties-Curneometer | — | — | — | — | — | — |
| Twenties/sixties-Tewameter | — | — | — | — | — | — |
| Twenties/sixties-pHmeter | — | — | — | — | — | — |

【Fig 14】

Scatter graph of statistically significant genes after correlation analysis

## Correlation

| | | Age | CM | TW | pH | PRMS | CT | PINK1 | CCL1A1 | FN1 | GSTA3 | PON1 | MSN | GDF11 | ROS1 | COL3A1 | HAS2 |
|---|---|---|---|---|---|---|---|---|---|---|---|---|---|---|---|---|---|
| Age | Pearson Correlation | 1 | -.014 | -.154 | -.279 | .807** | -.700** | -.662** | -.504* | -.753** | -.736** | -.310 | -.314 | -.360 | .334 | -.431 | -.432 |
| | Significance probability (two-sided) | | .954 | .516 | .234 | .000 | .000 | .001 | .023 | .000 | .000 | .183 | .177 | .119 | .151 | .058 | 0.57 |
| | N | 20 | 20 | 20 | 20 | 19 | 20 | 20 | 20 | 20 | 20 | 20 | 20 | 20 | 20 | 20 | 20 |
| CM | Pearson Correlation | -.014 | 1 | -.721** | -.326 | -.139 | .308 | -.031 | -.378 | -.189 | -.046 | -.042 | -.205 | .177 | -.266 | -.553* | -.254 |
| | Significance probability (two-sided) | .954 | | .000 | .160 | .570 | .186 | .896 | .100 | .424 | .847 | .861 | .386 | .454 | .257 | .11 | .280 |
| | N | 20 | 20 | 20 | 20 | 19 | 20 | 20 | 20 | 20 | 20 | 20 | 20 | 20 | 20 | 20 | 20 |
| TW | Pearson Correlation | -.154 | -.721** | 1 | .608** | -.051 | -.095 | .077 | -.572** | .228 | .150 | .527* | .255 | -.091 | .379 | .637** | .315 |
| | Significance probability (two-sided) | -.516 | .000 | | .004 | .836 | .690 | .745 | .008 | .334 | .529 | .017 | .279 | .703 | .100 | .003 | .176 |
| | N | 20 | 20 | 20 | 20 | 19 | 20 | 20 | 20 | 20 | 20 | 20 | 20 | 20 | 20 | 20 | 20 |
| pH | Pearson Correlation | -.279 | -.326 | .608** | 1 | -.365 | .164 | .331 | .610** | .297 | .399 | .547* | .127 | .278 | .047 | .476* | -.099 |
| | Significance probability (two-sided) | .234 | .160 | .004 | | .124 | .491 | .154 | .004 | .204 | .081 | .013 | .594 | .235 | .843 | .034 | .678 |
| | N | 20 | 20 | 20 | 20 | 19 | 20 | 20 | 20 | 20 | 20 | 20 | 20 | 20 | 20 | 20 | 20 |
| PRMS | Pearson Correlation | .807** | -.139 | -.051 | -.365 | 1 | -.799** | -.630** | -.436 | -.582** | -.695** | -.348 | -.267 | -.422 | .246 | -.305 | -.322 |
| | Significance probability (two-sided) | .000 | .570 | .836 | .124 | | 0.00 | .004 | .062 | .009 | .001 | .145 | .269 | .072 | .310 | .204 | .179 |
| | N | 19 | 19 | 19 | 19 | -.799* | 19 | 19 | 19 | 19 | 19 | 19 | 19 | 19 | 19 | 19 | 19 |
| CT | Pearson Correlation | -.700** | .308 | -.095 | .164 | .000 | 1 | .468** | .185 | .401 | .463* | .262 | .178 | .277 | -.179 | .174 | .221 |
| | Significance probability (two-sided) | .001 | .186 | 690 | .491 | .000 | | .37 | .434 | .079 | .040 | .264 | .452 | .238 | .450 | .464 | .350 |
| | N | 20 | 20 | 20 | 20 | 19 | 20 | 20 | 20 | 20 | 20 | 20 | 20 | 20 | 20 | 20 | 20 |

Five subjects by age group, total 20 subjects) RNA Microarray - Correlation analysis between age and measurement results of devices applicable to the human body

| Gene | COL1A1 | COL3A1 | FN1 | GSTA3 | PON1 | PINK1 |
|---|---|---|---|---|---|---|
| By age group-age | 0.05** | | 0.01** | 0.01** | — | 0.01** |
| By age group-PROMOS | — | — | 0.01** | 0.01** | — | 0.01** |
| By age group-Cutometer | — | — | — | 0.05** | — | 0.05** |
| By age group-Curneometer | — | — | — | — | — | — |
| By age group-Tewameter | — | — | — | — | — | — |
| By age group-pHmeter | — | — | — | — | — | — |

[Fig 15]

EP 4 194 565 A1

【Fig 16】

Scatter graph of statistically significant genes after correlation analysis

## Correlation

[Fig 17]

EP 4 194 565 A1

52

| | | primos | ct | IVL | HAS2 | HAS3 | AQP3 | CERS1 | CERS2 | CERS6 | CLDN1 | SLC9A1 | TGM1 | SPINK5 | KLF4 | ACAN | BGN | LCE1A | LCE1B | LCE1F | LCE2A |
|---|---|---|---|---|---|---|---|---|---|---|---|---|---|---|---|---|---|---|---|---|---|
| Age | Pearson Correlation | .748* | -.809** | -.829** | -.318 | -.394 | -.553 | -.252 | -.472 | -.647* | -.516 | -.495 | -.630 | -.673* | -.664* | -.507 | -.440 | -.436 | -.471 | -.374 | -.353 |
| | Significance probability (two-sided) | .013 | .005 | .003 | .371 | .260 | .098 | .483 | .168 | .043 | .127 | .146 | .051 | .033 | .036 | .135 | .203 | .208 | .170 | .287 | .317 |
| | N | 10 | 10 | 10 | 10 | 10 | 10 | 10 | 10 | 10 | 10 | 10 | 10 | 10 | 10 | 10 | 10 | 10 | 10 | 10 | 10 |
| cm | Pearson Correlation | -.144 | .736* | .274 | -.306 | .212 | .414 | .030 | .098 | .508 | .370 | .066 | .456 | .556 | .749* | -.175 | .304 | .144 | .113 | -.061 | -.116 |
| | Significance probability (two-sided) | .692 | .015 | .444 | .390 | .557 | .234 | .935 | .788 | .134 | .292 | .856 | .185 | .095 | .013 | .628 | .394 | .691 | .756 | .868 | .750 |
| | N | 10 | 10 | 10 | 10 | 10 | 10 | 10 | 10 | 10 | 10 | 10 | 10 | 10 | 10 | 10 | 10 | 10 | 10 | 10 | 10 |
| ph | Pearson Correlation | -.034 | -.004 | .171 | .197 | -.054 | .088 | .727* | .136 | .127 | .042 | -.035 | .188 | .023 | -.034 | .336 | .181 | .335 | .165 | .614 | .427 |
| | Significance probability (two-sided) | .926 | .991 | .637 | .585 | .881 | .809 | .017 | .708 | .727 | .908 | .924 | .603 | .949 | .926 | .343 | .618 | .344 | .647 | .059 | .218 |
| | N | 10 | 10 | 10 | 10 | 10 | 10 | 10 | 10 | 10 | 10 | 10 | 10 | 10 | 10 | 10 | 10 | 10 | 10 | 10 | 10 |
| tw | Pearson Correlation | .331 | -.561 | -.117 | .062 | -.385 | -.183 | .545 | -.128 | -.389 | -.284 | -.167 | -.252 | -.468 | -.494 | .303 | -.250 | -.046 | -.141 | .366 | .191 |
| | Significance probability (two-sided) | .350 | .092 | .748 | .864 | .271 | .613 | .104 | .725 | .267 | .427 | .645 | .483 | .173 | .147 | .395 | .487 | .899 | .697 | .299 | .597 |
| | N | 10 | 10 | 10 | 10 | 10 | 10 | 10 | 10 | 10 | 10 | 10 | 10 | 10 | 10 | 10 | 10 | 10 | 10 | 10 | 10 |
| primos | Pearson Correlation | 1 | -.549 | -.724* | -.732* | -.708* | -.340 | -.052 | -.444 | -.383 | -.342 | -.615 | -.371 | -.414 | -.329 | -.380 | -.363 | -.287 | -.350 | -.145 | -.183 |
| | Significance probability (two-sided) | | .100 | .018 | .016 | .022 | .337 | .888 | .199 | .275 | .333 | .058 | .292 | .234 | .353 | .278 | .303 | .422 | .321 | .690 | .613 |
| | N | 10 | 10 | 10 | 10 | 10 | 10 | 10 | 10 | 10 | 10 | 10 | 10 | 10 | 10 | 10 | 10 | 10 | 10 | 10 | 10 |
| ct | Pearson Correlation | -.549 | 1 | .685* | .048 | .365 | .553 | -.007 | .237 | .537 | .461 | .520 | .713* | .810** | .868** | .292 | .419 | .483 | .366 | .261 | .257 |
| | Significance probability (two-sided) | .100 | | .029 | .895 | .299 | .097 | .984 | .509 | .109 | .180 | .123 | .021 | .005 | .001 | .413 | .227 | .157 | .298 | .466 | .474 |
| | N | 10 | 10 | 10 | 10 | 10 | 10 | 10 | 10 | 10 | 10 | 10 | 10 | 10 | 10 | 10 | 10 | 10 | 10 | 10 | 10 |

Twenties/sixties (five subjects by age group, total ten subjects) Correlation analysis between RNA Microarray and measurement results of devices applicable to the human body

| Gene | IVL | HAS2 | HAS3 | AQP3 | CERS6 | CLDN1 | NHE1 | TGM1 | SPINK5 | KLF4 | BGN | LCE1A | LCE1B | LCE1F | LCE2A |
|---|---|---|---|---|---|---|---|---|---|---|---|---|---|---|---|
| Twenties/sixties-age | 0.01** | - | - | - | 0.05* | - | - | - | 0.05* | 0.05* | - | - | - | - | - |
| Twenties/sixties-PRIMOS | 0.05* | 0.05* | 0.05* | - | - | - | - | - | - | - | - | - | - | - | - |
| Twenties/sixties-Cutometer | 0.05* | - | - | - | - | - | - | - | 0.05* | 0.01** | 0.01** | - | - | - | - |
| Twenties/sixties-Curneometer | - | - | - | - | - | - | - | - | - | 0.05* | - | - | - | - | - |
| Twenties/sixties-Tewameter | - | - | - | - | - | - | - | - | - | - | - | - | - | - | - |
| Twenties/sixties-pHmeter | - | - | - | - | - | - | - | - | - | - | - | - | - | - | - |

【Fig 18】

Scatter graph of statistically significant genes after correlation analysis

[Fig 19]

## Correlation

| | | primos | ct | IVL | HAS2 | HAS3 | AQP3 | CERS1 | CERS2 | CERS6 | CLDN1 | SLC9A1 | TGM1 | SPINK5 | KLF4 | ACAN | BGN | LCE1A | LCE1B | LCE1F | LCE2A |
|---|---|---|---|---|---|---|---|---|---|---|---|---|---|---|---|---|---|---|---|---|---|
| Age | Pearson Correlation | .574* | -.670** | -.616** | -.201 | -.396 | -.412 | -.219 | -.394 | -.502* | -.368 | -.309 | -.485* | -.373 | -.445 | -.254 | -.341 | -.365 | -.335 | -.310 | -.108 |
| | Significance probability (two-sided) | .010 | .002 | .005 | .408 | .093 | .079 | .367 | .095 | .028 | .121 | .198 | .035 | .115 | .056 | .294 | .154 | .124 | .161 | .197 | .659 |
| | N | 19 | 19 | 19 | 19 | 19 | 19 | 19 | 19 | 19 | 19 | 19 | 19 | 19 | 19 | 19 | 19 | 19 | 19 | 19 | 19 |
| cm | Pearson Correlation | -.148 | .482* | .271 | -.456* | .044 | .222 | .018 | -.340 | .519* | .370 | -.012 | .340 | .408 | .632** | -.466* | .365 | -.006 | .172 | -.210 | -.079 |
| | Significance probability (two-sided) | .544 | .036 | .262 | .050 | .857 | .361 | .943 | .155 | .023 | .119 | .962 | .155 | .083 | .004 | .045 | .124 | .979 | .480 | .388 | .748 |
| | N | 19 | 19 | 19 | 19 | 19 | 19 | 19 | 19 | 19 | 19 | 19 | 19 | 19 | 19 | 19 | 19 | 19 | 19 | 19 | 19 |
| ph | Pearson Correlation | .022 | .007 | .182 | .125 | -.077 | .159 | .419 | .187 | .139 | .130 | -.030 | .216 | .118 | -.014 | .079 | .192 | .284 | .182 | .565* | .333 |
| | Significance probability (two-sided) | .929 | .978 | .457 | .610 | .754 | .516 | .074 | .443 | .571 | .595 | .904 | .374 | .631 | .953 | .748 | .431 | .239 | .457 | .012 | .163 |
| | N | 19 | 19 | 19 | 19 | 19 | 19 | 19 | 19 | 19 | 19 | 19 | 19 | 19 | 19 | 19 | 19 | 19 | 19 | 19 | 19 |
| tw | Pearson Correlation | .218 | -.360 | -.122 | .297 | -.264 | -.104 | .200 | .315 | -.429 | -.337 | -.086 | -.267 | -.384 | -.522* | .525* | -.295 | -.021 | -.150 | .372 | .080 |
| | Significance probability (two-sided) | .371 | .130 | .620 | .217 | .274 | .671 | .412 | .189 | .067 | .158 | .726 | .268 | .104 | .022 | .021 | .220 | .931 | .541 | .117 | .745 |
| | N | 19 | 19 | 19 | 19 | 19 | 19 | 19 | 19 | 19 | 19 | 19 | 19 | 19 | 19 | 19 | 19 | 19 | 19 | 19 | 19 |
| primos | Pearson Correlation | 1 | -.525* | -.282 | .383 | -.430 | -.219 | -.016 | -.132 | -.180 | -.173 | -.302 | -.154 | -.092 | -.131 | -.114 | -.461* | -.142 | -.285 | -.059 | .043 |
| | Significance probability (two-sided) | | .021 | .242 | .105 | .066 | .367 | .947 | .592 | .461 | .478 | .209 | .528 | .707 | .592 | .642 | .047 | .562 | .237 | .809 | .860 |
| | N | 19 | 19 | 19 | 19 | 19 | 19 | 19 | 19 | 19 | 19 | 19 | 19 | 19 | 19 | 19 | 19 | 19 | 19 | 19 | 19 |
| ct | Pearson Correlation | -.525* | 1 | .463* | -.028 | .250 | .395 | .014 | .012 | .414 | .407 | .316 | .592** | .488* | .624** | -.036 | .369 | .289 | .195 | .114 | .099 |
| | Significance probability (two-sided) | .021 | | .046 | .910 | .303 | .094 | .954 | .962 | .078 | .083 | .187 | .008 | .034 | .004 | .885 | .120 | .230 | .423 | .643 | .686 |
| | N | 19 | 19 | 19 | 19 | 19 | 19 | 19 | 19 | 19 | 19 | 19 | 19 | 19 | 19 | 19 | 19 | 19 | 19 | 19 | 19 |

**Five subjects by age group, total 20 subjects) Correlation analysis between RNA Microarray and measurement results of devices applicable to the human body**

| Gene | IVL | HAS2 | HAS3 | AQP3 | CERS6 | CLDN1 | NHE1 | TGM1 | SPINK5 | KLF4 | BGN | LCE1A | LCE1B | LCE1F | LCE2A |
|---|---|---|---|---|---|---|---|---|---|---|---|---|---|---|---|
| By age group-age | 0.01** | - | - | - | 0.05* | - | - | 0.05* | - | - | - | - | - | - | - |
| By age group-PROMOS | - | - | - | - | - | - | - | - | - | - | 0.05* | - | - | - | - |
| By age group-Cutometer | 0.05* | - | - | - | - | - | - | 0.01** | 0.05* | 0.01** | - | - | - | - | - |
| By age group - Comeometer | - | - | - | - | 0.05* | - | - | - | - | 0.01** | - | - | - | - | - |
| By age group-Tewameter | - | - | - | - | - | - | - | - | - | 0.05* | - | - | - | - | - |
| By age group-pHmeter | - | - | - | - | - | - | - | - | - | - | - | - | - | - | - |

【Fig 20】

Scatter graph of statistically significant genes after correlation analysis

【Fig 21】

【Fig 22】

| Roughness of wrinkles around the eyes (Ra) | 0W | 4W |
|---|---|---|
| T001 | 27.87 | 22.97 |
| R002 | 36.80 | 22.77 |

【Fig 23】

| Elasticity around the eyes (R2) | 0W | 4W |
|---|---|---|
| T001 | 0.4886 | 0.5909 |
| R002 | 0.5460 | 0.6252 |

【Fig 24】

| Water content around the eyes(A.U.) | 0W | 4W |
|---|---|---|
| T001 | 59.53 | 69.37 |
| R002 | 58.36 | 71.68 |

【Fig 25】

| Gene symbol | 0 W -1 | 0 W -2 | 4 W -1 | 4 W -2 | Fold change | p-value |
|---|---|---|---|---|---|---|
| COL1A1 | 2.449034 | 2.335002 | 2.699059 | 2.018936 | 1.023152 | 0.926065 |
| FN1 | 1.883158 | 1.849314 | 1.729957 | 1.825828 | 1.063149 | 0.403159 |
| MMP8 | 1.441456 | 1.436758 | 1.646401 | 1.691645 | -1.172766 | 0.068873 |
| CAT | 2.233172 | 2.290817 | 2.079501 | 2.232333 | 1.076298 | 0.268491 |
| GSTA3 | 2.263873 | 1.795039 | 1.755929 | 1.593184 | 1.278896 | 0.259192 |
| PINK1 | 3.100212 | 3.148795 | 2.975259 | 3.067531 | 1.074085 | 0.132909 |
| GDF11 | 1.485253 | 1.551538 | 1.367485 | 1.399861 | 1.097882 | 0.079698 |

【Fig 26】

| Gene symbol | 0 W -1 | 0 W -2 | 4 W -1 | 4 W -2 | Fold change | p-value |
|---|---|---|---|---|---|---|
| LCE1A | 4.092489 | 3.832023 | 3.026973 | 2.702675 | 2.139735 | 0.019 |
| LCE1B | 2.876677 | 2.840483 | 2.557539 | 2.642891 | 1.196122 | 0.147 |
| LCE1F | 4.006274 | 6.066337 | 2.006516 | 5.336328 | 2.575555 | 0.277 |
| LCE2A | 3.781727 | 2.695657 | 2.797444 | 2.711755 | 1.398706 | 0.51 |
| LOR | 8.123314 | 7.585344 | 4.87494 | 7.913719 | 2.751083 | 0.564 |
| AQP3 | 2.400722 | 2.348153 | 1.883315 | 2.338258 | 1.200513 | 0.488 |
| AQP10 | 2.170699 | 2.457443 | 1.579779 | 1.689616 | 1.601444 | 0.082 |
| BGN | 2.494806 | 2.505289 | 2.288379 | 2.116467 | 1.229119 | 0.189 |
| HAS1 | 1.94256 | 2.089252 | 1.887068 | 1.705898 | 1.164268 | 0.408 |
| HAS2 | 1.804053 | 1.862323 | 1.50461 | 1.462357 | 1.2743 | 0.091 |
| HAS3 | 2.094559 | 2.087211 | 1.609301 | 1.813139 | 1.30104 | 0.173 |
| CER1 | 1.768248 | 1.50009 | 1.37936 | 1.520033 | 1.136401 | 0.533 |
| CERS1 | 2.060184 | 1.681295 | 1.85749 | 1.668711 | 1.077464 | 0.461 |
| CERS3 | 2.04553 | 1.712301 | 1.783986 | 1.734231 | 1.086589 | 0.553 |
| KLF4 | 2.320606 | 2.985049 | 2.661772 | 2.405572 | 1.086099 | 0.839 |

【Fig 27】

✔ **Genes related to skin aging related to collagen/elasticity**

【Fig 28】

✔ **Genes related to skin aging related to skin barrier/moisture**

[Fig 29]

# Correlation

| | | PRIMOS | Cuto | Comeo | COL1A1 | COL4A4 | FN1 | MMP8 | CAT | GSTA3 | PINK1 | SFN | GDF11 | ACAN | BGN | HAS1 | HAS2 | HAS3 | CER1 | CERS1 | CERS3 |
|---|---|---|---|---|---|---|---|---|---|---|---|---|---|---|---|---|---|---|---|---|---|
| RIMOS | Pearson Correlation | 1 | -.521 | -.895 | .488 | -.963* | -.978* | .747 | -.942 | -.472 | -.959* | -.805 | -.860 | -.965* | -.560 | -.401 | -.775 | -.975* | -.747 | -.059 | -.311 |
| | Significance probability (two-sided) | | .479 | .105 | .512 | .037 | .022 | .253 | .058 | .528 | .041 | .195 | .140 | .035 | .440 | .599 | .225 | .025 | .253 | .941 | .689 |
| | N | 4 | 4 | 4 | 4 | 4 | 4 | 4 | 4 | 4 | 4 | 4 | 4 | 4 | 4 | 4 | 4 | 4 | 4 | 4 | 4 |
| Cuto | Pearson Correlation | -.521 | 1 | .846 | .489 | .731 | .502 | -.930 | .265 | .880 | .453 | .903 | .687 | .680 | .965* | .805 | .884 | .691 | .598 | .666 | .660 |
| | Significance probability (two-sided) | .479 | | .154 | .511 | .269 | .498 | .070 | .735 | .120 | .547 | .097 | .313 | .320 | .035 | .195 | .116 | 309 | .402 | .334 | .340 |
| | N | 4 | 4 | 4 | 4 | 4 | 4 | 4 | 4 | 4 | 4 | 4 | 4 | 4 | 4 | 4 | 4 | 4 | 4 | 4 | 4 |
| :omeo | Pearson Correlation | -.895 | .846 | 1 | -.048 | .982* | .866 | -.956* | .731 | .740 | .842 | .968* | .906 | .952* | .859 | .685 | .951* | .970* | .762 | .367 | .519 |
| | Significance probability (two-sided) | .105 | .154 | | .952 | .018 | .134 | .044 | .269 | .260 | .158 | .032 | .094 | .048 | .141 | .315 | .049 | .030 | .238 | .633 | .481 |
| | N | 4 | 4 | 4 | 4 | 4 | 4 | 4 | 4 | 4 | 4 | 4 | 4 | 4 | 4 | 4 | 4 | 4 | 4 | 4 | 4 |

## Before and after application Correlation analysis between RNA Microarray and measurement results of devices applicable to the human body

| Gene | COL1A1 | COL4A4 | FN1 | MMP8 | CAT | GSTA3 | PINK1 | GDF11 |
|---|---|---|---|---|---|---|---|---|
| Before and after application –PRIMOS | - | 0.05* | 0.05* | - | - | - | 0.05* | - |
| Before and after application –Cutometer | - | - | - | - | - | - | - | - |
| Before and after application –Corneometer | - | 0.05* | - | 0.05* | - | - | - | - |

【Fig 30】

Scatter graph of statistically significant genes after correlation analysis

# Correlation

[Fig 31]

EP 4 194 565 A1

| | | LCE1A | LCE1B | LCEF1 | LCE2A | LOR | AQP3 | AQP10 | AZGP1 | ACAN | BGN | HAS1 | HAS2 | HAS3 | CER1 | CERS1 | CERS3 | KLF4 | TGM1 | SPINK5 | CLDN1 |
|---|---|---|---|---|---|---|---|---|---|---|---|---|---|---|---|---|---|---|---|---|---|
| PRIMOS | Pearson Correlation | -.693 | -.933 | -.785 | -.416 | -.919 | -.954* | -.849 | -.557 | -.965* | -.560 | -.401 | -.775 | -.975* | -.747 | -.059 | -.311 | .014 | -.259 | -.823 | -.637 |
| | Significance probability (two-sided) | .307 | .067 | .215 | .584 | .081 | .046 | .151 | .443 | .035 | .440 | .599 | .225 | .025 | .253 | .941 | .689 | .986 | .741 | .177 | .363 |
| | N | 4 | 4 | 4 | 4 | 4 | 4 | 4 | 4 | 4 | 4 | 4 | 4 | 4 | 4 | 4 | 4 | 4 | 4 | 4 | 4 |
| Cuto | Pearson Correlation | .975* | .793 | -.022 | .698 | .203 | .275 | .734 | .972* | .680 | .965* | .805 | .884 | .691 | .598 | .666 | .660 | .128 | .912 | -.017 | -.234 |
| | Significance probability (two-sided) | .025 | .207 | .978 | .302 | .797 | .725 | .266 | .028 | .320 | .035 | .195 | .116 | .309 | .402 | .334 | .340 | .872 | .088 | .983 | .766 |
| | N | 4 | 4 | 4 | 4 | 4 | 4 | 4 | 4 | 4 | 4 | 4 | 4 | 4 | 4 | 4 | 4 | 4 | 4 | 4 | 4 |
| Corneo | Pearson Correlation | .942 | .996* | .488 | .605 | .673 | .735 | .923 | .848 | .952* | .859 | .685 | .951* | .970* | .762 | .367 | .519 | .083 | .628 | .511 | .265 |
| | Significance probability (two-sided) | .058 | .004 | .512 | .395 | .327 | .265 | .077 | .152 | .048 | .141 | .315 | .049 | .030 | .238 | .633 | .481 | .917 | .372 | .489 | .735 |
| | N | 4 | 4 | 4 | 4 | 4 | 4 | 4 | 4 | 4 | 4 | 4 | 4 | 4 | 4 | 4 | 4 | 4 | 4 | 4 | 4 |

## Before and after application Correlation analysis between RNA Microarray and measurement results of devices applicable to the human body

| Gene | LCE1A | LCE1B | LCE1F | LCE2A | LOR | AQP3 | AZGP1 | BGN | HAS1 | HAS2 | HAS3 | CER1 | CERS1 | CERS3 | KLF4 | TGM1 | SPINK5 | CLDN1 |
|---|---|---|---|---|---|---|---|---|---|---|---|---|---|---|---|---|---|---|
| Before and after application –PRIMOS | - | - | - | - | - | 0.05* | - | - | - | - | 0.05* | - | - | - | - | - | - | - |
| Before and after application –Cutometer | 0.05* | - | - | - | - | - | 0.05* | 0.05* | - | - | - | - | - | - | - | - | - | - |
| Before and after application –Corneometer | - | 0.01** | - | - | - | - | - | - | - | 0.05* | 0.05* | - | - | - | - | - | - | - |

【Fig 32】

Scatter graph of statistically significant genes after correlation analysis

【Fig 33】

Sig_Hierarchical clustering (Dry vs Moist)

【Fig 34】

| Gene symbol | Test group 1 | Test group 2 | Test group 3 | Test group 4 | Test group 5 | Control group 1 | Control group 2 | Control group 3 | Control group 4 | Control group 5 | Fold change | p-value |
|---|---|---|---|---|---|---|---|---|---|---|---|---|
| CDSN | 3.29232 | 3.05368 | 2.81539 | 2.31832 | 3.36148 | 4.10628 | 3.15219 | 3.53790 | 3.41589 | 3.36965 | -1.46220 | 0.06076 |
| FLG | 7.37995 | 7.96650 | 7.89872 | 7.71499 | 8.07594 | 8.95956 | 8.18707 | 9.39815 | 8.73327 | 9.40186 | -2.18671 | 0.01021 |
| FLG2 | 6.76780 | 7.23536 | 7.08916 | 6.36868 | 7.21131 | 8.08827 | 7.57679 | 8.09646 | 8.20241 | 8.36391 | -2.19027 | 0.00946 |
| LOR | 9.93223 | 10.02970 | 9.65256 | 9.28509 | 9.38772 | 10.81693 | 9.94010 | 11.29831 | 10.82858 | 10.30129 | -1.97189 | 0.03409 |
| KLF4 | 2.70644 | 3.20311 | 3.02223 | 2.92735 | 3.71995 | 4.50525 | 3.27883 | 3.79392 | 3.84160 | 4.32222 | -1.78083 | 0.04108 |
| KRT10 | 7.48853 | 8.06481 | 8.88380 | 6.22591 | 8.76057 | 10.29611 | 8.18422 | 9.57592 | 9.41495 | 9.24761 | -2.74925 | 0.08412 |
| LCE1A | 6.30225 | 6.61091 | 6.86339 | 6.20261 | 7.40451 | 7.96405 | 7.53185 | 7.40801 | 6.82119 | 6.96042 | -1.58049 | 0.12368 |
| LCE2A | 5.52683 | 5.83853 | 6.03735 | 5.54395 | 5.47562 | 6.70292 | 6.49590 | 5.83165 | 5.39860 | 5.54395 | -1.23983 | 0.30652 |
| LCE2B | 7.28533 | 8.37457 | 7.56120 | 7.72608 | 7.51691 | 8.68325 | 8.46799 | 7.82650 | 7.90897 | 7.24965 | -1.26090 | 0.29972 |
| LCE2C | 6.42428 | 6.89902 | 5.51825 | 5.04368 | 5.61370 | 7.22099 | 5.66119 | 7.32573 | 5.87881 | 6.28501 | -1.48922 | 0.31163 |
| SMPD3 | 3.17542 | 3.79378 | 2.81936 | 4.67247 | 3.95476 | 4.41613 | 3.92444 | 3.50156 | 4.10142 | 4.36205 | -1.29950 | 0.27616 |
| CDH1 | 4.05153 | 4.30208 | 4.60819 | 2.72517 | 5.48141 | 7.23115 | 6.64607 | 6.23655 | 6.33431 | 6.02782 | -4.79491 | 0.01454 |
| ITGB4 | 2.00289 | 2.13147 | 1.93433 | 1.89071 | 2.94778 | 4.24747 | 5.13633 | 2.69350 | 5.18746 | 3.97238 | -4.18722 | 0.01555 |
| IVL | 5.04467 | 4.88584 | 4.92031 | 4.24007 | 3.26157 | 6.11250 | 5.94287 | 3.77012 | 7.04138 | 7.04895 | -2.85338 | 0.14847 |
| SPINK5 | 8.61039 | 9.32645 | 8.96345 | 7.92705 | 10.28034 | 10.55474 | 10.25222 | 10.08302 | 10.52116 | 10.32285 | -2.50578 | 0.03900 |
| CLDN1 | 3.42166 | 3.60824 | 3.94682 | 3.32070 | 4.95759 | 5.77688 | 4.87760 | 4.78474 | 5.20828 | 4.04402 | -2.12476 | 0.12580 |
| AQP3 | 3.97963 | 3.29720 | 4.05652 | 3.13080 | 4.15112 | 5.42561 | 4.43396 | 4.26861 | 4.37891 | 3.76206 | -1.65953 | 0.10594 |
| BGN | 2.17103 | 2.62142 | 2.17010 | 2.08619 | 2.90259 | 4.34036 | 3.81091 | 3.08693 | 2.75063 | 2.10725 | -1.77639 | 0.15895 |
| TGM1 | 3.24710 | 4.30617 | 4.02220 | 3.83041 | 4.21451 | 5.07263 | 4.23636 | 5.17356 | 4.47778 | 5.02257 | -1.83084 | 0.04834 |
| CLDN7 | 2.13995 | 1.96317 | 1.96570 | 1.84163 | 2.73956 | 2.83310 | 2.46956 | 2.64755 | 2.47776 | 2.49308 | -1.37003 | 0.06346 |
| CERS3 | 1.34976 | 1.54328 | 1.07821 | 1.70254 | 3.22853 | 2.62802 | 3.72732 | 1.73718 | 1.66802 | 1.80453 | -1.44648 | 0.43212 |
| CLDN4 | 1.77201 | 1.63065 | 1.82637 | 1.62660 | 1.63570 | 1.92989 | 2.56187 | 1.91016 | 1.68938 | 2.55449 | -1.34807 | 0.10027 |
| KRT1 | 3.36826 | 2.85130 | 4.67363 | 2.33929 | 4.62068 | 5.28021 | 3.49379 | 4.61625 | 3.59526 | 3.29492 | -1.40003 | 0.43344 |
| HAS3 | 1.75912 | 1.96133 | 1.78833 | 1.99692 | 1.98842 | 2.24868 | 1.77257 | 2.29918 | 3.12730 | 1.76078 | -1.26828 | 0.24648 |
| CERS1 | 1.39908 | 2.02673 | 1.42313 | 1.46825 | 1.69658 | 1.63072 | 1.39528 | 1.74091 | 1.75617 | 1.64392 | -1.02147 | 0.87173 |

【Fig 35】

【Fig 36】

【Fig 37】

【Fig 38】

## Correlation

| Corneometer | | SMPD3 | CDSN | KLF4 | LOR | LCE2A | LCE2C | FLG | KRT10 | LCE2B | LCE1A | CDH1 | FLG2 |
|---|---|---|---|---|---|---|---|---|---|---|---|---|---|
| Corneometer | Pearson Correlation | 1 | .390 | .536 | .750* | .694* | .243 | .438 | .882** | .612 | .332 | .598 | .786** | .887** |
| | Significance probability (two-sided) | | .265 | .110 | .013 | .026 | .498 | .206 | .001 | .060 | .350 | .068 | .007 | .001 |
| | N | 10 | 10 | 10 | 10 | 10 | 10 | 10 | 10 | 10 | 10 | 10 | 10 | 10 |

| Corneometer | | ITGB4 | IVL | SPINK5 | CLDN1 | AQP3 | BGN | TGM1 | CLDN7 | CERS3 | CLDN4 | KRT1 | HAS3 |
|---|---|---|---|---|---|---|---|---|---|---|---|---|---|
| Corneometer | Pearson Correlation | 1 | .719* | .457 | .801** | .574 | .359 | .479 | .853** | .649* | .376 | .638* | .157 | .307 |
| | Significance probability (two-sided) | | .019 | .184 | .005 | .083 | .309 | .161 | .002 | .042 | .285 | .047 | .664 | .389 |
| | N | 10 | 10 | 10 | 10 | 10 | 10 | 10 | 10 | 10 | 10 | 10 | 10 | 10 |

## Correlation

| Tewameter | | SMPD3 | CDSN | KLF4 | LOR | LCE2A | LCE2C | FLG | KRT10 | LCE2B | LCE1A | CDH1 | FLG2 |
|---|---|---|---|---|---|---|---|---|---|---|---|---|---|---|
| Tewameter | Pearson Correlation | 1 | -.493 | -.563 | -.768** | -.668* | -.390 | -.294 | -.804** | -.634* | -.513 | -.719* | .853** | .830** |
| | Significance probability (two-sided) | | .148 | .090 | .001 | .035 | .265 | .410 | .005 | .049 | .130 | .019 | .002 | .003 |
| | N | 10 | 10 | 10 | 10 | 10 | 10 | 10 | 10 | 10 | 10 | 10 | 10 | 10 |

| Tewameter | | ITGB4 | IVL | SPINK5 | CLDN1 | AQP3 | BGN | TGM1 | CLDN7 | CERS3 | CLDN4 | KRT1 | HAS3 |
|---|---|---|---|---|---|---|---|---|---|---|---|---|---|
| Tewameter | Pearson Correlation | 1 | -.852** | -.459 | -.825** | -.796** | -.577 | -.686* | -.806** | -.728* | -.521 | -.508 | -.294 | -.499 |
| | Significance probability (two-sided) | | .002 | .182 | .003 | .006 | .081 | .029 | .029 | .017 | .123 | .134 | .410 | .142 |
| | N | 10 | 10 | 10 | 10 | 10 | 10 | 10 | 10 | 10 | 10 | 10 | 10 | 10 |

## Correlation

| Skin-pH-meter | | SMPD3 | CDSN | KLF4 | LOR | LCE2A | LCE2C | FLG | KRT10 | LCE2B | LCE1A | CDH1 | FLG2 |
|---|---|---|---|---|---|---|---|---|---|---|---|---|---|---|
| Skin-pH-meter | Pearson Correlation | 1 | -.498 | -.636* | -.673* | -.546 | -.288 | -.263 | -.597 | -.477 | -.217 | -.606 | -.646* | -.539 |
| | Significance probability (two-sided) | | .143 | .048 | .033 | .103 | .420 | .463 | .069 | .163 | .547 | .063 | .044 | .108 |
| | N | 10 | 10 | 10 | 10 | 10 | 10 | 10 | 10 | 10 | 10 | 10 | 10 | 10 |

| Skin-pH-meter | | ITGB4 | IVL | SPINK5 | CLDN1 | AQP3 | BGN | TGM1 | CLDN7 | CERS3 | CLDN4 | KRT1 | HAS3 |
|---|---|---|---|---|---|---|---|---|---|---|---|---|---|
| Skin-pH-meter | Pearson Correlation | 1 | -.594 | -.261 | -.534 | -.715* | -.712* | -.609 | -.525 | -.771** | -.429 | -.289 | -.425 | -.394 |
| | Significance probability (two-sided) | | .070 | .466 | .112 | .020 | .021 | .062 | .119 | .009 | .216 | .418 | .221 | .259 |
| | N | 10 | 10 | 10 | 10 | 10 | 10 | 10 | 10 | 10 | 10 | 10 | 10 | 10 |

[Fig 39]

【Fig 40】

| Gene | Test/Control – Corneometer | Test/Control – Tewameter | Test/Control – Skin-pHmeter |
|------|---------------------------|--------------------------|----------------------------|
| SMPD3 | - | - | - |
| CDSN | - | - | 0.05* |
| KLF4 | 0.05* | 0.01** | 0.05* |
| LOR | 0.05* | 0.05* | - |
| LCE2A | - | - | - |
| LCE2C | - | - | - |
| FLG | 0.01** | 0.01** | - |
| KRT10 | - | 0.05* | - |
| LCE2B | - | - | - |
| LCE1A | - | 0.05* | - |
| CDH1 | 0.01** | 0.01** | 0.05* |
| FLG2 | 0.01** | 0.01** | - |
| ITGB4 | 0.05* | 0.01** | - |
| IVL | - | - | - |
| SPINK5 | 0.01** | 0.01** | - |
| CLDN1 | - | 0.01** | 0.05* |
| AQP3 | - | - | 0.05* |
| BGN | - | 0.05* | - |
| TGM1 | 0.01** | 0.01** | - |
| CLDN7 | 0.05* | 0.05* | 0.01** |
| CERS3 | - | - | - |
| CLDN4 | 0.05* | - | - |
| KRT1 | - | - | - |
| HAS3 | - | - | - |

Test/Control (five subjects by group, total ten subjects) Correlation analysis between RNA Microarray and measurement results of devices applicable to the human body

【Fig 41】

【Fig 42】

【Fig 43】

[Fig 44]

## Correlation

| | | Corneometer | SMPD3 | CDSN | KLF4 | LOR | LCE2A | LCE2C | FLG | KRT10 | LCE2B | LCE1A | CDH1 | FLG2 |
|---|---|---|---|---|---|---|---|---|---|---|---|---|---|---|
| Corneometer | Pearson Correlation | 1 | .549* | .621* | .878** | .429 | .213 | .234 | .591* | .804** | .001 | .500 | .829** | .710** |
| | Significance probability (two-sided) | | .034 | .013 | .000 | .111 | .446 | .402 | .020 | .000 | .996 | .058 | .000 | .003 |
| | N | 15 | 15 | 15 | 15 | 15 | 15 | 15 | 15 | 15 | 15 | 15 | 15 | 15 |

| | | Corneometer | ITGB4 | IVL | SPINK5 | CLDN1 | AQP3 | BGN | TGM1 | CLDN7 | CERS3 | CLDN4 | HAS3 | KRT1 |
|---|---|---|---|---|---|---|---|---|---|---|---|---|---|---|
| Corneometer | Pearson Correlation | 1 | .783** | .679** | .867** | .691** | .609* | .491 | .830** | .595* | .508 | .651** | .189 | .726** |
| | Significance probability (two-sided) | | .001 | .005 | .000 | .004 | .016 | .063 | .000 | .019 | .053 | .009 | .501 | .002 |
| | N | 15 | 15 | 15 | 15 | 15 | 15 | 15 | 15 | 15 | 15 | 15 | 15 | 15 |

## Correlation

| | | Tewameter | SMPD3 | CDSN | KLF4 | LOR | LCE2A | LCE2C | FLG | KRT10 | LCE2B | LCE1A | CDH1 | FLG2 |
|---|---|---|---|---|---|---|---|---|---|---|---|---|---|---|
| Tewameter | Pearson Correlation | 1 | -.598* | -.596* | -.800** | -.575* | -.469 | -.351 | -.722** | -.709** | -.321 | -.586* | -.844** | -.751** |
| | Significance probability (two-sided) | | .018 | .019 | .000 | .025 | .078 | .199 | .002 | .003 | .243 | .022 | .000 | .001 |
| | N | 15 | 15 | 15 | 15 | 15 | 15 | 15 | 15 | 15 | 15 | 15 | 15 | 15 |

| | | Tewameter | ITGB4 | IVL | SPINK5 | CLDN1 | AQP3 | BGN | TGM1 | CLDN7 | CERS3 | CLDN4 | HAS3 | KRT1 |
|---|---|---|---|---|---|---|---|---|---|---|---|---|---|---|
| Tewameter | Pearson Correlation | 1 | -.818** | -.527* | -.752** | -.776** | -.650** | -.763** | -.735** | -.708** | -.564* | -.578* | -.441 | -.472 |
| | Significance probability (two-sided) | | .000 | .044 | .001 | .001 | .009 | .001 | .002 | .003 | .029 | .024 | .100 | .076 |
| | N | 15 | 15 | 15 | 15 | 15 | 15 | 15 | 15 | 15 | 15 | 15 | 15 | 15 |

## Correlation

| | | pHmeter | SMPD3 | CDSN | KLF4 | LOR | LCE2A | LCE2C | FLG | KRT10 | LCE2B | LCE1A | CDH1 | FLG2 |
|---|---|---|---|---|---|---|---|---|---|---|---|---|---|---|
| pHmeter | Pearson Correlation | 1 | -.573* | -.805** | -.793** | -.812** | -.498 | -.496 | -.661* | -.776** | -.703** | -.554* | -.767** | -.749** |
| | Significance probability (two-sided) | | .041 | .001 | .001 | .001 | .083 | .085 | .014 | .002 | .007 | .049 | .002 | .003 |
| | N | 13 | 13 | 13 | 13 | 13 | 13 | 13 | 13 | 13 | 13 | 13 | 13 | 13 |

| | | pHmeter | ITGB4 | IVL | SPINK5 | CLDN1 | AQP3 | BGN | TGM1 | CLDN7 | CERS3 | CLDN4 | HAS3 | KRT1 |
|---|---|---|---|---|---|---|---|---|---|---|---|---|---|---|
| pHmeter | Pearson Correlation | 1 | -.471 | -.313 | -.711** | -.698** | -.797** | -.409 | -.594* | -.360 | -.317 | -.405 | -.787** | -.129 |
| | Significance probability (two-sided) | | .105 | .297 | .006 | .008 | .001 | .166 | .032 | .228 | .291 | .170 | .001 | .674 |
| | N | 13 | 13 | 13 | 13 | 13 | 13 | 13 | 13 | 13 | 13 | 13 | 13 | 13 |

【Fig 45】

| Correlation analysis between RNA Microarray and measurement results of devices applicable to the human body | | | |
|---|---|---|---|
| Gene | Corneometer (15 subjects) | Tewameter (15 subjects) | Skin-pHmeter(13 subjects) |
| SMPD3 | 0.05* | 0.05* | 0.05* |
| CDSN | 0.05* | 0.05* | 0.01** |
| KLF4 | 0.01** | 0.01** | 0.01** |
| LOR | - | 0.05* | 0.01** |
| LCE2A | - | - | - |
| LCE2C | - | - | - |
| FLG | 0.05* | 0.01** | 0.05* |
| KRT10 | 0.01** | 0.01** | 0.01** |
| LCE2B | - | - | 0.01** |
| LCE1A | - | 0.05* | 0.05* |
| CDH1 | 0.01** | 0.01** | 0.01** |
| FLG2 | 0.01** | 0.01** | 0.01** |
| ITGB4 | 0.01** | 0.01** | - |
| IVL | 0.01** | 0.05* | - |
| SPINK5 | 0.01** | 0.01** | 0.01** |
| CLDN1 | 0.01** | 0.01** | 0.01** |
| AQP3 | 0.05* | 0.01** | 0.01** |
| BGN | - | 0.01** | - |
| TGM1 | 0.01** | 0.01** | 0.05* |
| CLDN7 | 0.05* | 0.01** | - |
| CERS3 | - | 0.05* | - |
| CLDN4 | 0.01** | 0.05* | - |
| KRT1 | 0.01** | - | 0.01** |
| HAS3 | - | - | - |

【Fig 46】

Efficacy 0w/2w results

【Fig 47】

| Gene symbol | Week 0 | Week 2 | Fold change |
|:---:|:---:|:---:|:---:|
| CDSN | 3.07224 | 4.65453 | 2.99445 |
| FLG | 7.11296 | 9.07048 | 3.88394 |
| FLG2 | 6.14761 | 7.09102 | 1.92307 |
| LOR | 9.73963 | 11.56980 | 3.55578 |
| KLF4 | 2.99610 | 3.16088 | 1.12099 |
| KRT10 | 5.78145 | 6.65388 | 1.83075 |
| LCE1A | 6.40705 | 8.28028 | 3.66352 |
| LCE2A | 3.13323 | 4.90128 | 3.40594 |
| LCE2B | 8.15969 | 10.81078 | 6.28146 |
| LCE2C | 6.46216 | 9.25694 | 6.93928 |
| SMPD3 | 4.54770 | 5.02508 | 1.39221 |
| CLDN1 | 2.14780 | 2.19416 | 1.03266 |
| BGN | 2.42198 | 2.53831 | 1.08397 |
| CLDN4 | 1.75122 | 1.77669 | 1.01781 |

【Fig 48】

【Fig 49】

【Fig 50】

| Gene symbol | Test group 1 | Test group 2 | Test group 3 | Test group 4 | Test group 5 | Control group 1 | Control group 2 | Control group 3 | Control group 4 | Control group 5 | Fold change | p-value |
|---|---|---|---|---|---|---|---|---|---|---|---|---|
| CAT | 2.08054 | 1.87316 | 1.6241 | 1.52817 | 1.65907 | 3.48175 | 3.50925 | 2.13533 | 3.2703 | 1.76805 | -2.11393 | 0.01470 |
| CLU | 1.14374 | 1.23627 | 1.22743 | 1.00412 | 1.01775 | 2.58105 | 1.69152 | 2.36469 | 2.87759 | 2.36399 | -2.37826 | 0.00287 |
| DSG1 | 2.64345 | 2.42104 | 3.17793 | 2.469 | 3.74325 | 6.95147 | 5.85107 | 6.89763 | 8.0714 | 5.87054 | -14.29549 | 0.00125 |
| GPNMB | 1.85204 | 1.71446 | 1.63204 | 1.63815 | 1.70151 | 3.9054 | 4.29587 | 2.34219 | 3.68399 | 2.17375 | -2.97440 | 0.00956 |
| GPX4 | 2.32566 | 2.58506 | 2.34718 | 2.55626 | 3.25788 | 3.84967 | 3.26032 | 3.15053 | 4.24694 | 3.56507 | -2.00014 | 0.00944 |
| GSTM2 | 1.35823 | 1.77126 | 1.85332 | 1.58986 | 1.94855 | 2.55668 | 2.40221 | 2.43999 | 1.89549 | 2.56366 | -1.58817 | 0.00507 |
| GSTP1 | 4.14331 | 4.49577 | 4.44964 | 3.35896 | 3.75031 | 6.40164 | 5.14882 | 5.48633 | 6.49252 | 5.93764 | -3.61449 | 0.00722 |
| MC2R | 1.83639 | 1.51351 | 1.742 | 1.41866 | 1.12555 | 1.1927 | 1.38081 | 1.27701 | 1.26954 | 1.1428 | 1.20970 | 0.04300 |
| MC4R | 1.05983 | 1.91237 | 1.46286 | 1.8106 | 1.36472 | 1.15585 | 1.04983 | 1.22308 | 1.31686 | 1.58457 | 1.19419 | 0.13162 |
| MFSD12 | 2.18281 | 2.16865 | 1.3647 | 1.74454 | 1.12331 | 1.46813 | 1.33712 | 1.43146 | 2.75308 | 1.32835 | 1.03755 | 0.44088 |
| MLANA | 1.05637 | 1.22021 | 0.96146 | 1.09556 | 1.13627 | 0.90556 | 0.84351 | 1.00721 | 0.84811 | 0.83826 | 1.15304 | 0.02378 |
| PAX3 | 1.09299 | 1.15621 | 1.38859 | 1.48812 | 1.32282 | 0.96305 | 1.03736 | 1.13613 | 1.1407 | 1.18222 | 1.14699 | 0.00561 |
| PDE4D | 0.96121 | 1.21096 | 1.08186 | 1.11177 | 1.08917 | 1.14083 | 1.14953 | 1.09486 | 0.8947 | 1.15563 | 1.00270 | 0.47802 |
| POMC | 1.06798 | 1.09048 | 1.01128 | 1.3119 | 1.10418 | 1.28596 | 1.00748 | 1.33373 | 1.26384 | 1.29825 | -1.08725 | 0.10116 |
| SOX10 | 1.53609 | 1.46345 | 1.43427 | 1.51209 | 1.48071 | 0.97582 | 1.21627 | 1.24026 | 1.20878 | 1.21856 | 1.24262 | 0.00407 |
| TFAP2A | 1.40562 | 1.18298 | 1.33891 | 1.49567 | 1.17293 | 0.95584 | 1.18298 | 1.131 | 1.18298 | 1.18298 | 1.14240 | 0.04875 |
| TYR | 1.12481 | 1.21259 | 1.00885 | 1.27535 | 1.13131 | 0.88077 | 0.88206 | 0.84263 | 0.93053 | 1.13283 | 1.16217 | 0.01336 |
| TYRP1 | 1.30938 | 1.55196 | 1.6536 | 1.46682 | 1.02661 | 1.2449 | 0.94895 | 0.96329 | 1.17702 | 1.32433 | 1.20579 | 0.10465 |

【Fig 51】

【Fig 52】

[Fig 53]

# Correlation

| | | Spectro | CAT | CLDN1 | CLU | DSG1 | GPNMB | GPX4 | GSTM2 | GSTP1 | MC2R | MC4R | MFSD12 | MLANA | PAX3 | PDE4D | POMC | SOX10 | TFAP2A | TYR | TYRP1 |
|---|---|---|---|---|---|---|---|---|---|---|---|---|---|---|---|---|---|---|---|---|---|
| Spectro | Pearson Correlation | 1 | .666* | .825** | .886** | .887** | .761* | .797** | .840** | .902** | -.596 | -.262 | -.047 | -.805** | -.575 | .104 | .413 | -.896** | -.674* | -.656* | -.413 |
| | Significance probability (two-sided) | | .035 | .003 | .001 | .001 | .011 | .006 | .002 | .000 | .069 | .465 | .896 | .005 | .082 | .774 | .236 | .000 | .033 | .039 | .235 |
| | N | 10 | 10 | 10 | 10 | 10 | 10 | 10 | 10 | 10 | 10 | 10 | 10 | 10 | 10 | 10 | 10 | 10 | 10 | 10 | 10 |

| | | Mexa | CAT | CLDN1 | CLU | DSG1 | GPNMB | GPX4 | GSTM2 | GSTP1 | MC2R | MC4R | MFSD12 | MLANA | PAX3 | PDE4D | POMC | SOX10 | TFAP2A | TYR | TYRP1 |
|---|---|---|---|---|---|---|---|---|---|---|---|---|---|---|---|---|---|---|---|---|---|
| Mexa | Pearson Correlation | 1 | -.639* | -.753* | -.790** | -.782** | -.700* | -.720* | -.666* | -.773** | .513 | .493 | -.010 | .744* | .700* | .088 | -.487 | .779** | .513 | .505 | .556 |
| | Significance probability (two-sided) | | .047 | .012 | .006 | .008 | .024 | .019 | .035 | .009 | .129 | .147 | .977 | .014 | .024 | .809 | .154 | .008 | .130 | .137 | .095 |
| | N | 10 | 10 | 10 | 10 | 10 | 10 | 10 | 10 | 10 | 10 | 10 | 10 | 10 | 10 | 10 | 10 | 10 | 10 | 10 | 10 |

ITA° under 28/ ITA° above 41 (five subjects by group, total ten subjects) Correlation analysis between RNA Microarray and measurement results of devices applicable to the human body

| Gene | CAT | CLU | DSG1 | GPNMB | GPX4 | GSTM2 | GSTP1 | MLANA | PAX3 | SOX10 | TFAP2A | TYR | TYRP1 |
|---|---|---|---|---|---|---|---|---|---|---|---|---|---|
| ITA° under 28/ ITA° above 41-Spectrophotometer | 0.05* | 0.01** | 0.01** | 0.05* | 0.01** | 0.01** | 0.01** | 0.01** | - | 0.01** | 0.05* | 0.05* | - |
| ITA° under 28/ ITA° above 41-Mexameter | 0.05* | 0.01** | 0.01** | 0.05* | 0.05* | 0.05* | 0.01** | 0.05* | 0.05* | 0.01** | - | - | - |

【Fig 54】

ITA° under 28/ ITA° above 41-Spectrophotometer
Scatter graph of statistically significant genes after correlation analysis

【Fig 55】

ITA° under 28/ ITA° above 41 -Mexameter
Scatter graph of statistically significant genes after correlation analysis

[Fig 56]

EP 4 194 565 A1

## Correlation

|  |  | CAT | CLDN1 | CLU | DSG1 | GPNMB | GPX4 | GSTM2 | GSTP1 | MC2R | MC4R | MFSD12 | MLANA | MLANA2 | PAX3 | PDE4D | POMC | SOX10 | TFAP2A | TYR | TYRP1 |
|---|---|---|---|---|---|---|---|---|---|---|---|---|---|---|---|---|---|---|---|---|---|
| ITA | Pearson Correlation | .642** | .759** | .741** | .839** | .688** | .657** | .460* | .848** | -.460* | -.195 | -.009 | -.092 | -.553* | -.485* | -.041 | .134 | -.568* | -.435 | -.469* | -.391 |
|  | Significance probability (two-sided) | .003 | .000 | .000 | .000 | .001 | .002 | .047 | .000 | .047 | .423 | .972 | .709 | .014 | .035 | .866 | .854 | .011 | .063 | .043 | .098 |
|  | N | 19 | 19 | 19 | 19 | 19 | 19 | 19 | 19 | 19 | 19 | 19 | 19 | 19 | 19 | 19 | 19 | 19 | 19 | 19 | 19 |

|  |  | CAT | CLDN1 | CLU | DSG1 | GPNMB | GPX4 | GSTM2 | GSTP1 | MC2R | MC4R | MFSD12 | MLANA | MLANA2 | PAX3 | PDE4D | POMC | SOX10 | TFAP2A | TYR | TYRP1 |
|---|---|---|---|---|---|---|---|---|---|---|---|---|---|---|---|---|---|---|---|---|---|
| ITA | Pearson Correlation | -.493* | -.632 | -.664** | -.795** | -.502* | -.577** | -.492* | -.727** | .359 | .355 | -.115 | .151 | .625** | .467* | .222 | -.242 | .462* | .335 | .392 | .494* |
|  | Significance probability (two-sided) | .032 | .004 | .002 | .000 | .029 | .029 | .032 | .000 | .131 | .136 | .639 | .536 | .004 | .044 | .360 | .317 | .047 | .161 | .97 | .032 |
|  | N | 19 | 19 | 19 | 19 | 19 | 19 | 19 | 19 | 19 | 19 | 19 | 19 | 19 | 19 | 19 | 19 | 19 | 19 | 19 | 19 |

ITA° under 28(five subjects)/ ITA° between 20 and 40 (seven subjects)/ ITA° above 41 (seven subjects) (Total 19 subjects)
Correlation analysis between RNA Microarray and measurement results of devices applicable to the human body

| Gene | CAT | CLU | DSG1 | GPNMB | GPX4 | GSTM2 | GSTP1 | MLANA | PAX3 | SOX10 | TFAP2A | TYR | TYRP1 |
|---|---|---|---|---|---|---|---|---|---|---|---|---|---|
| ITA° under 28/ ITA° between 20 and 40/ ITA° above 41 - Spectrophotometer | 0.01** | 0.01** | 0.01** | 0.01** | 0.01** | 0.05** | 0.01** | 0.05** | 0.05** | 0.05** | - | 0.05** | - |
| ITA° under 28/ ITA° between 20 and 40/ ITA° above 41 - Mexameter | 0.05** | 0.01** | 0.01** | 0.05** | 0.05** | 0.05** | 0.01** | 0.01** | 0.05** | 0.05** | - | - | 0.05* |

【Fig 57】

ITA° under 28/ ITA° between 20 and 40/ ITA° above 41 ~Spectrophotometer
Scatter graph of statistically significant genes after correlation analysis

【Fig 58】

ITA° under 28/ ITA° between 20 and 40/ ITA° above 41-mexameter
Scatter graph of statistically significant genes after correlation analysis

【Fig 59】

| Color around the eyes (ITA°) | 0W | 2W |
|---|---|---|
| R020 | 40.34 | 40.24 |
| R047 | 28.77 | 30.83 |

【Fig 60】

| Melanin around the eyes (Melanin index) | 0W | 2W |
|---|---|---|
| R020 | 152.8 | 152.7 |
| R047 | 176.9 | 163.5 |

【Fig 61】

| Gene symbol | 0W - 1 | 0W - 2 | 2W - 1 | 2W - 2 | Fold change | p-value |
|---|---|---|---|---|---|---|
| MC1R | 2.529963 | 2.549245 | 1.966737 | 2.017397 | -1.46159 | 0.018237 |
| MC4R | 1.78587 | 1.825073 | 1.731074 | 1.733453 | -1.05205 | 0.156858 |
| MLANA | 1.761138 | 1.698777 | 1.705887 | 1.672663 | -1.0286 | 0.218917 |
| SOX10 | 1.75478 | 1.915435 | 1.622678 | 1.707392 | -1.12512 | 0.139839 |
| TFAP2A | 1.948227 | 2.262936 | 1.984129 | 2.125251 | -1.0359 | 0.662386 |
| TYR | 1.293841 | 1.504916 | 1.643299 | 1.398392 | 1.08784 | 0.688362 |
| TYRP1 | 1.537883 | 1.873577 | 1.502049 | 1.827621 | -1.02875 | 0.078387 |
| DCT | 1.790526 | 1.968813 | 1.918312 | 1.43192 | -1.15233 | 0.648754 |
| F2RL1 | 1.667817 | 1.807433 | 2.127531 | 2.236489 | 1.360734 | 0.021951 |
| CAT | 2.239805 | 2.170289 | 2.043444 | 2.227798 | -1.0493 | 0.681377 |
| CLDN1 | 1.880074 | 2.086061 | 2.316745 | 2.806727 | 1.49347 | 0.153191 |
| DSG1 | 2.538787 | 2.161201 | 2.405755 | 3.793139 | 1.681155 | 0.551781 |
| GPNMB | 1.866311 | 2.112271 | 1.79978 | 2.284558 | 1.037332 | 0.734608 |
| GSTP1 | 3.361785 | 3.133771 | 3.927215 | 4.52272 | 1.968627 | 0.25388 |

【Fig 62】

[Fig 63]

## Correlation

| | | CM | CLDN1 | CLU | DCT | DSG1 | F2RL1 | GPNMB | GPX4 | GSTM2 | GSTP1 | MC1R | MC4R | MFSD12 | MLANA | PMEL | POMC | SOX10 | TFAP2A | TYR | TYRP1 |
|---|---|---|---|---|---|---|---|---|---|---|---|---|---|---|---|---|---|---|---|---|---|
| ITA | Pearson Correlation | -.323 | -.401 | .054 | .238 | -.270 | -.181 | -.889 | .029 | -.382 | -.046 | -.152 | -.374 | -.820 | .699 | -.365 | .914 | -.662 | -.961* | .018 | -.996** |
| | Significance probability (two-sided) | .677 | .599 | .946 | .762 | .730 | .819 | .111 | .971 | .618 | .954 | .848 | .626 | .180 | .301 | .635 | .086 | .338 | .039 | .982 | .004 |
| | N | 4 | 4 | 4 | 4 | 4 | 4 | 4 | 4 | 4 | 4 | 4 | 4 | 4 | 4 | 4 | 4 | 4 | 4 | 4 | 4 |
| Melanin | Pearson Correlation | .203 | .089 | .381 | .108 | -.084 | -.080. | -678 | .290 | .413 | .287 | .386 | .630 | .968* | -.513 | .141 | -.725 | .837 | .099** | .078 | .919 |
| | Significance probability (two-sided) | .797 | .911 | .619 | .892 | .916 | .920 | .322 | .710 | .587 | .713 | .614 | .370 | .032 | .487 | .859 | .275 | .163 | .010 | .922 | .081 |
| | N | 4 | 4 | 4 | 4 | 4 | 4 | 4 | 4 | 4 | 4 | 4 | 4 | 4 | 4 | 4 | 4 | 4 | 4 | 4 | 4 |

## Before and after application Correlation analysis between RNA Microarray and measurement results of devices applicable to the human body

| Gene | CAT | CLU | DSG1 | GPNMB | GPX4 | GSTM2 | GSTP1 | MC1R | MC4R | MLANA | PMEL | POMC | SOX10 | TFAP2A | TYR | TYRP1 |
|---|---|---|---|---|---|---|---|---|---|---|---|---|---|---|---|---|
| Before and after application -Spectrophotometer | - | - | - | - | - | - | - | - | - | - | - | - | - | 0.05* | - | 0.01** |
| Before and after application -Mexameter | - | - | - | - | - | - | - | - | - | - | - | - | - | 0.01** | - | - |

EP 4 194 565 A1

【Fig 64】

Scatter graph of statistically significant genes after correlation analysis

【Fig 65】

Sig_Hierarchical clustering (Oily vs Non-oily)

【Fig 66】

| Gene symbol | Test group 1 | Test group 2 | Test group 3 | Test group 4 | Test group 5 | Control group 1 | Control group 2 | Control group 3 | Control group 4 | Control group 5 | Fold change | p-value |
|---|---|---|---|---|---|---|---|---|---|---|---|---|
| MFAP2 | 1.60627 | 1.54897 | 1.53311 | 1.75649 | 1.32472 | 1.03915 | 1.46202 | 1.14320 | 1.21219 | 1.29359 | 1.25169 | 0.04527 |
| AQP3 | 2.57341 | 2.65544 | 3.09282 | 4.18572 | 3.34294 | 5.83088 | 4.58861 | 4.37891 | 4.43396 | 4.26861 | -2.88811 | 0.04007 |
| SREBF2 | 2.06845 | 1.41287 | 1.49201 | 2.11640 | 1.88872 | 3.04901 | 4.02928 | 3.18916 | 2.70469 | 2.04528 | -2.30984 | 0.04961 |
| HSD17B2 | 1.89911 | 1.89339 | 1.48301 | 3.31552 | 1.58110 | 3.85719 | 5.48641 | 2.28011 | 1.63902 | 3.93458 | -2.64824 | 0.18973 |
| GPAM | 1.35691 | 1.56348 | 1.46562 | 1.11181 | 1.29317 | 1.07256 | 1.38844 | 0.91692 | 1.03937 | 1.02756 | 1.20516 | 0.02746 |
| CPT1C | 2.17789 | 2.47012 | 2.14288 | 2.77783 | 2.79297 | 2.24204 | 2.07265 | 1.84013 | 2.10117 | 2.11638 | 1.31756 | 0.04437 |
| IGF1 | 1.52012 | 1.44338 | 1.18181 | 1.03414 | 1.55368 | 1.08947 | 0.95497 | 1.17513 | 1.21670 | 0.93145 | 1.20839 | 0.15000 |
| HSD11B1 | 1.33336 | 1.40723 | 1.40849 | 1.60757 | 1.59915 | 1.07158 | 1.28473 | 1.22573 | 1.25476 | 1.28037 | 1.18733 | 0.00433 |
| MPZL3 | 3.53936 | 3.54820 | 3.30772 | 3.99365 | 3.57368 | 6.48692 | 5.58138 | 5.87906 | 6.48784 | 4.60184 | -4.64245 | 0.00258 |
| FADS2 | 1.99098 | 2.02249 | 1.98881 | 1.63928 | 2.04764 | 1.69172 | 1.50925 | 1.65132 | 1.53852 | 2.18359 | 1.16713 | 0.11526 |
| IGF1R | 1.26812 | 1.17770 | 1.38632 | 1.28971 | 1.16594 | 1.14682 | 1.28705 | 1.51454 | 1.12883 | 1.09330 | 1.01639 | 0.71495 |
| SRD5A2 | 0.88503 | 1.21674 | 1.12002 | 0.99081 | 1.46731 | 1.00611 | 0.89846 | 1.10144 | 1.15806 | 1.05434 | 1.06607 | 0.47313 |
| IL1A | 1.27616 | 1.13613 | 1.67720 | 1.02472 | 1.07808 | 1.08030 | 1.04064 | 1.00363 | 1.07132 | 0.88437 | 1.16668 | 0.14028 |
| IL1B | 0.80775 | 1.16112 | 1.16349 | 0.94353 | 0.95845 | 1.03963 | 0.90501 | 0.97543 | 0.97174 | 0.94337 | 1.02799 | 0.66722 |
| AR | 1.13232 | 1.40136 | 1.27542 | 1.21285 | 1.10362 | 0.86426 | 1.10484 | 1.14992 | 1.12012 | 1.05810 | 1.12168 | 0.02863 |
| FAS | 1.71767 | 1.54388 | 1.65418 | 2.07047 | 1.70252 | 1.53862 | 2.04214 | 1.17232 | 1.59644 | 1.53879 | 1.11735 | 0.42006 |
| NR1H3 | 1.23861 | 1.31304 | 1.34183 | 1.35131 | 1.18753 | 1.26022 | 1.18153 | 1.38563 | 1.34183 | 1.23374 | 1.00408 | 0.86723 |
| ACACB | 1.84334 | 1.28573 | 1.24362 | 1.02317 | 1.10714 | 0.97459 | 1.14375 | 1.24362 | 1.53208 | 1.12109 | 1.06997 | 0.68310 |
| AWAT1 | 1.08724 | 1.39758 | 1.14944 | 1.04983 | 1.04983 | 1.04983 | 0.76810 | 1.03745 | 0.93194 | 0.82016 | 1.16901 | 0.09984 |
| AWAT2 | 0.78611 | 1.17292 | 0.88592 | 1.03818 | 1.04466 | 0.83708 | 1.08833 | 0.98934 | 1.03266 | 1.00657 | 0.99638 | 0.88161 |

【Fig 67】

【Fig 68】

# Correlation

|  | | Sebumeter | MFAP2 | IGF1 | HSD11B1 | GPAM | CPT1C | AR | MPZL2 | AQP3 | SREBF2 | HSD17B2 |
|---|---|---|---|---|---|---|---|---|---|---|---|---|
| Sebumeter | Pearson Correlation | 1 | .704* | .644* | .456 | .658* | .382 | .483 | -.742* | -.741 | -.561 | -.464 |
|  | Significance probability (two-sided) |  | .023 | .045 | .185 | .039 | .276 | .158 | .014 | .014 | .092 | .177 |
|  | N | 10 | 10 | 10 | 10 | 10 | 10 | 10 | 10 | 10 | 10 | 10 |

Sebum/Control (five subjects by group, total ten subjects) Correlation analysis between RNA Microarray and measurement results of devices applicable to the human body

| Gene | Sebum/Control-Sebumeter |
|---|---|
| MFAP2 | 0.05* |
| IGF1 | 0.05* |
| HSD11B1 | - |
| GPAM | 0.05* |
| CPT1C | - |
| AR | - |
| MPZL3 | 0.05* |
| AQP3 | 0.05* |
| SREBF2 | - |
| HSD17B2 | - |

【Fig 69】

【Fig 70】

【Fig 71】

| Gene symbol | Test group 1 | Test group 2 | Test group 3 | Test group 4 | Test group 5 | Control group 1 | Control group 2 | Control group 3 | Control group 4 | Control group 5 | Fold change | p-value |
|---|---|---|---|---|---|---|---|---|---|---|---|---|
| CXCR5 | 1.65367 | 1.66377 | 1.58396 | 1.48838 | 1.43003 | 1.36964 | 1.59932 | 2.30299 | 2.37773 | 1.73330 | -1.24197 | 0.23385 |
| KLK5 | 6.44316 | 5.64173 | 5.79381 | 5.84014 | 7.53182 | 3.99852 | 5.07511 | 5.24154 | 4.38700 | 6.53885 | 2.30047 | 0.02689 |
| CAMP | 2.55921 | 1.82154 | 1.73833 | 1.93032 | 2.17557 | 1.85471 | 1.67325 | 1.92695 | 1.59892 | 1.67471 | 1.23054 | 0.12167 |
| MMP9 | 1.86496 | 2.10407 | 1.83864 | 2.09222 | 1.91745 | 1.75442 | 1.58697 | 1.56199 | 1.93836 | 1.58993 | 1.21179 | 0.01814 |
| CXCL2 | 1.14012 | 1.62232 | 2.44079 | 1.55420 | 1.30358 | 1.46800 | 1.56441 | 2.20504 | 2.72119 | 1.77560 | -1.26107 | 0.24219 |
| PSMB9 | 0.86925 | 1.34145 | 1.40092 | 1.15089 | 1.41282 | 2.00142 | 1.44798 | 1.51555 | 1.59136 | 1.17387 | -1.24054 | 0.25071 |
| IL13RA1 | 1.59695 | 1.95098 | 1.94904 | 1.36672 | 5.09642 | 1.37574 | 1.10679 | 1.23204 | 1.23551 | 1.67305 | 2.09565 | 0.15235 |
| HSD3B1 | 1.60592 | 1.64757 | 1.31221 | 2.14172 | 2.24408 | 1.19803 | 1.34489 | 1.23156 | 2.14567 | 1.89170 | 1.17115 | 0.04692 |
| ANGPT2 | 1.41126 | 1.46541 | 1.30053 | 2.05573 | 1.46129 | 1.24324 | 0.92917 | 1.19655 | 1.37012 | 1.26631 | 1.26380 | 0.04245 |
| TRPA1 | 1.18013 | 1.21320 | 1.38155 | 1.12651 | 1.25678 | 0.89336 | 1.14740 | 1.17970 | 1.00050 | 1.03033 | 1.13396 | 0.00942 |
| CXCR4 | 2.46339 | 2.08228 | 1.46883 | 1.38436 | 2.64468 | 2.05365 | 1.23450 | 1.38012 | 1.48293 | 1.80273 | 1.33600 | 0.09549 |
| TAC1 | 1.01990 | 1.20647 | 1.19365 | 1.25984 | 1.12908 | 1.08335 | 0.97180 | 0.82222 | 0.86270 | 0.90117 | 1.17572 | 0.04626 |
| COL3A1 | 2.34923 | 2.43493 | 2.62154 | 1.79504 | 1.86270 | 1.40330 | 1.34525 | 2.25568 | 2.01368 | 1.79941 | 1.36531 | 0.14785 |
| CXCR1 | 1.27129 | 1.34913 | 1.40343 | 1.51036 | 1.04844 | 0.89044 | 1.15531 | 1.61065 | 0.92169 | 1.17428 | 1.12199 | 0.33039 |
| CXCL5 | 2.04161 | 2.19554 | 2.18241 | 1.73729 | 1.70039 | 1.56094 | 1.90333 | 2.16716 | 1.93547 | 1.67435 | 1.08915 | 0.35713 |
| HLA-DRA | 3.04682 | 1.38267 | 1.52447 | 1.09859 | 2.04083 | 2.85264 | 1.1927 | 1.17935 | 1.40553 | 1.28975 | 1.17665 | 0.23884 |
| IL17A | 1.92044 | 1.95397 | 1.65919 | 1.78632 | 1.81401 | 1.29547 | 1.81198 | 1.94107 | 1.26028 | 1.81914 | 1.14965 | 0.29721 |
| CCL7 | 1.47094 | 0.74343 | 1.14187 | 1.45036 | 0.97559 | 0.85833 | 1.08431 | 1.06378 | 0.96487 | 0.72759 | 1.16204 | 0.26510 |
| CXCL6 | 1.0507 | 1.04856 | 1.48745 | 1.3224 | 1.32925 | 1.1573 | 1.25033 | 1.02495 | 0.93096 | 1.32797 | 1.07876 | 0.46037 |

【Fig 72】

【Fig 73】

## Correlation

| | Mexameter | COL3A1 | TAC1 | KLK5 | CAMP | MMP9 | TRPA1 | IL13RA1 | HSD3B1 | CXCR4 | ANGPT2 | CXCL2 | CXCR5 | PSMB9 |
|---|---|---|---|---|---|---|---|---|---|---|---|---|---|---|
| Mecameter Pearson Correlation | 1 | .589 | .689* | .416 | .644* | .651* | .598 | .240 | .032 | .472 | .433 | -.423 | -.414 | -.545 |
| Significance probability (two-sided) | | .073 | .028 | .232 | .045 | .041 | .068 | .504 | .931 | .168 | .211 | .224 | .234 | .103 |
| N | 10 | 10 | 10 | 10 | 10 | 10 | 10 | 10 | 10 | 10 | 10 | 10 | 10 | 10 |

## Correlation

| | Spectrophotometer | COL3A1 | TAC1 | KLK5 | CAMP | MMP9 | TRPA1 | IL13RA1 | HSD3B1 | CXCR4 | ANGPT2 | CXCL2 | CXCR5 | PSMB9 |
|---|---|---|---|---|---|---|---|---|---|---|---|---|---|---|
| Spectrophotometer Pearson Correlation | 1 | .578 | .717* | 425 | .595 | .829** | .541 | .304 | .306 | .408 | .702* | -.304 | -.296 | -.527 |
| Significance probability (two-sided) | | .080 | .020 | .221 | .070 | .003 | .106 | .393 | .390 | .243 | .024 | .393 | .406 | .117 |
| N | 10 | 10 | 10 | 10 | 10 | 10 | 10 | 10 | 10 | 10 | 10 | 10 | 10 | 10 |

### Rosacea/Control (five subjects by group, total ten subjects) Correlation analysis between RNA Microarray and measurement results of devices applicable to the human body

| Gene | Rosacea/Control -Mexameter | Rosacea/Control -Spectrophotometer |
|---|---|---|
| COL3A1 | - | - |
| TAC1 | 0.05* | 0.05* |
| KLK5 | - | - |
| CAMP | 0.05* | - |
| MMP9 | 0.05* | 0.01** |
| TRPA1 | - | - |
| IL13RA1 | - | - |
| HSD3B1 | - | - |
| CXCR4 | - | - |
| ANGPT2 | - | 0.05* |
| CXCL2 | - | - |
| CXCR5 | - | - |
| PSMB9 | - | - |

【Fig 74】

【Fig 75】

R² linear(L) = 0.514

y=2.82+8.22*x

TAC1

R² linear(L) = 0.688

y=1.95+7.33*x

MMP9

R² linear(L) = 0.493

y=5.52+4.3*x

ANGPT2

【Fig 76】

● 5% lactic acid
● Distilled water

designed by ● freepik.com

【Fig 77】

【Fig 78】

| Gene symbol | Test group 1 | Test group 2 | Test group 3 | Test group 4 | Test group 5 | Control group 1 | Control group 2 | Control group 3 | Control group 4 | Control group 5 | Fold change | p-value |
|---|---|---|---|---|---|---|---|---|---|---|---|---|
| ARTN | 1.4434 | 1.52042 | 1.1794 | 1.59326 | 1.57352 | 1.56158 | 1.22477 | 1.3063 | 1.43581 | 1.40347 | 1.053809 | 0.421145 |
| IVL | 4.35063 | 5.94287 | 4.52896 | 3.40624 | 3.26157 | 4.62286 | 4.88584 | 5.81081 | 4.92031 | 8.01456 | -2.55411 | 0.232983 |
| LOR | 7.26962 | 9.9401 | 7.78847 | 8.24689 | 9.38772 | 8.38845 | 10.0297 | 11.20085 | 9.65256 | 10.36102 | -2.63894 | 0.063304 |
| FLG | 2.85 | 8.18707 | 5.86267 | 6.12453 | 8.07594 | 7.07357 | 7.9665 | 9.23215 | 7.89872 | 9.40011 | -4.2698 | 0.055242 |
| FLG2 | 4.15947 | 7.57679 | 6.02149 | 6.11747 | 7.21131 | 6.47614 | 7.23536 | 8.64231 | 7.08916 | 8.02067 | -2.4207 | 0.077263 |
| PGF | 1.76356 | 1.77392 | 2.3618 | 2.01666 | 1.89696 | 1.76295 | 1.94863 | 1.48202 | 1.56346 | 1.4904 | 1.242364 | 0.163976 |
| CYR61 | 1.84884 | 1.8708 | 1.96367 | 1.86825 | 1.93836 | 1.67208 | 1.66278 | 1.83124 | 1.4826 | 1.58392 | 1.190411 | 0.007402 |
| PTGS2 | 1.06918 | 1.69728 | 1.30103 | 1.19434 | 0.86018 | 1.23222 | 1.05105 | 0.86571 | 0.82491 | 0.92060 | 1.185492 | 0.18483 |
| PTGER2 | 1.4773 | 1.62808 | 1.38049 | 1.40232 | 1.41836 | 1.23662 | 1.36338 | 1.52867 | 1.1996 | 1.13777 | 1.123579 | 0.103969 |
| SERPINE1 | 1.76937 | 1.70092 | 2.21889 | 1.65135 | 1.98022 | 1.80487 | 2.29732 | 1.3215 | 1.63166 | 1.40872 | 1.126101 | 0.54559 |
| KLK4 | 1.54549 | 1.59644 | 1.35978 | 1.49101 | 1.75918 | 1.50995 | 1.27147 | 1.46541 | 1.43715 | 1.60592 | 1.066142 | 0.264824 |
| VEGFA | 1.68593 | 2.14978 | 1.39651 | 1.85637 | 1.99105 | 1.76582 | 1.74661 | 2.03606 | 2.12439 | 2.47586 | -1.15975 | 0.303236 |
| HLA-B | 3.65226 | 3.28131 | 2.41125 | 2.51196 | 2.72687 | 1.80966 | 2.19412 | 2.5838 | 2.32376 | 2.68608 | 1.512826 | 0.189428 |
| IGHA1 | 1.58588 | 1.98813 | 1.30588 | 1.98018 | 2.68537 | 1.47689 | 1.76693 | 1.35999 | 1.43632 | 1.2495 | 1.367179 | 0.163776 |
| BDNF | 1.38316 | 1.59933 | 1.53928 | 1.7238 | 1.50744 | 1.37246 | 1.37693 | 1.42466 | 1.71548 | 1.21055 | 1.094738 | 0.084877 |
| MMP3 | 3.68791 | 3.11483 | 4.40378 | 4.60635 | 4.19074 | 3.47404 | 3.43639 | 3.5111 | 4.04162 | 2.68192 | 1.46282 | 0.137774 |
| RBP4 | 1.24453 | 1.2273 | 1.10599 | 1.19801 | 1.39172 | 1.30933 | 1.42885 | 1.41841 | 1.41841 | 1.39368 | -1.11746 | 0.045797 |
| G0S2 | 2.64756 | 2.46616 | 1.99679 | 2.29322 | 2.18096 | 2.51531 | 2.10142 | 2.94242 | 2.8794 | 4.10135 | -1.50633 | 0.221038 |

【Fig 79】

【Fig 80】

[Fig 81]

EP 4 194 565 A1

## Correlation

| | LA sensitivity | ARTN | ML | LOR | FLG | FLG2 | PGF | CYR61 | PTGS2 | PTGER2 | SERPINE1 | KLK4 | VEGFA | HLAB | IGHA1 | BDNF | MMP3 | RBP4 |
|---|---|---|---|---|---|---|---|---|---|---|---|---|---|---|---|---|---|---|
| LA sensitivity Pearson Correlation | 1 | .197 | -.492 | -.563 | -.518 | -.487 | .662* | .839** | .505 | .573 | .341 | .332 | -.400 | .549 | .517 | .414 | .503 | -.758* |
| Significance probability (two-sided) | | .585 | .149 | .090 | .125 | .153 | .037 | .002 | .136 | .083 | .335 | .349 | .253 | .101 | .126 | .234 | .138 | .011 |
| N | 10 | 10 | 10 | 10 | 10 | 10 | 10 | 10 | 10 | 10 | 10 | 10 | 10 | 10 | 10 | 10 | 10 | 10 |

**Lactic acid sting test score greater than 0.5 (five subjects)/score of 0 (five subjects) (total ten subjects)**
**Correlation analysis between RNA microarray and lactic acid sting test score**

| Gene | ARTN | CYR61 | LOR | GOS2 | FLG | FLG2 | PTGS2 | RBP4 | BDNF | MMP12 | PGF | IGHA1 | PSPH | PTGER2 | HLA-B |
|---|---|---|---|---|---|---|---|---|---|---|---|---|---|---|---|
| Irritation score greater than 0.5 (five subjects) -Lactic acid sting test score | - | 0.01* | - | - | - | - | - | 0.05* | - | - | 0.05** | - | - | - | - |

【Fig 82】

Irritation score greater than 0.5 (five subjects)-Lactic acid sting test score
Scatter graph of statistically significant genes after correlation analysis

[Fig 83]

# Correlation

| | STING | ARTN | CYR61 | LOR | GOS2 | FLG | FLG2 | PTGS2 | RBP4 | BDNF | MMP12 | PGF | IGHA1 | PSPH | PTGER2 | HLAB |
|---|---|---|---|---|---|---|---|---|---|---|---|---|---|---|---|---|
| STING Pearson Correlation | 1 | 0.77 | .230 | -.373* | -.220 | -.289 | -.226 | .360* | -.195 | -.195 | .102 | .480** | .141 | .210 | .210 | .367* |
| Significance probability (two-sided) | | .669 | .198 | .033 | .220 | .103 | .205 | .040 | .278 | .278 | .572 | .005 | .433 | .240 | .241 | .035 |
| N | 33 | 33 | 33 | 33 | 33 | 33 | 33 | 33 | 33 | 33 | 33 | 33 | 33 | 33 | 33 | 33 |

## Total subjects (33 subjects) Correlation analysis between RNA microarray and lactic acid sting test score

| Gene | ARTN | CYR61 | LOR | GOS2 | FLG | FLG2 | PTGS2 | RBP4 | BDNF | MMP12 | PGF | IGHA1 | PSPH | PTGER2 | HLA-B |
|---|---|---|---|---|---|---|---|---|---|---|---|---|---|---|---|
| Total subjects -Lactic acid sting test score | - | - | 0.05* | - | - | - | 0.05* | - | - | - | 0.01** | - | - | - | 0.05* |

EP 4 194 565 A1

EP 4 194 565 A1

【Fig 84】

Total subjects - Lactic acid sting test score
Scatter graph of statistically significant genes after correlation analysis

111

【Fig 85】

【Fig 86】

| Gene symbol | Test group 1 | Test group 2 | Test group 3 | Test group 4 | Test group 5 | Control group 1 | Control group 2 | Control group 3 | Control group 4 | Control group 5 | Fold change | p-value |
|---|---|---|---|---|---|---|---|---|---|---|---|---|
| MMP1 | 0.95769 | 1.23915 | 1.1473 | 1.3729 | 1.26982 | 1.0219 | 1.04949 | 0.84907 | 1.21788 | 0.82628 | 1.152245 | 0.071464 |
| MMP3 | 3.58942 | 3.70435 | 3.85974 | 4.40378 | 4.74075 | 3.59603 | 3.47478 | 2.68192 | 3.64428 | 3.10455 | 1.692664 | 0.065145 |
| MMP12 | 3.53145 | 3.20151 | 2.89515 | 2.67884 | 3.09711 | 2.55279 | 2.1784 | 2.4989 | 2.44769 | 2.23459 | 1.622634 | 0.012363 |
| CCR1 | 1.30398 | 1.29101 | 0.92037 | 1.22781 | 1.14205 | 0.9975 | 0.97682 | 0.89479 | 0.89005 | 1.01493 | 1.166532 | 0.022954 |
| AKR1B10 | 2.4131 | 2.40471 | 2.60637 | 2.52101 | 2.23346 | 1.99003 | 1.99991 | 1.75291 | 2.68984 | 2.34784 | 1.213878 | 0.215043 |
| THY1 | 2.56452 | 3.16641 | 2.67027 | 3.02548 | 2.51469 | 2.50964 | 2.23344 | 2.16194 | 2.5278 | 2.18633 | 1.379785 | 0.031419 |
| IL1A | 2.04037 | 2.35164 | 2.24178 | 2.26282 | 1.87198 | 2.33556 | 1.68043 | 1.79577 | 2.08836 | 1.94062 | 1.137266 | 0.344175 |
| IL6 | 1.5651 | 1.82928 | 1.53686 | 1.60775 | 1.34882 | 1.32026 | 1.1545 | 1.26182 | 1.44908 | 1.28673 | 1.216793 | 0.053894 |
| IL12B | 1.2801 | 1.0915 | 1.38896 | 1.44399 | 1.55139 | 1.31096 | 1.14706 | 1.03298 | 1.18953 | 1.40313 | 1.097679 | 0.166949 |
| F10 | 1.66649 | 1.69507 | 1.7286 | 1.32394 | 1.73346 | 1.59841 | 1.598 | 1.66713 | 1.64568 | 1.64168 | -1.00046 | 0.993781 |

【Fig 87】

【Fig 88】

## Correlation

|  |  | Acne | MMP1 | MMP3 | MMP12 | CCR1 | AKR1B10 | THY1 | IL1A | IL6 | IL12B | F10 |
|---|---|---|---|---|---|---|---|---|---|---|---|---|
| Acne | Pearson Correlation | 1 | .585 | .685* | .836** | .723* | .491 | .732* | .415 | .742* | .425 | -.003 |
|  | Significance probability (two-sided) |  | .076 | .029 | .003 | .018 | .149 | .016 | .233 | .014 | .221 | .993 |
|  | N | 10 | 10 | 10 | 10 | 10 | 10 | 10 | 10 | 10 | 10 | 10 |

【Fig 89】

| Acne existence/non-existence (five subjects by group, total ten subjects) Physical findings correlation analysis | | | | | | | | | | |
|---|---|---|---|---|---|---|---|---|---|---|
| Gene | MMP1 | MMP3 | MMP12 | CCR1 | AKR1B10 | THY1 | IL1A | IL6 | IL12B | F10 |
| Acne existence/non-existence Physical findings (acne) | - | 0.05* | 0.01* | 0.05* | - | 0.05* | - | 0.05* | - | - |

【Fig 90】

【Fig 91】

【Fig 92】

【Fig 93】

【Fig 94】

【Fig 95】

## Correlation

|  | | Oily skin | MMP1 | MMP3 | MMP12 | CCR1 | AKR1B10 | THY1 | IL1A | IL6 | IL12B | F10 |
|---|---|---|---|---|---|---|---|---|---|---|---|---|
| **Oily skin** | Pearson Correlation | 1 | .057 | .109 | .811** | .725* | .080 | .378 | .034 | .529 | -.178 | .143 |
| | Significance probability (two-sided) | | .877 | .765 | .004 | .018 | .827 | .282 | .926 | .116 | .628 | .693 |
| | N | 10 | 10 | 10 | 10 | 10 | 10 | 10 | 10 | 10 | 10 | 10 |

【Fig 96】

## Acne existence/non-existence (five subjects by group, total ten subjects) Physical findings correlation analysis

| Gene | MMP1 | MMP3 | MMP12 | CCR1 | AKR1B10 | THY1 | IL1A | IL6 | IL12B | F10 |
|---|---|---|---|---|---|---|---|---|---|---|
| Acne existence/non-existence Physical findings (oily skin) | - | - | 0.01* | 0.05* | - | - | - | - | - | - |

【Fig 97】

【Fig 98】

【Fig 99】

## Correlation

| | | Sebu | MMP1 | MMP3 | MMP12 | CCR1 | AKR1B10 | THY1 | IL1A | IL6 | IL12B | F10 |
|---|---|---|---|---|---|---|---|---|---|---|---|---|
| Sebu | Pearson Correlation | 1 | .370 | .251 | .291 | .639* | .028 | .619 | .465 | .499 | -.003 | -.757* |
| | Significance probability (two-sided) | | .292 | .485 | .415 | .047 | .938 | .056 | .176 | .142 | .993 | .011 |
| | N | 10 | 10 | 10 | 10 | 10 | 10 | 10 | 10 | 10 | 10 | 10 |

【Fig 100】

| Acne existence/non-existence (five subjects by group, total ten subjects) Device applicable to the human body measurement result correlation analysis | | | | | | | | | | |
|---|---|---|---|---|---|---|---|---|---|---|
| Gene | MMP1 | MMP3 | MMP12 | CCR1 | AKR1B10 | THY1 | IL1A | IL6 | IL12B | F10 |
| Acne existence/non-existence - Sebumeter | - | - | - | 0.05* | - | - | - | - | - | - |

【Fig 101】

R² linear(L) = 0. 409

y=0.86+2.21E-3*x

## INTERNATIONAL SEARCH REPORT

| International application No. |
| --- |
| **PCT/KR2021/009297** |

| A. | CLASSIFICATION OF SUBJECT MATTER |
| --- | --- |
| | **C12Q 1/6837**(2018.01)i; **A61B 10/02**(2006.01)i |

According to International Patent Classification (IPC) or to both national classification and IPC

| B. | FIELDS SEARCHED |
| --- | --- |

Minimum documentation searched (classification system followed by classification symbols)

C12Q 1/6837(2018.01); A61B 10/02(2006.01); A61B 5/00(2006.01); A61K 8/14(2006.01); A61K 8/98(2006.01); C12N 15/09(2006.01); C12N 15/10(2006.01); C12Q 1/68(2006.01); G01N 33/15(2006.01); G01N 33/68(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 마이크로니들 패치(microneedle patch), 피부주름(skin wrinkle), 피부보습(skin evaluating), 색소침착(pigmentation), 피부유분(skin oil), 홍조성 민감피부(rosacea type sensitive skin), 주관적 자극성 민감피부(sensitive skin degree), 화장품 여드름(acne cosmetica), COL1A1, COL3A1, FN1, PINK1, IVL, HAS3, AQP3, BGN, AZGP1, FLG, AQP3, LOR, MLANA, TYRP1, GPNMB, MPZL3, GPAM, IGF1, MMP9, TAC1, CAMP, PGF, RBP4, CYR61, MMP3, MMP12, CCR1

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y <br><br> A | KR 10-2122208 B1 (RAPHAS CO., LTD.) 12 June 2020 (2020-06-12) <br> See paragraph [0066] and claim 6. | 1,3,5,7,9,11,13,15 <br><br> 2,4,6,8,10,12,14 |
| Y | KR 10-2012-0058995 A (AMOREPACIFIC CORPORATION et al.) 08 June 2012 (2012-06-08) <br> See paragraphs [0007] and [0015]-[0016] and claims 1-10. | 1,15 |
| Y | KR 10-2019-0069291 A (EXOCOBIO INC.) 19 June 2019 (2019-06-19) <br> See paragraphs [0137]-[0142]. | 3 |
| Y | JP 2007-209208 A (NARIS COSMETICS CO., LTD.) 23 August 2007 (2007-08-23) <br> See paragraph [0009] and claims 1-5. | 5 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **25 October 2021** | **25 October 2021** |

| Name and mailing address of the ISA/KR | Authorized officer |
| --- | --- |
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

| International application No. |
|---|
| **PCT/KR2021/009297** |

**C.    DOCUMENTS CONSIDERED TO BE RELEVANT**

| Category* | Citation of document, with indication, where appropriate, of the relevant passages | Relevant to claim No. |
|---|---|---|
| Y | KR 10-2008-0031590 A (GOODGENE INC. et al.) 10 April 2008 (2008-04-10)<br>See paragraphs [0066] and [0553] and claims 25-27. | 7 |
| Y | KR 10-2012-0115321 A (UNIVERSITAT MUNSTER et al.) 17 October 2012 (2012-10-17)<br>See paragraphs [0003], [0011] and [0055]-[0069] and claims 1-7. | 9 |
| Y | KR 10-2009-0123049 A (AMOREPACIFIC CORPORATION et al.) 02 December 2009 (2009-12-02)<br>See paragraphs [0052]-[0054] and claims 1-16. | 11 |
| Y | KR 10-2014-0047674 A (GALDERMA RESEARCH & DEVELOPMENT) 22 April 2014 (2014-04-22)<br>See paragraphs [0096] and [0104]-[0120] and claims 1-12. | 13 |
| PX | KR 10-2216910 B1 (CUTIS BIOMEDICAL RESEARCH CENTER) 18 February 2021 (2021-02-18)<br>See claims 1 and 3.<br>*This document is a published earlier application that serves as a basis for claiming priority of the present international application. | 1,2,15 |
| PX | KR 10-2216913 B1 (CUTIS BIOMEDICAL RESEARCH CENTER) 18 February 2021 (2021-02-18)<br>See claim 1.<br>*This document is a published earlier application that serves as a basis for claiming priority of the present international application. | 3,4 |
| PX | KR 10-2216923 B1 (CUTIS BIOMEDICAL RESEARCH CENTER) 18 February 2021.<br>See claim 1.<br>*This document is a published earlier application that serves as a basis for claiming priority of the present international application. | 5,6 |
| PX | KR 10-2216937 B1 (CUTIS BIOMEDICAL RESEARCH CENTER) 18 February 2021 (2021-02-18)<br>See claim 1.<br>*This document is a published earlier application that serves as a basis for claiming priority of the present international application. | 7,8 |
| PX | KR 10-2216941 B1 (CUTIS BIOMEDICAL RESEARCH CENTER) 18 February 2021 (2021-02-18)<br>See claim 1.<br>*This document is a published earlier application that serves as a basis for claiming priority of the present international application. | 9,10 |
| PX | KR 10-2216945 B1 (CUTIS BIOMEDICAL RESEARCH CENTER) 18 February 2021 (2021-02-18)<br>See claim 1.<br>*This document is a published earlier application that serves as a basis for claiming priority of the present international application. | 11,12 |
| PX | KR 10-2216927 B1 (CUTIS BIOMEDICAL RESEARCH CENTER) 18 February 2021 (2021-02-18)<br>See claim 1.<br>*This document is a published earlier application that serves as a basis for claiming priority of the present international application. | 13,14 |

Form PCT/ISA/210 (second sheet) (July 2019)

**INTERNATIONAL SEARCH REPORT**

International application No.

**PCT/KR2021/009297**

**Box No. III      Observations where unity of invention is lacking (Continuation of item 3 of first sheet)**

This International Searching Authority found multiple inventions in this international application, as follows:

Claims 1, 2 and 15 pertain to a minimally invasive skin condition diagnosing kit, which can diagnose skin aging by quantitating the expression level of biomarker genes related to collagen/elasticity or biomarker genes related to skin barrier function/moisture synthesis,

Claims 3 and 4 pertain to a minimally invasive skin condition diagnosing kit, which can evaluate the amount of skin moisture by quantitating the expression level of biomarker genes related to skin moisture,

Claims 5 and 6 pertain to a minimally invasive skin condition diagnosing kit, which can evaluate the amount of skin pigmentation by quantitating the expression level of biomarker genes related to skin pigmentation,

Claims 7 and 8 pertain to a minimally invasive skin condition diagnosing kit, which can evaluate the amount of skin oil by quantitating the expression level of biomarker genes related to skin oil,

Claims 9 and 10 pertain to a minimally invasive skin condition diagnosing kit, which can evaluate flushing and sensitive skin by quantitating the expression level of biomarker gene related to flushing and sensitive skin,

Claims 11 and 12 pertain to a minimally invasive skin condition diagnosing kit, which can evaluate subjective irritated and sensitive skin by quantitating the expression level of biomarker genes related to subjective irritated and sensitive skin,

Claims 13 and 14 pertain to a minimally invasive skin condition diagnosing kit, which can predict cosmetic-induced acne by quantitating the expression level of biomarker genes related to cosmetic-induced acne.

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2019)

INTERNATIONAL SEARCH REPORT
Information on patent family members

International application No.

PCT/KR2021/009297

| Patent document cited in search report | | | Publication date (day/month/year) | Patent family member(s) | | | Publication date (day/month/year) |
|---|---|---|---|---|---|---|---|
| KR | 10-2122208 | B1 | 12 June 2020 | None | | | |
| KR | 10-2012-0058995 | A | 08 June 2012 | CN | 103249842 | A | 14 August 2013 |
| | | | | CN | 103249842 | B | 06 July 2016 |
| | | | | EP | 2647724 | A2 | 09 October 2013 |
| | | | | EP | 2647724 | B1 | 08 March 2017 |
| | | | | HK | 1187079 | A1 | 28 March 2014 |
| | | | | JP | 2014-504155 | A | 20 February 2014 |
| | | | | JP | 2017-038619 | A | 23 February 2017 |
| | | | | KR | 10-1886342 | B1 | 10 August 2018 |
| | | | | US | 2013-0252845 | A1 | 26 September 2013 |
| | | | | WO | 2012-074246 | A2 | 07 June 2012 |
| KR | 10-2019-0069291 | A | 19 June 2019 | CN | 110869033 | A | 06 March 2020 |
| | | | | EP | 3646877 | A2 | 06 May 2020 |
| | | | | JP | 2020-532489 | A | 12 November 2020 |
| | | | | KR | 10-1964991 | B1 | 02 April 2019 |
| | | | | KR | 10-1964992 | B1 | 02 April 2019 |
| | | | | KR | 10-2019-0067758 | A | 17 June 2019 |
| | | | | KR | 10-2147169 | B1 | 24 August 2020 |
| | | | | US | 2020-0121722 | A1 | 23 April 2020 |
| | | | | WO | 2019-112229 | A2 | 13 June 2019 |
| | | | | WO | 2019-231050 | A1 | 05 December 2019 |
| JP | 2007-209208 | A | 23 August 2007 | None | | | |
| KR | 10-2008-0031590 | A | 10 April 2008 | KR | 10-1267430 | B1 | 30 May 2013 |
| KR | 10-2012-0115321 | A | 17 October 2012 | AU | 2010-332876 | A1 | 12 July 2012 |
| | | | | AU | 2010-332876 | A1 | 23 June 2011 |
| | | | | BR | 112012014678 | A2 | 05 April 2016 |
| | | | | CA | 2783693 | A1 | 23 June 2011 |
| | | | | CN | 102762742 | A | 31 October 2012 |
| | | | | EP | 2513328 | A1 | 24 October 2012 |
| | | | | IN | 6279DEN2012 | A | 25 September 2015 |
| | | | | JP | 2013-514070 | A | 25 April 2013 |
| | | | | MX | 2012006623 | A | 30 November 2012 |
| | | | | RU | 2012130069 | A | 27 January 2014 |
| | | | | US | 2013-0017969 | A1 | 17 January 2013 |
| | | | | WO | 2011-073321 | A1 | 23 June 2011 |
| KR | 10-2009-0123049 | A | 02 December 2009 | KR | 10-1500902 | B1 | 12 March 2015 |
| | | | | KR | 10-1543172 | B1 | 07 August 2015 |
| | | | | KR | 10-1543173 | B1 | 07 August 2015 |
| | | | | KR | 10-1543174 | B1 | 07 August 2015 |
| | | | | KR | 10-1543175 | B1 | 07 August 2015 |
| | | | | KR | 10-1543176 | B1 | 07 August 2015 |
| | | | | KR | 10-1543177 | B1 | 07 August 2015 |
| | | | | KR | 10-1543178 | B1 | 07 August 2015 |
| | | | | KR | 10-1543179 | B1 | 07 August 2015 |
| | | | | KR | 10-1543180 | B1 | 07 August 2015 |
| | | | | KR | 10-1543181 | B1 | 07 August 2015 |
| | | | | KR | 10-1543182 | B1 | 07 August 2015 |
| | | | | KR | 10-1543183 | B1 | 07 August 2015 |
| | | | | KR | 10-1543184 | B1 | 07 August 2015 |

Form PCT/ISA/210 (patent family annex) (July 2019)

**INTERNATIONAL SEARCH REPORT**
Information on patent family members

International application No.

**PCT/KR2021/009297**

| Patent document cited in search report | | | Publication date (day/month/year) | Patent family member(s) | | | Publication date (day/month/year) |
|---|---|---|---|---|---|---|---|
| | | | | KR | 10-1543185 | B1 | 07 August 2015 |
| | | | | KR | 10-1543186 | B1 | 07 August 2015 |
| KR | 10-2014-0047674 | A | 22 April 2014 | AU | 2012-277951 | A1 | 20 February 2014 |
| | | | | AU | 2017-202172 | A1 | 20 April 2017 |
| | | | | BR | 112013033617 | A2 | 01 March 2017 |
| | | | | CA | 2840403 | A1 | 03 January 2013 |
| | | | | CN | 103930565 | A | 16 July 2014 |
| | | | | CN | 107012201 | A | 04 August 2017 |
| | | | | EP | 2723889 | A1 | 30 April 2014 |
| | | | | EP | 2723889 | B1 | 06 November 2019 |
| | | | | IN | 586CHN2014 | A | 03 April 2015 |
| | | | | JP | 2014-519855 | A | 21 August 2014 |
| | | | | JP | 2017-051198 | A | 16 March 2017 |
| | | | | MX | 2014000169 | A | 19 February 2014 |
| | | | | MX | 349048 | B | 07 July 2017 |
| | | | | US | 2014-0235475 | A1 | 21 August 2014 |
| | | | | US | 2016-0237503 | A1 | 18 August 2016 |
| | | | | WO | 2013-000869 | A1 | 03 January 2013 |
| KR | 10-2216910 | B1 | 18 February 2021 | None | | | |
| KR | 10-2216913 | B1 | 18 February 2021 | None | | | |
| KR | 10-2216923 | B1 | 18 February 2021 | None | | | |
| KR | 10-2216937 | B1 | 18 February 2021 | None | | | |
| KR | 10-2216941 | B1 | 18 February 2021 | None | | | |
| KR | 10-2216945 | B1 | 18 February 2021 | None | | | |
| KR | 10-2216927 | B1 | 18 February 2021 | None | | | |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020200100151
- KR 1020200100165
- KR 1020200100168
- KR 1020200100579
- KR 1020200101117
- KR 1020200101124
- KR 1020200100561

### Non-patent literature cited in the description

- **YOSHIDA-AMANO.** *Int J Dermatol,* 2017
- **HOMBURG.** *Reprod Biol Endocrinol,* 2017
- Korean acne severity system. *Korean Journal of Dermatology,* 2004, vol. 24 (10